(19) **Europäisches Patentamt / European Patent Office / Office européen des brevets**

(11) **EP 2 613 171 B1**

(12) **EUROPEAN PATENT SPECIFICATION**

(45) Date of publication and mention of the grant of the patent:
**13.04.2016 Bulletin 2016/15**

(21) Application number: **13162029.6**

(22) Date of filing: **09.01.2009**

(51) Int Cl.:
*G01S 7/52* (2006.01)    *G01S 15/89* (2006.01)

(54) **Nonlinear elastic imaging with two-frequency elastic pulse complexes**

Nichtlineare elastische Bildgebung mit elastischen Impulskomplexen mit zwei Frequenzen

Imagerie élastique non linéaire avec deux ensembles d'impulsion élastique à deux fréquences

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **09.01.2008 US 10486 P**
**16.05.2008 US 127898 P**

(43) Date of publication of application:
**10.07.2013 Bulletin 2013/28**

(62) Document number(s) of the earlier application(s) in accordance with Art. 76 EPC:
**09700892.4 / 2 294 448**

(73) Proprietor: **Surf Technology AS**
**7491 Trondheim (NO)**

(72) Inventors:
• **Hansen, Rune**
**7048 Trondheim (NO)**
• **Johansen, Tonni F**
**N-7034 Trondheim (NO)**
• **Måsøy, Svein-Erik**
**N-7034 Trondheim (NO)**
• **Näsholm, Sven Peter**
**N-7034 Trondheim (NO)**
• **Tangen, Thor Andreas**
**N-7034 Trondheim (NO)**

• **Angelsen, Bjorn**
**7051 Trondheim (NO)**

(74) Representative: **Prock, Thomas**
**Marks & Clerk LLP**
**90 Long Acre**
**London WC2E 9RA (GB)**

(56) References cited:
**US-A1- 2005 277 835    US-A1- 2006 052 699**

• **ANGELSEN B A J ET AL: "SURF Imaging - A New Method for Ultrasound Contrast Agent Imaging", ULTRASONICS SYMPOSIUM, 2007. IEEE, IEEE, PISCATAWAY, NJ, USA, 30 October 2007 (2007-10-30), pages 531-541, XP031195031, ISBN: 978-1-4244-1383-6**
• **MASOY, SVEIN-ERIK ET. AL.: "SURF Imaging: In vivo demonstration of an ultrasound contrast agent detection technique", INTERNET, 19 September 2007 (2007-09-19), pages 1-20, XP002530972, Trondheim**
• **DENG C X ET AL: "Study of ultrasonic contrast agents using a dual-frequency band technique", ULTRASOUND IN MEDICINE AND BIOLOGY, NEW YORK, NY, US, vol. 26, no. 5, 1 June 2000 (2000-06-01), pages 819-831, XP004295620, ISSN: 0301-5629**

**Description**

*1. Field of the invention*

The present invention is directed to methods and instrumentation that utilizes nonlinear elasticity for measurements and imaging with elastic waves in materials, as for example, but not limited to medical ultrasound imaging, ultrasound nondestructive testing, sub sea SONAR applications, and geological applications.

*2. Background of the invention*

Nonlinear elasticity means that the material elastic stiffness changes with elastic deformation of the material. For example does the material volume compression stiffness increase with volume compression of the material with a subsequent increase in the volume compression wave propagation velocity. Similarly does volume expansion reduce the material volume compresion stiffness with a subsequent reduction in volume compression wave propagation velocity.

In solids one also has a shear deformation elasticity which makes shear deformation waves possible in the solids. Gases and fluids are fully shape deformable, and hence do not have shear elasticity and shear waves. Soft biological tissues behave for pressure waves mainly as a fluid (water), but the solid constituents (cells) introduce a shear deformation elasticity with low shear modulus. The propagation velocity of pressure compression waves are for example ~ 1500 m/sec in soft tissues, while shear waves have propagation velocities ~ 1 - 10 m/sec only. Compared to volume compression, shear deformation of solid materials has a more complex nonlinear elasticity, where in general for isotropic materials any shear deformation increases the shear modulus with a subsequent increase in shear wave velocity. The shear modulus is also in general influenced by volume compression, where as for the bulk modulus a volume compression increases the shear modulus with a subsequent increase in shear wave velocity while volume expansion decreases the shear modulus with a subsequent decrease in shear wave velocity. For anisotropic materials the dependency of the shear modulus with shear deformation can be more complex, where shear deformation in certain directions can give a decrease in shear elastic modulus with a decrease in shear wave velocity.

As different materials have different nonlinear elasticity, compression/expansion/deformation of a spatially heterogeneous material will change the spatial variation of the elasticity and hence produce a scattering that depends on the material strain. The scattered signal can hence be separated into a linear scattering component produced by the heterogeneous elasticity at low strain, and a nonlinear scattering component of elastic waves from the modification of the heterogeneous elasticity produced by large strain in the material. Nonlinear elasticity hence influences both propagation and scattering of pressure waves in gases, fluids and solids, and also of shear waves in solids. The nonlinear volume elasticity effect is generally strongest with gases, intermediate with fluids, and weakest with solid materials.

Acoustic noise produced by multiple scattering and wave front aberrations reduces the image quality and produces problems for extraction of the nonlinearly scattered signal and propagation and scattering parameters. Current ultrasound image reconstruction techniques take as an assumption that the wave propagation velocity do not have spatial variations, and that the ultrasound pulse is scattered only once from each scatterer within the beam (1st order scattering). In most situations, especially in difficult to image patients, the 1st order scattered pulse will be rescattered by a 2nd scatterer (2nd order scattered wave), which is rescattered by a 3rd scatterer (3rd order scattered wave) etc. Odd orders of scattered waves will have an added propagation delay and show as acoustic noise in the image.

In US Pat Appl US2005277835 and US2006052699 methods are described where one transmits at least two elastic wave pulse complexes composed of a pulse in a high frequency (HF) band and a pulse in a low frequency (LF) band, both for suppression of acoustic pulse reverberation noise (multiple scattering noise) and for estimation of elastic wave nonlinear propagation properties and elastic wave nonlinear scattering in heterogeneous materials. The LF pulse is used to nonlinearly manipulate the material elasticity that is observed by the HF pulse along its propagation path, and hence nonlinearly manipulate both the propagation velocity and the scattering for the HF pulse. The applications exemplify the method for ultrasound imaging of soft tissues, but it is clear that the method is applicable to all types of elastic wave imaging, as for example but not limited to, nondestructive testing of materials, sub sea SONAR applications, geological applications, etc. The methods are applicable with compression waves in gases, fluids, and solids, and also with shear waves in solids. Shear waves can for example be transmitted with special transducers, be generated by the radiation force from compression, or by skewed inclination of pressure waves at material interfaces. Similarly can pressure waves be generated both directly with transducers and with skewed inclination of shear waves at material interfaces.

When the LF pulse pressure varies along the HF pulse, the different parts of the HF pulse gets different propagation velocities that introduces a change of the pulse length and possibly also a distortion of the pulse form of the HF pulse that accumulates along the propagation path. Such a variation of the LF pulse pressure can be found when the HF pulse is located on a spatial gradient of the LF pulse, but also when a comparatively long HF pulse is found around the pressure maxima and minima of the LF pulse. We will in the following refer to these modifications of the HF pulse length and form by the LF pulse as HF *pulse distortion.*

With an LF aperture that is so wide that the whole HF imaging range is within the near field of the LF beam, one can obtain a defined phase relation between the HF and LF pulse, where the HF pulse can be at the crest or through of the LF pulse for the whole imaging range. With diffraction limited, focused LF beams, the pressure in the focal zone is the time derivative of the pressure at the transducer surface. The phase relation between HF pulse and the LF pulse will hence in this case slide with depth. For the HF pulse to be at the crest (or trough) of the LF pulse in the LF focal region, the pulse must be transmitted at the negative (positive) spatial gradient of the LF pulse at the transducer. This produces an accumulative length compression of the HF pulse when it is located along a negative spatial gradient of the LF pulse, and an accumulative length stretching of the HF pulse when it is found along a positive spatial gradient of the LF pulse. In order to obtain adequately collimated LF beams, it is often advantageous to use a LF transmit aperture that is wider than the HF transmit aperture, and/or the LF transmit focus is different from the HF transmit focus. This gives an additional phase sliding with the propagation distance of the HF pulse relative to the LF pulse. To suppress multiple scattering noise, one often use a LF transmit aperture with an inactive region around its center. This gives a LF beam with increased phase sliding between the HF and LF pulses.

When the phase between the HF and LF pulses slides with propagation distance, the LF pulse can provide different modifications to the HF pulse at different depths. For example can the HF pulse be at the negative spatial gradient of the LF pulse at low depths and slide via an extremum with negligible spatial gradient of the LF pulse towards a positive spatial gradient of the LF pulse along the HF pulse at deep ranges. The HF pulse in this example observes accumulative pulse compression at shallow depths, via an intermediate region with limited pulse distortion, towards an accumulative pulse length expansion at deep ranges, where the deep range pulse length expansion counteracts the shallow range pulse length compression. Switching the polarity of the LF pulse changes the pulse compression to pulse expansion and vice versa. As the pulse distortion changes the frequency content of the HF pulse, the frequency varying diffraction and power absorption will also change the HF pulse amplitude with the distortion, and we include these phenomena in the concept of HF pulse distortion.

The HF pulse distortion will hence be different for different amplitudes, phases and polarities of the LF pulse, a phenomenon that limits the suppression of the linearly scattered signal to obtain the nonlinearly scattered signal with pure delay correction, for example as described in US Pat Appl US2005277835 and US2006052699. The current invention presents methods that improve the suppression of the linear scattering for improved estimation of the nonlinear scattering, and also introduces improved methods of suppression of pulse reverberation noise.

## 3. Summary of the invention

This summary gives a brief overview of components of the invention and does not present any limitations as to the extent of the invention, where the invention is solely defined by the claims appended hereto. The methods are applicable both for backscatter and transmission tomographic image reconstruction methods.

At least two elastic wave pulse complexes composed of a pulse in a low frequency (LF) band and a pulse in a high frequency (HF) band, are transmitted into the object, where at least the transmitted LF pulses varies in phase (relative to the transmitted HF pulse), and/or amplitude, and/or frequency for each transmitted pulse complex. The LF pulses are used to nonlinearly manipulate the material elasticity observed by the HF pulses along at least parts of the propagation path of the HF pulses. By the received HF signal we do in this description mean received signal from the transmitted HF pulses that are one or both of scattered from the object and transmitted through the object, and where signal components from the transmitted LF pulse with potential harmonic components thereof, are removed from the signal, for example through filtering of the received HF signal. Such filtering can be found in the ultrasound transducers themselves or in the receiver channel of an instrument. For adequately stationary objects, one can also suppress received components of the transmitted LF pulse by transmitting a LF pulse with zero HF pulse and subtracting the received signal from this LF pulse with zero transmitted HF pulse from the received HF signal with a transmitted LF/HF pulse complex. The received HF signal can contain harmonic components of the HF band produced by propagation and scattering deformation of the HF pulse, and in the processing one can filter the received HF signal so that the fundamental band, or any harmonic band, or any combination thereof, of the HF pulse is used for image reconstruction. The harmonic components of the HF pulse can also be extracted with the well known pulse inversion (PI) method where the received HF signals from transmitted HF pulses with opposite polarity are added. By the received HF signal we hence mean at least one of the received HF radio frequency (RF) signal at the receiver transducer with any order of harmonic components thereof, and any demodulated form of the HF RF signal that contain the same information as the received HF RF signal, such as an I-Q demodulated version of the HF RF signal, or any shifting of the HF RF signal to another frequency band than that found at the receiver transducer. Such frequency shifting is known to anyone skilled in the art, and it is also known that processing on the RF signal can equivalently be done on any frequency shifted version of the RF signal.

For back-scatter imaging, the received HF signal will be picked up by a focused receive beam, usually dynamically focused, so that we will by large observe only the nonlinear manipulation of the object by the LF pulse for the HF pulse close to the axis of the receive beam. With tomographic image reconstruction methods the image reconstruction introduces a spatial resolution so that we in each pixel observe the nonlinear manipulation by the LF pulse for the HF pulse along the propagation path through said image pixel. The HF pulses from said at least two transmitted pulse complexes hence observe different propagation velocities and different nonlinear scattering from the object, the differences being produced by the differences in the transmitted LF pulses. The LF pulses often have opposite polarity for the said at least two different pulse complexes, but one also can vary the amplitude and/or phase and/or frequency and/or transmit aperture and/or transmit focus of the LF pulse for each transmitted pulse complex.

The maximal transmit amplitude is often limited by the Mechanical Index (MI) of the pulse complex, which is determined by the negative swing of the pressure. One can hence use higher amplitudes when the HF pulse is placed at the positive pressure swing of the LF pulse, compared to when the HF pulse is placed at the negative pressure swing of the LF pulse. Variations in the amplitude of the LF pulse between complexes are then accounted for in the processing of the received signal. The HF pulse can also be varied for transmitted pulse complexes, for example by switching the polarity and/or the amplitude of the HF pulses, where the variation is accounted for in the processing of the received HF signals.

The LF pulse can be a simple narrowband pulse, or it can be a more complex pulse with frequencies in the LF band. For example, in one method according to the invention the LF pulse is composed of a fundamental band and a $2^{nd}$ harmonic band, or a fundamental band and a band around 1.5 the fundamental center frequency, in order to obtain similar pulse distortion for positive and negative polarities of the LF pulse. The HF pulse can also be a simple narrowband pulse, or it can be a more complex pulse with frequencies in the HF band, for example Barker, Golay, or chirp code pulses which allows transmission of higher power with limited pulse amplitude, for example limited by the MI, where improved resolution is obtained with pulse compression in the receive processing, according to known methods.

The received HF signal from the said at least two transmitted pulse complexes are processed to form image signals, where said processing occurs both in the fast time (depth-time) and as a combination between received HF signals from at least two transmitted pulse complexes, which is referred to as *combination* or *filtering in the slow time* also referred to as *combination* or *filtering along the pulse number coordinate*, where slow time is represented by the pulse number coordinate. According to the invention the processing at least includes one or both of the steps:

a) relative delay correction in the fast time (depth-time) of the received HF signals from different transmitted pulse complexes to compensate for the differences in propagation delay of the HF pulse produced by the nonlinear manipulation of the propagation velocity by the LF pulse, averaged along the HF pulse, and

b) pulse distortion correction of the received HF signal in the fast time to compensate for the HF pulse distortion produced by variations of the propagation velocity along the HF pulse produced by variations in the LF pressure along the HF pulse in non-negligible regions of the depth along the HF beam axis. Said pulse distortion correction can be done through one or more of i) fast time filtering of the received HF signal in pulse distortion correction filters, and ii) pre-distortion of the transmitted HF pulses that counteracts the propagation distortion of the HF pulses, and iii) frequency mixing of the received HF signal, and iv) a piece-wise fast time expansion/compression and amplitude correction of the received HF signal, v) other methods, and vi) a combination of the above. The pulse distortion correction includes both a modification in fast time frequency content and amplitude to compensate for the distortion effect of nonlinear propagation and frequency varying diffraction, absorption, and also conversion of HF power to higher harmonic bands. The corrections for pulse distortion and propagation delay variations can be interchanged, and the filters can also perform a combined pulse distortion and delay correction. However, for efficient processing one would correct for the nonlinear propagation delays with an interpolated delay operation and use a filter for correction of the pulse distortion, as this requires the shortest filter impulse response.

After the delay and/or pulse distortion corrections in the fast time the received HF signals from said at least two transmitted pulse complexes are combined in slow time (i.e. pulse number coordinate) for one or both of

i) suppression of pulse reverberation noise (multiple scattering noise) to enhance the first order scattered HF signal from the object, and

ii) suppression of the linearly scattered HF signal from the object to relatively enhance the nonlinearly scattered HF signal from the object.

A typical form of slow time combination of the signals is a subtraction of the fast time processed received HF signals, or other type of filter in the slow time (along the pulse number coordinate), typically a high pass filter. One can also for example change the polarity of the HF pulse for each transmitted pulse complex, where subtraction of the signals for suppression of linear scattering or multiple scattering noise is substituted with a sum of the signals by which the slow time high pass filter is substituted by a low pass filter, according to known methods.

The processed HF signals are further processed to form image signals such as scattering amplitude images, color images representing Doppler frequencies and displacement strain, computer tomographic image reconstruction for transmitted signals at different directions, etc., where many such methods are known in prior art.

The correction delays and pulse distortion corrections can for example be calculated from simulated LF and HF pulse propagation using local nonlinear elastic wave propagation parameters of the medium that for example can be assumed, or manually adjusted, or estimated from the received HF signal, or the correction delays and pulse distortion corrections can be directly estimated from the received HF signals from said at least two transmitted pulse complexes, or a combination thereof. The wave propagation parameters are composed of the local mass density and linear and nonlinear components of the local elasticity matrix of the material.

One can further by example estimate the corrections delays from the received HF signals from transmitted pulse complexes with different LF pulses, for example as described in US Pat Appl US2005277835 and US2006052699. The fast time gradient of the estimated nonlinear propagation delays provides quantitative estimates of local, nonlinear elasticity parameters that can be used to estimate pulse distortion corrections from simulations of the composite LF and HF pulse beams. Such differentiation can be obtained with band limited filters or model based estimation where one compares the estimated delays with the simulated amplitude and phase between the HF and LF pulses as a function of depth along the beam axis. One can also do a manual adjustment of the nonlinear propagation delays or local nonlinear elasticity parameters as inputs to a simulation of the pulse distortion corrections and potentially also delay corrections, where the local nonlinear elasticity parameters are adjusted for maximal suppression of pulse reverberation noise (multiple scattering noise) or the linear scattering components in the received HF signal, for example as observed on the image display screen. The pulse distortion corrections can also be estimated from correlation functions or Fourier spectra of the received HF signals at similar depth ranges from transmitted pulse complexes with different LF pulses.

The invention also includes design criteria for LF and HF transmit apertures that minimize the sliding between the HF and the LF pulse to obtain best possible results of suppression of pulse reverberation noise and extract nonlinear scattering and propagation parameters. We further present methods for combined suppression of pulse reverberation noise, estimation of nonlinear scattering, and estimation of nonlinear propagation and scattering parameters. The signals after the suppression of pulse reverberation noise are very useful for improved estimation of corrections for wave front aberrations in spatially heterogeneous objects, for example according to the methods described in US Pat 6,485,423, US Pat 6,905,465, US Pat 7,273,455, and US Pat Appl US2005277835 and US2006052699.

The invention further includes instruments that incorporate the methods in practical elastic wave imaging of objects.

### 4. Summary of the drawings

FIG. 1 shows examples of pulse complexes with LF and HF pulses with different phase relationships that occur in the application of the methods,

FIG. 2 shows propagation sliding in the phase between the HF and LF pulses and the effect on pulse form distortion.

FIG. 3 shows example radiation surfaces for the LF and HF pulses for analysis of the signal processing together with example phase relations between LF and HF pulses in the object,

FIG. 4 shows dual frequency component Lf pulses with reduced pulse form distortion,

FIG. 5 shows required phase relations between HF and LF pulses for best imaging of microbubbles when the LF frequency is below and close to the bubble resonance frequency.

FIG. 6 shows an illustration to how multiple scattering noise is generated and how it can be suppressed with methods according to the invention,

FIG. 7 shows conceptual illustrations to pulse reverberation noise of Class I - III,

FIG. 8 shows conceptual illustration of combined suppression for Class I and II pulse reverberation noise,

FIG. 9 shows conceptual illustration of combined suppression for Class I and II pulse reverberation noise,

FIG. 10 shows a block diagram of signal processing for suppression of one or both of pulse reverberation noise, and linear scattering components to extract the nonlinearly scattered components,

FIG. 11 shows a block diagram of an instrument for backscatter imaging according to the invention,

FIG. 12 shows a block diagram of an instrument for computer tomographic image reconstruction from transmitted and scattered waves, and

FIG. 13 shows a conceptual illustration for imaging of geologic structures around an oil well according to the invention.

### 5. Detailed description of the Invention

Example embodiments according to the invention will now be described with reference to the drawings.

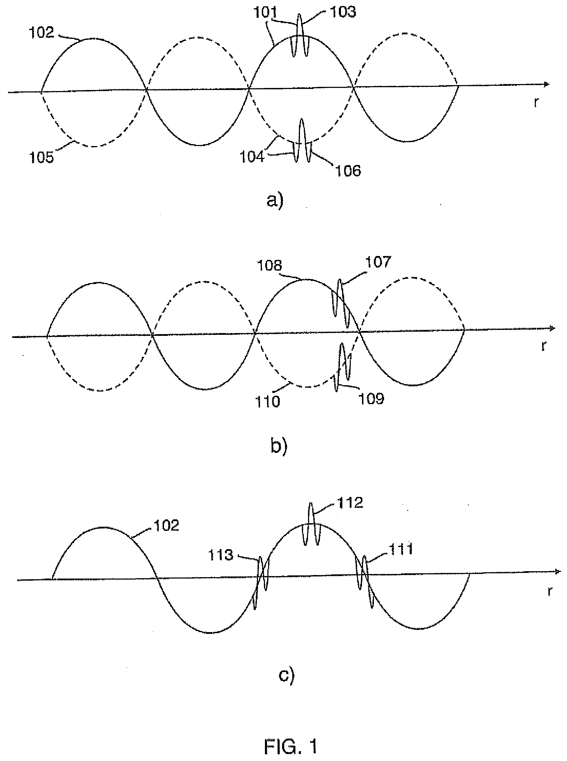

**FIG. 1a** shows a 1st example transmitted pulse complex **101** composed of a low frequency (LF) pulse **102** and a high frequency (HF) pulse **103**, together with a 2nd example pulse complex **104** composed of a LF pulse **105** and a HF pulse **106**. The LF pulse **102** will compress the material at the location of the HF pulse **103**, and nonlinear elasticity will then increase the material stiffness and also the propagation velocity observed by the HF pulse **103** along its propagation path. In the 2nd pulse complex **104** the LF pulse **105** expands the material at the location of the HF pulse **106**, with a subsequent reduction in material stiffness and reduction in propagation velocity of the HF pulse **106** due to the nonlinear elasticity of the material. For fluids and solids, the elasticity can generally be approximated to the 2nd order in the pressure, i.e. the volume compression $\delta V$ of a small volume $\Delta V$ is related to the pressure p as

$$\frac{\delta V}{\Delta V} = \left(1 - \beta_n(\underline{r})\kappa(\underline{r})p\right)\kappa(\underline{r})p \qquad (1)$$

where $\underline{r}$ is the spatial position vector, $\kappa(\underline{r})$ is the linear bulk compressibility of the material, and $\beta_n(\underline{r})=1+B(\underline{r})/2A(\underline{r})$ is a nonlinearity parameter of the bulk compressibility. The material parameters have a spatial variation due to heterogeneity in the material. Gases generally show stronger nonlinear elasticity, where higher order terms in the pressure often must be included. Micro gas-bubbles in fluids with diameter much less than the ultrasound wavelength, also shows a resonant compression response to an oscillating pressure, which is discussed below.

The propagation velocity for the HF pulse will then for a 2nd order elastic material be affected by the LF pulse pressure $p_{LF}$ at the locations of the propagating HF pulse, and for approximation of the pressure dependency to the 1st order in the pressure, we get

$$c\left(\underline{r}, p_{LF}\right) = c_0(\underline{r})\left\{1 + \beta_n(\underline{r})\kappa(\underline{r})p_{LF}\right\} \qquad (2)$$

where $c_0(\underline{r})$ is the unmodified propagation velocity as a function of HF pulse location $\underline{r}$. For a dynamically focused HF receive beam one do with back-scatter imaging observe the object at a narrow region around the beam axis. The propagation lag of the HF pulse along the beam axis can then be approximated as

$$t(r) = \int_{\Gamma(r)} \frac{ds}{c\left(s, p_{LF}(s)\right)} = t_0(r) + \tau(r)$$

$$t_0(r) = \int_{\Gamma(r)} \frac{ds}{c_0(s)} \qquad \tau(r) = -\int_{\Gamma(r)} \frac{ds}{c_0(s)} \beta_n(s)\kappa(s)p_{LF}(s) \qquad (3)$$

$$\tau(t) = -\int_0^t dt_0 \beta_n\left(s(t_0)\right)\kappa\left(s(t_0)\right)p_{LF}\left(s(t_0)\right)$$

where $\Gamma(r)$ is the propagation path of the HF pulse to depth r and the coordinate s denotes the ray location along the HF pulse at any time. The propagation lag without manipulation of the propagation velocity by the LF pulse is $t_0(r)$. For

a homogeneous material $t_0(r) = (propagation\ length)/c_0$, where for backscatter imaging at a range r, we have *propagation length = 2r* and $t_0(r) = 2r/c_0$. $\tau(r)$ is the added nonlinear propagation delay produced by the nonlinear manipulation of the propagation velocity for the HF pulse by the LF pulse. We note that when a scattering/reflection occurs the LF pulse pressure $p_{LF}$ drops considerably at the location of the scattered HF pulse so that the LF modification of the propagation velocity is negligible for the scattered wave. This means that we only get contribution in the integral for $\tau(r)$ up to the 1st scattering, an effect that we will use to suppress multiple scattered waves in the received signal.

We have here given a single coordinate r along the propagation path of the pulse. This is a good approximation with adequately focused beams for the received HF signal, so that one observes a limited cross section along the path of the HF pulse, and can also be obtained for angular scattering and transmission tomographic image reconstruction. The basic idea of the invention do however go beyond such a limited description, but the limited description is used for simplicity to illustrate basic aspects of the invention that also applies to situations where a full 3D description of the pulse propagation is required.

The variation of the propagation velocity with the pressure, will also produce a self distortion of both the LF and HF pulses, that introduces harmonic components of the fundamental bands of the pulses. The different harmonic components have different diffraction, which also influences the net self-distortion from the nonlinear propagation velocity. The LF beam is generally composed of a near-field region where the beam is mainly defined by geometric extension of the LF radiation aperture, and a diffraction limited region, which is the focal region for a focused aperture, and the far-field for an unfocused aperture. In the diffraction limited region, the self distortion of the positive (**102**) and negative (**105**) LF pulses will be different so that the elasticity manipulation of the two pulses will be different in this region.

By the received HF signal we do in this description mean the received signal from the transmitted HF pulses that are either scattered from the object or transmitted through the object, and where signal components from the transmitted LF pulse with potential harmonic components thereof, are removed from the signal, for example through filtering of the received HF signal. Such filtering can be found in the ultrasound transducers themselves or in the receiver channel of an instrument. For adequately stationary objects, one can also suppress received components of the transmitted LF pulse by transmitting a LF pulse with zero HF pulse and subtracting the received signal from this LF pulse with zero transmitted HF pulse from the received HF signal with a transmitted LF/HF pulse complex. The received HF signal can contain harmonic components of the HF band produced by propagation and scattering deformation of the HF pulse, and in the processing one can filter the received HF signal so that the fundamental band, or any harmonic band, or any combination thereof, of the HF pulse is used for image reconstruction. The harmonic components of the HF pulse can also be extracted with the well known pulse inversion (PI) method where the received HF signals from transmitted HF pulses with opposite polarity are added. By the received HF signal we hence mean at least one of the received HF radio frequency (RF) signal at the receiver transducer and any order of harmonic components thereof, and any demodulated form of the HF RF signal that contain the same information as the received HF RF signal, such as an I-Q demodulated version of the HF RF signal, or any shifting of the HF RF signal to another frequency band than that found at the receiver transducer. Such frequency shifting is known to anyone skilled in the art, and it is also known that processing on the RF signal can equivalently be done on any frequency shifted version of the RF signal.

Different materials, as for example gas bubbles, micro calcifications, connective tissue, fat, polymers, wax, scales, concrete, metals, etc. have different nonlinear elastic parameters. The incident LF pulse will therefore in a nonlinearly heterogeneous material change the spatial variation of the local elasticity observed by the HF pulse, and hence produce a local scattering of the HF pulse that depends on the LF pressure at the HF pulse. The scattered HF pulse can therefore be separated into a linear component of the scattered signal that is found for zero LF pulse, and a nonlinear modification to the linear scattering obtained with the presence of a LF pulse. This nonlinear modification of the scattered signal is referred to as the *nonlinear scattering, component* of elastic waves in the heterogeneous material, or the *nonlinearly scattered wave* or *signal.*

For materials where the elasticity can be approximated to the 2nd order in the pressure, the elastic parameters and hence the nonlinearly scattered signal will be approximately linear in the amplitude of the LF pressure at the location of the HF pulse. For micro gas-bubbles in fluids with diameter much less than the ultrasound wavelength, the elastic compression is more complex than the 2nd order approximation to elasticity. For the first, do gases show a stronger nonlinear elasticity where higher order than the 2nd order term in the pressure in Eq. (1) must be included. For the second, when the bubble diameter is much smaller than the ultrasound wave length in the fluid, one obtains large shear deformation of the fluid around the bubble when the bubble diameter changes. This shear deforming fluid behaves as a co-oscillating mass that interacts with the bubble elasticity to form a resonant oscillation dynamics of the bubble diameter. The volume of this co-oscillating mass is approximately 3 times the bubble volume.

The scattering from such micro-bubbles will then depend on both the frequencies and the amplitudes of the LF and HF pulses. For frequencies well below the resonance frequency, the bubble compression is dominated by the nonlinear bubble elasticity, which for high pressures requires higher than 2nd order approximation in the pressure. For frequencies around the resonance, one gets a phase shift of around 90 deg between the pressure and the volume compression. For frequencies well above the resonance frequency the bubble compression is dominated by the co-oscillating mass with a phase shift of 180 deg to the incident pressure, which gives a negative compliance to the pressure. Micro bubbles are used for ultrasound contrast agent in medicine, but their density in tissue is usually so low that their effect on the wave propagation can usually be neglected, where they are mainly observed as local, nonlinear point scatterers. The forward pulse propagation therefore usually observes a $2^{nd}$ order elasticity of the surrounding tissue that produces a nonlinear propagation lag according to Eq.(3). With high densities of bubbles in blood filled regions like the heart ventricles and blood vessels, the micro-bubbles can have marked nonlinear effect on the pressure variation of the wave propagation velocity, and also introduce a frequency dependent propagation velocity (dispersion) due to bubble resonances. With the method according to this invention, one can estimate the nonlinear propagation lag and pulse form distortion produced by the gas bubbles in the blood, and hence separate the accumulative, forward propagation effect of the bubbles and the local nonlinear scattering from the bubbles. Further details on the scattering from micro bubbles are discussed in relation to Eqs. (35, 36, 40).

For this most general situation we can then model the received HF signal for the transmitted pulse complexes **101** and **104** as

$$s_1(t) = p_{h1} x_l\left(t - \tau_1(t)\right) + x_{n1}\left(t - \tau_1(t); p_{h1}, p_{l1}\right) \qquad a)$$

$$\langle 4 \rangle$$

$$s_2(t) = p_{h2} x_l\left(t - \tau_2(t)\right) + x_{n2}\left(t - \tau_2(t); p_{h2}, p_{l2}\right) \qquad b)$$

where $p_{h1,h2}(t)$ are the HF pulses (**103/106**) with amplitudes $p_{h1,h2}$, and $p_{l1,l2}(t)$ are the LF pressure pulses (**102/106**) with amplitudes $p_{l1,l2}$. A change of polarity of the HF and LF pulses is then represented by a change in sign of $p_{h1,h2}$ or $p_{l1,l2}$. $\tau_{1,2}(t)$ are the nonlinear propagation delays of the HF pulses **103/106** as a function of the fast time t, produced by the nonlinear manipulation of the wave propagation velocity for the HF pulses by the LF pulses **102/105** in FIG. 1a. We define $p_l = p_{l2}/p_{l1}$ as the ratio of the two LF pulses which gives $\tau_2(t) = p_l\tau_1(t)$ for $2^{nd}$ order propagation elasticity. The nonlinearly scattered HF signals from the two pulse complexes **101** and **104** are $x_{n1}(...)$ and $x_{n2}(...)$. The linearly scattered signal is proportional to the HF amplitude and is listed as $p_{h1}x_1(t)$ and $p_{h2}x_1(t)$ for the HF pulses **103** and **106,** where $x_1(t)$ is the signature of the linearly scattered signal. For materials where the nonlinear elasticity can be approximated to the $2^{nd}$ order in the pressure, the nonlinearly scattered signal can be approximated as

$$x_{n1}\left(t; p_{h1}, p_{l1}\right) \approx p_{h1} p_{l1} x_n(t)$$

$$x_{n2}\left(t; p_{h2}, p_{l2}\right) \approx p_{h2} p_{l2} x_n(t)$$

By estimation of the nonlinear propagation delay $\hat{\tau}(t)$ as an estimate $\tau_1(t)$, for example as described in US Pat Appl US2005277835 and US2006052699, we can eliminate the linearly scattered term from Eq. (4) to produce an estimate of the nonlinear scattering

$$x_{ne}(t) = \frac{p_{h2} s_1\left(t + \hat{\tau}(t)\right) - p_{h1} s_2\left(t + p_l\hat{\tau}(t)\right)}{p_{h2} p_{h1} p_{l1} - p_{h1} p_{h2} p_{l2}}$$

$$= \frac{p_{h2} x_{n1}\left(t; p_{h1}, p_{l1}\right) - p_{h1} x_{n2}\left(t; p_{h2}, p_{l2}\right)}{p_{h2} p_{h1} p_{l1} - p_{h1} p_{h2} p_{l2}} = x_n(t) \qquad (6)$$

where the last equality is found with the $2^{nd}$ order elasticity approximation for the nonlinear scattering in Eq.(5).

The Mechanical Index (MI) is given by the negative pressure swing of the pulse complex and is often limited by safety regulations to avoid cavitation and destruction of the object. In such situations the amplitude of both the LF pulse **105** and the HF pulse **106** of the pulse complex **104** can have stronger limitations than the amplitudes for the pulse complex **101.** One then can have advantage of operating with lower amplitudes of the pulse complex **104** compared to pulse complex **101.** This difference in amplitudes is then taken care of by $p_{h2}$ and $p_{12}$. For practical reasons in the signal processing it can sometimes be interesting to invert the polarity of the second HF pulse **106** compared to the $1^{st}$ HF pulse **103**, a modification that is taken care of by a negative value of $p_{h2}$. The subtraction of the signals $p_{h2}s_1$ and $p_{h1}s_2$ in Eg. (4) is then changed to a sum. The coefficients $p_{h1/h2}$ and $p_{l1/l2}$ also take care of inaccuracies in the transmit amplifiers and modifications of the pulse amplitudes due to nonlinear propagation and diffraction, this modification being different from the positive and negative pulses. In case of inaccuracies in the transmitters, and unknown nonlinear propagation modification of the amplitudes, one can for example estimate $p_{h1}/p_{h2}$ and $p_{11}/p_{12}$ from a minimization of the

power in $x_{ne}$ as described in the US Pat Appl US2005277835 and US2006052699 and also in the combination with a minimization of the power in $x_{1e}$ of Eq.(13) described below.

The nonlinearly scattered signal has shown to enhance the 16 image contrast for

- soft scatterers (high nonlinear elasticity) as for example gas bubbles either found naturally or injected as contrast agent micro bubbles, liposomes and other fatty structures, polymers, wax on the inside wall of oil&gas pipes, etc., and
- hard scatterers (low nonlinear elasticity) as micro calcifications, gall- and kidney-stones, connective tissue, scales on the wall of oil and gas pipes, metal objects like mines buried in the sea bed, etc.

We see from Eq.(3) that the gradient in fast time (depth-time) of the estimated nonlinear propagation delay is

$$\frac{d\tau(t)}{dt} = \beta_n\left(r(t)\right)\kappa\left(r(t)\right)p_{LF}\left(r(t)\right) \tag{7}$$

where $r(t)$ is the integrated depth variable that for back scatter imaging is

$$r(t) = \int_{\Gamma(t)} c(\tau)d\tau \approx \frac{c_0 t}{2} \tag{8}$$

where the approximation is done with approximately constant propagation velocity $c_0$ for back scattered (divide by 2) signals. The gradient in Eq. (7) hence represents the local nonlinear propagation parameters of the object. The low frequency is so low that one can most often neglect individual variations in power absorption and wave front aberrations, and measure or calculate in computer simulations the LF pressure at the location of the HF pulse at any position along the beam. Dividing said local nonlinear propagation parameter with this estimated local amplitude of the manipulating LF pressure, one obtains a quantitative local nonlinear propagation parameter that represents local quantitative elasticity parameters of the object, as

$$np\left(r(t)\right) = \frac{1}{p_{LF}\left(r(t)\right)}\frac{d\tau(t)}{dt} = \beta_n\left(r(t)\right)\kappa\left(r(t)\right) \tag{9}$$

This parameter can be used to characterize the material, where for example in soft biological tissues fat or high fat content gives a high value of $\beta_n\kappa$.

The LF field simulation must however be done with defined parameters of the mass density $\rho$ and compressibility $\kappa$, and in the actual material one can have deviations from the parameters used for simulation, particularly in heterogeneous materials where the parameters have a spatial variation. When the load material has low characteristic impedance compared to the output mechanical impedance of the transducer elements, the vibration velocity of the element surface $u_{LF}(0)$ is close to independent of the load material characteristic impedance, and hence given by the electric drive voltage.

Assume that we carry through the simulations with a characteristic impedance $Z_S = \sqrt{\rho_S/\kappa_S} = \rho_S c_S = 1/\kappa_S c_S$ where the subscript S denotes values used for simulation. When the surface characteristic impedance deviates from the simulation value we get the pressure at the transducer element surface as $p_{LF}(0)=(Z(0)/Z_S)p_S(0)$ where $Z(0)$ is the characteristic impedance of the actual load material at the element surface. The intensity is related to the pressure as $I = p^2/2Z$, and when the characteristic impedance changes throughout the load material, the intensity is kept constant as scattering at the low frequencies is low and can be neglected, and one can then relate the actual pressure to the simulated pressure $p_S(r)$ as

$$p_{LF}(r) = \sqrt{\frac{Z(r)}{Z(0)}}\frac{Z(0)}{Z_S}p_S(r) = \frac{\sqrt{Z(r)Z(0)}}{Z_S}p_S(r) \tag{10}$$

As one do not know the actual material parameters, and one normalizes with $p_s(r)$ in Eq. (9) the quantitative nonlinear propagation parameter is related to the material parameters as

$$np(r) = \frac{p_{LF}(r)}{p_S(r)} \beta_n(r)\kappa(r) = \frac{\sqrt{Z(r)Z(0)}}{Z_S} \beta_n(r)\kappa(r) = \beta_n(r)\frac{c_S\kappa_S}{\sqrt{c(0)c(r)}}\sqrt{\frac{\kappa(r)}{\kappa(0)}} \qquad (11$$

One hence see that quantitative estimates of elasticity parameters can be obtained from the fast time gradient of estimates of the nonlinear propagation time lag. As such estimates are noisy, the gradient must be obtained through band limited differentiation, but one can also use a model based estimation where an estimate of the nonlinear propagation delay is estimated from simulations of the composite LF /HF pulse fields with given local elasticity parameters, and the parameters are adjusted in an estimation algorithm that produces the same simulated nonlinear propagation lag as the estimated one, according to known methods.

One can also obtain a local nonlinear scattering parameter as follows: The envelope of the nonlinearly scattered signal as a function of range r = $c_0t/2$ is proportional to

$$a_{ne}(r) = Env\{x_{ne}(2r/c_0)\} \sim k_{HF}^2 \upsilon_n(r)p_{LF}(r)G(r)\exp\left\{-2f_{HF}\int_0^r ds\mu(s)\right\} \qquad (12)$$

where $k_{HF} = \omega_{HF}/c_0$ is the wave vector for the center high frequency $\omega_{HF} = 2\pi f_{HF}$, $\upsilon_n(r)$ is a nonlinear scattering parameter for the HF band laterally averaged across the receive beam, $G(r)$ is a depth variable gain factor given by beam shape and receiver depth variable gain settings, and the exponential function represents ultrasound power absorption in the HF band. Under the assumption of a material with 2$^{nd}$ order elasticity where Eq. (5) is valid, the linearly scattered component can be extracted from the received HF signals in Eq. (4) as

$$x_{le}(t) = \frac{p_{h1}p_{l1}s_2(t + p_l\hat{\tau}(t)) - p_{h2}p_{l2}s_1(t + \hat{\tau}(t))}{p_{h1}p_{h2}(p_{l1} - p_{l2})} \qquad (13)$$

When $x_{n1}$ and $x_{n2}$ are of the more general form in Eq.(4) as found for micro-bubbles, the nonlinear component is usually small compared to the linear scattering from the tissue and is found only at the discrete locations of the micro-bubbles and so that the expression in Eq. (13) is a good approximation also in these cases. In fact, any of Eq. (4a,b) with delay correction can often be used as an estimate of the linearly scattered signal because the nonlinearly scattered signal is found at local points and often has much less amplitude than the linearly scattered signal. The envelope of the linearly scattered signal as a function of range r is similarly proportional to

$$a_{le}(r) = Env\{x_{le}(2r/c)\} \sim k_{HF}^2 \upsilon_l(r)G(r)\exp\left\{-2f_{HF}\int_0^r ds\mu(s)\right\} \qquad (14)$$

where $\upsilon_l(r)$ is a linear scattering parameter for the HF band and the other variables are as in Eq. (12).

We note that the linearly and nonlinearly scattered signal components have the same amplitude variation due to beam divergence/convergence and power absorption, and one can obtain a local nonlinear scattering parameter by forming the ratio of the envelopes $a_{ne}(r)$ and $a_{le}(r)$ as

$$\frac{a_{ne}(r)}{a_{le}(r)} = \frac{\upsilon_n(r)}{\upsilon_l(r)}p_{LF}(r) \qquad (15)$$

,

further dividing by an estimate of the local LF pressure as in Eq.(9), one obtains a quantitative local nonlinear scattering parameter as

$$ns(r) = \frac{a_{ne}(r)}{a_{le}(r)p_{LF}(r)} = \frac{\upsilon_n(r)}{\upsilon_l(r)} \qquad (16)$$

and using a simulated LF pressure we get

$$ns(r) = \frac{a_{ne}(r)}{a_{le}(r)p_S(r)} = \frac{p_{LF}(r)}{p_S(r)}\frac{v_n(r)}{v_l(r)} = \frac{\sqrt{Z(r)Z(0)}}{Z_S}\frac{v_n(r)}{v_l(r)} \qquad (17)$$

Variations in both the quantitative local nonlinear propagation parameter and the local nonlinear scattering parameter of the object with heating or cooling of the object can then be used to assess local temperature changes of the object, for example with thermal treatment of the object. The temperature dependency of the propagation velocity (dc/dT) of soft tissue in medical ultrasound, depends on the tissue composition where for fat or large fat content one has dc/dT < 0 while for other tissues, like liver parenchyma, one has dc/dT > 0 and predictable. One or both of $np \sim \beta_n \kappa$ from Eqs. (9, 11) and $ns \sim v_n/v_l$ from Eqs. (16, 17) can then be used to estimate the fat content and hence dc/dT in the tissue, so that variations in the propagation lag with heating or cooling can be used to assess temperature.

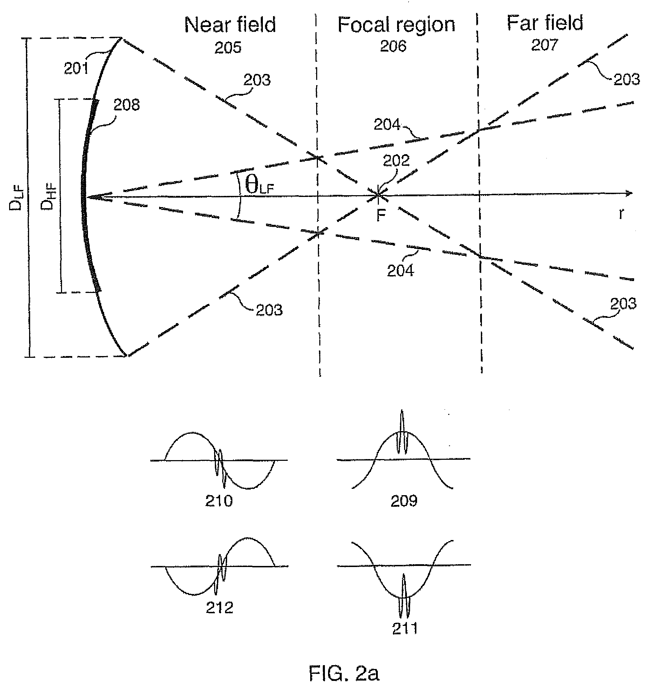

The phase relation between the HF and LF pulses as shown in FIG. 1a, can only be achieved over the whole propagation path for plane waves, which in practice means that the HF image range must be within the near field of the LF aperture. For practical, diffraction limited beams there will be a sliding between the relative position of the HF and LF pulses throughout the depth of the beam. This phenomenon is further described with reference to FIG. 2a, which shows typical LF and HF apertures 201 and 208 with diameters $D_{LF}$ and $D_{HF}$, respectively, both focused at 202. The lines 203 shows the geometric boundaries for a LF beam without diffraction, while 204 describes the LF beam diffraction cone with diffraction opening angle $\theta_{LF} = 2\lambda_{LF}/D_{LF}$ where $\lambda_{LF}$ is the LF wave length and $D_{LF}$ is the LF aperture diameter. The LF beam is essentially determined by the outer most of these boundaries, where in the near field region 205 the beam width reduces with depth by the geometric cone as it is wider than the diffraction cone, while in the focal region 206 the beam width expands with the diffraction cone where this is the widest, and further in the far field region 207 the beam expands again with the geometric cone. The LF pressure pulse in the focal point 202 is the temporal derivative of the LF pressure pulse at the transducer array surface, and this form of the LF pulse is a good approximation throughout the focal region. This differentiation provides a sliding of the position of the HF pulse relative to the LF pulse of $T_{LF}/4$, where $T_{LF}$ is the temporal period of the LF pulse.

The field from a flat (unfocused) LF aperture is given by the modification of FIG. 2a where the focal point 202 is moved to infinite distance from the aperture. The near field will then be a cylindrical extension of the LF aperture, and the diffraction limited far-field can be defined to start where the diffraction limited cone exceeds this cylindrical extension at $r = D_{LF}^2/2\lambda_{LF}$ . Inside this limit the LF field is well approximated by a plane wave, where a fixed phase relation between the HF and LF pulses as in FIG. 1a is maintained for the whole range.

The HF pulse undergoes a similar differentiation from the transducer array surface to its own focal region, but as the HF pulse is much shorter than the LF pulse, it is the differentiation of the LF pulse that affects the phase relationship between the HF and LF pulses. The receive HF beam do generally have dynamic focusing and aperture, where we mainly will observe the interaction between the HF and LF pulses close to the HF beam axis. For the HF pulse to be found at the crest of the LF pulse in the focal region, shown as 209, the HF pulse must due to the differentiation of the LF pulse in the focus be transmitted at the array surface at the zero crossing with the negative spatial gradient of the LF pulse, shown as 210. In the same way, for the HF pulse to be found at the trough of the LF pulse in the focal region, shown as 211, the HF pulse must at the array surface be transmitted at the zero crossing with the positive spatial gradient of the LF pulse, shown as 212. The position of the HF pulse within the LF pulse complex will hence slide $\lambda_{LF}/4$ from the array surface to the focal region. During the diffraction limited focal region the HF pulse will then be close to the crest or trough of the LF pulse. Beyond the focal region the HF pulse will again slide to the gradient of the LF pulse.

Hence, in the near field region where the HF pulse is found at a gradient of the LF pulse as illustrated in FIG. 1b, the different parts of the HF pulse will propagate with different velocities leading to a pulse compression for the HF pulse 107 that propagates on a negative spatial gradient of the LF pulse 108, because the tail of the pulse propagates faster than the front of the pulse. Similarly one gets a pulse stretching of the HF pulse 109 that propagates on a positive spatial gradient of the LF pulse 110, because the tail of the pulse propagates slower than the front of the pulse. With more complex variations of the LF pulse pressure within the HF pulse, one can get more complex distortions of the HF pulse shape. For example, if the HF pulse is long, or one have less difference between the high and low frequencies, one can get variable compression or stretching along the HF pulse, and even compression of one part and stretching of another part. One can then even get non-negligible distortion of the HF pulse when its center is located at a crest or trough of the LF pulse. As the pulse is distorted, the frequency content is changed which changes the diffraction. Absorption also increases with frequency and also produces a down-sliding of the pulse center frequency. The total pulse distortion is hence a combination of the spatial variation in the propagation velocity along the HF pulse produced by the nonlinear elasticity manipulation of the LF pulse, diffraction, and absorption.

We assume a dynamically focused receive beam that is so narrow that the variation of the transmitted, distorted HF pulse at a depth $r = ct_0/2$ is negligible across the receive beam (see comments after Eq.(3)). The effect of pulse form distortion on the received signal can then be modeled by a filter as

$$s_k(t) = \int dt_0 v_k(t - t_0, t_0) x_k(t_0 - \tau_k(t_0)) = s_{lk}(t) + s_{nk}(t)$$

(18)

$$x_k(t_0) = x_{lk}(t_0) + x_{nk}(t_0)$$

where $v_k(t, t_0)$ represents the change in frequency content of the received HF signal due to pulse form distortion. $x_k(t_0)$ represents the scattered signal that would have been obtained without pulse form distortion and nonlinear propagation delay at the fast time $t_0$ with a nonlinear propagation delay $\tau_k(t_0)$. $x_{lk}(t_0)$ is the linearly scattered signal that varies with k because the transmitted HF amplitude and polarity can vary, but also because diffraction and the power in harmonic components of the HF pulse can depend on the LF pulse. $x_{nk}(t_0)$ is the nonlinearly scattered signal for a transmitted LF pulse $p_{lk}(t_0)$, and it depends on the pulse number k because the LF pulse and potentially the HF pulse varies with k. For materials with 2nd order elasticity $x_{nx}(t_0)$ is proportional to the LF and HF amplitudes as in Eq. (5), while for micro-bubbles the nonlinear dependency is more complex where even the scattered HF pulse form from the micro-bubble can depend on the LF pressure as discussed in relation to Eqs. (35, 36) below. The linear effect of variations in the amplitude and polarity of the HF pulse can be included in $v_k(t,t_0)$, while the nonlinear effect is included in the k variation of $x_{nk}(t_0)$.

Even if there is some variation of the transmitted, distorted HF pulse across the HF receive beam, Eq.(18) gives an adequate signal model where $v_k(t,t_0)$ now represents an average pulse distortion across the beam with the weight of the scattering density. This weighting of the scattering density introduces a rapidly varying component of $v_k(t,t_0)$ with the depth time $t_0$, that depends on the randomly varying scattering density. This component can introduce added noise in estimates of $v_k(t,t_0)$ from the received signal.

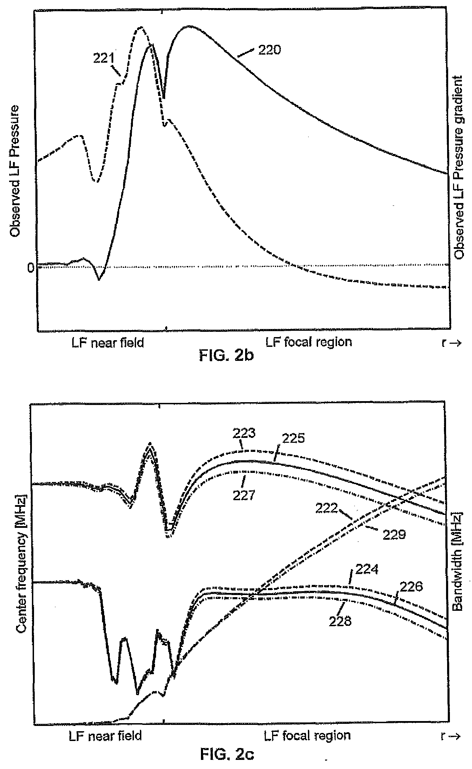

In **FIG. 2b** and **2c** are shown results of a simulation of the effect of the observed LF pressure and LF pressure gradient on the HF pulse as a function of depth. The HF pulse is placed at the negative spatial gradient of the LF pulse at the array surface. **220** shows the observed LF pressure at the center of the HF pulse, while **221** shows the observed LF pressure gradient at the center of the HF pulse, both as a function of depth r. The observed LF pressure is given by the LF pressure amplitude as a function of depth, influenced by diffraction and absorption, and the local position of the HF pulse relative to the LF pulse. In the near field the HF pulse is near a zero crossing of the LF pulse and the observed LF pressure is hence low, while the observed LF pressure gradient is high. As the pulse complex propagates into the focal region, the LF pressure amplitude increases and the HF pulse slides towards the crest of the LF pulse, which increases the observed LF pressure while the observed LF pressure gradient drops.

The resulting nonlinear propagation delay of the HF pulse for the positive LF pulse is shown as **222** in **FIG. 2c**, together with the center frequency **223** and the bandwidth **224** of the HF pulse. The center frequency and the bandwidth of the HF pulse for zero LF pulse is shown as **225** and **226** for comparison. We note that in the near field the observed LF pressure gradient produces an increase of both the center frequency and the bandwidth of the HF pulse that accumulates with depth, compared to the HF pulse for zero LF pressure. The nonlinear propagation delay is low as the observed LF pressure is low. As the pulse complex enters the LF focal region, the increased observed LF pressure produces an accumulative increase in the nonlinear propagation delay, **222,** while the drop in the pressure gradient along the pulse reduces the increase in the difference between the center frequency and bandwidth of the HF pulses. Moving into the far-field, the LF amplitude drops due to the geometric spread of the beam and both the observed LF pressure and pressure gradient at the HF pulse drops. The HF pulse also slides relative to the LF pulse to finally place the HF pulse at a zero crossing of the LF pulse, but this sliding is slower than in the near field.

The dash-dot curves **227** and **228** shows the mean frequency and the bandwidth of the HF pulse for opposite polarity of the LF pulse. The dash-dot curve **229** shows the negative nonlinear propagation delay, $-\tau_k(t)$, with opposite polarity of the LF pulse. In this example, the HF pulse is short compared to the LF wave length, so that the LF pulse gradient mainly produces a HF pulse length compression/expansion. The HF pulse bandwidth then follows the HF pulse center frequency as is clear in **FIG. 2c**. With longer HF pulses relative to the LF period there will be a more complex distortion of the HF pulse, where one needs more parameters than the center frequency and the bandwidth to describe the distortion, as discussed in relation to **FIG. 1b** above and in relation to Eq.(26).

The nonlinear propagation delay, Eq.(3), changes accumulatively with depth, which implies that the fast time variation of $\tau_k(t)$ is fairly slow. This allows us to do the following approximation

$$s_k(t) \approx z_{lk}\left(t - \tau_k(t)\right) + z_{nk}\left(t - \tau_k(t)\right)$$

$$(19)$$

$$z_{lk}(t) = \int dt_0 v_k\left(t - t_0, t_0\right) x_{lk}(t_0) \qquad z_{nk}(t) = \int dt_0 v_k\left(t - t_0, t_0\right) x_{nk}(t_0)$$

With the same approximation we can select an interval $T_1 = T(t_1)$ around $t_1$, and approximate $v_k(t,t_0) = v_k(t,t_1)$ in this interval. This allows us to do a Fourier transform of Eq.(19) as

$$S_k(\omega,t_l) = V_k(\omega,t_l) X_k(\omega) e^{-i\omega\tau_k(t_l)}$$

$$(20)$$

where we have also approximated $\tau_k(t_0) \approx \tau_k(t_1)$ over the interval $T(t_1)$. We then can device a pulse distortion correction filter, for example as a Wiener filter approximation to the inverse filter given by the formula

$$V_k(\omega,t_l)^{-1} \approx H_k(\omega,t_l) = \frac{1}{V_k(\omega,t_l)} \frac{1}{1 + N/\left|V_k(\omega,t_l)\right|^2}$$

$$(21)$$

where N is a noise power parameter to avoid noisy blow-up in $H_k(\omega,t_l)$ where the amplitude of $V_k(\omega,t_l)$ is low compared to noise in the measurement, while we get an inverse filter when the amplitude is sufficiently high above the noise, i.e.

$$H_k(\omega,t_l) \approx \begin{cases} \dfrac{1}{V_k(\omega,t_l)} & \text{for} \quad \left|V_k(\omega,t_l)\right|^2 \gg N \\[4mm] \dfrac{1}{N} V_k^*(\omega,t_l) & \text{for} \quad \left|V_k(\omega,t_l)\right|^2 \ll N \end{cases}$$

$$(22)$$

We hence can correct the received signal for the nonlinear propagation delay and pulse form distortion with the following filter

$$\hat{X}_k(\omega,t_l) = H_k(\omega,t_l) S_k(\omega,t_l) e^{i\omega\tau_k(t_l)}$$

$$(23)$$

Inverse Fourier transform of $H_k(\omega,t_l)$ in Eq. (21) gives a filter impulse response as $h_k(t,t_l)$. Fourier inversion of Eq. (23) and interpolation between $t_1$ gives an impulse response that is continuous in $t_1$, and we can write the nonlinear delay and pulse distortion corrected signal in the time domain as

$$\hat{x}_k(t) = \int dt_0 h_k\left(t - t_0, t_0\right) s_k\left(t_0 + \tau_k(t_0)\right)$$

$$(24)$$

The nonlinear delay correction can be included in the inverse filter impulse response $h_k(t-t_0,t_0)$, but for calculation efficiency it is advantageous to separate the correction for nonlinear propagation delay as a pure delay correction where the filter then corrects only for pulse form distortion, as the filter impulse response then becomes shorter. The signal is normally digitally sampled, and Eqs.(18-24) are then modified by the sampled formulas known to anyone skilled in the art. The length of $T(t_1)$ would typically be a couple of HF pulse lengths, and one would typically use intervals that overlap down to the density that $t_1$ represents each sample point of the received signal. When the pulse distortion can be approximated by a time compression-expansion, the received signal from a point scatterer can be written as $w_k(t,t_l;b_k)=u(b_k t,t_l)=\int dt_1 v_k(t-t_1,t_l)u(t_1,t_l)$ where $b_k = 1+a_k$ is the time compression factor and $u(t_1,t_l)$ is the undistorted received pulse from the point scatterer. We can then write

$$V_k(\omega,t_i;b_k) = \frac{U(\omega/b_k,t_i)}{b_k U(\omega,t_i)} \frac{1}{1+N_V\big/|U(\omega,t_i)|^2}$$

$$(25)$$

$$H_k(\omega,t_i;b_k) = \frac{U(\omega,t_i)}{U(\omega/b_k,t_i)} \frac{b_k}{1+N_H\big/|U(\omega/b_k,t_i)|^2}$$

where $U(\omega,t_l)$ is the Fourier transform of the undistorted received pulse form from depth interval $T_1$ obtained with zero LF. The LF pulse is often at such a low frequency that individual variations of the acoustic LF pulse absorption between different measurement situations can be neglected as discussed above. One can then use simulated or measured values of the LF pulse field at the location of the HF pulse, as a basis for estimation of the HF pulse form distortion $v_k(t,t_l)$. An uncertainty in this simulation is the actual nonlinear elasticity of the object. The invention devices several methods to assess the nonlinear elasticity, and also direct estimation of $Hk(\omega,t_l)$ from the signals as discussed following Eq.(52) below.

When the LF pressure gradient has larger variation along the HF pulse length so that the pure time compression/expansion is no longer an adequate approximation of the HF pulse distortion, we can do a polynomial modification of the time scale as

$$w_k(t,t_l;\underline{a}_k) = u\big((1+a_{k1})t - a_{k2}t^2 + a_{k3}t^3 - \ldots,t_l\big) = \int dt_1 v_k\big(t-t_1,t_l;\underline{a}_k\big)u\big(t_1,t_l\big)$$

$$V_k(\omega,t_i;\underline{a}_k) = \frac{W(\omega,t_i;\underline{a}_k)}{U(\omega,t_i)} \frac{1}{1+N_V\big/|U(\omega,t_i)|^2}$$

$$(26)$$

$$H_k(\omega,t_i;\underline{a}_k) = \frac{U(\omega,t_i)}{W(\omega,t_i;\underline{a}_k)} \frac{1}{1+N_H\big/|W(\omega,t_i;\underline{a}_k)|^2}$$

where $\underline{a}_k = (a_{k1},a_{k2},a_{k3},\ldots)$. After the correction for nonlinear propagation delay and pulse form distortion, the received signals from at least two pulse complexes can be combined as in Eqs.(6,13) to extract the nonlinearly and linearly scattered signal from the object.

In another method according to the invention, one can do a depth variable frequency mixing (Single Side band mixing, as known to anyone skilled in the art) of the received HF signal with nonzero transmitted LF pulse so that the center frequency of the HF signal for example is moved to the vicinity of the received HF signal with zero LF pulse. This frequency shift do not change the bandwidth of the received HF signal, where the bandwidth can be modified with a filtering of the frequency mixed HF signal. With large influence on the received HF signal bandwidth by the LF pulse, it can be convenient to frequency mix the received HF signals so that the center frequency of all mixed signals is close to the center frequency of the received HF signal with lowest bandwidth. The filtering correction of the bandwidth for the other signals is then composed of a reduction in bandwidth, which is a robust operation.

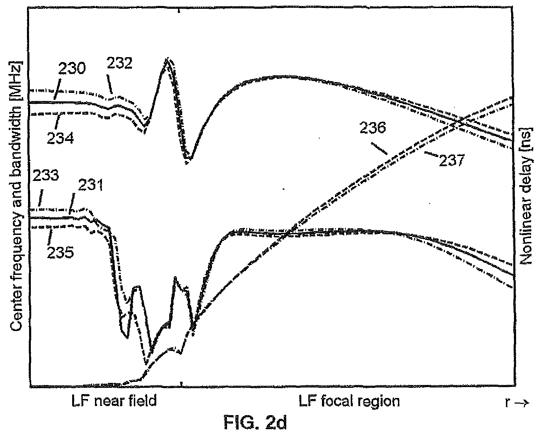

In yet another method according to the invention, one modify (e.g. stretch or compress or more complex modification) the transmitted HF pulse, so that the pulse distortion in front of the interesting imaging range (for example the LF focal range) modifies the pulses to similar pulses for positive and negative polarity of the LF pulse within the interesting imaging range. In **FIG. 2d** is shown the results of such variation in the transmit pulses, where **230** and **231** shows the depth variation of the center frequency and the bandwidth of the original transmitted HF pulse with zero transmitted LF pulse, **232** and **233** shows the center frequency and the bandwidth of time expanded transmitted HF pulse and how they are modified by the co-propagating positive LF pulse, while **234** and **235** shows the center frequency and the bandwidth of time compressed transmitted HF pulse and how they are modified by the co-propagating negative LF pulse. The nonlinear propagation delay for the positive LF pulse is shown as **236** and the negative nonlinear propagation delay for the negative LF pulse is shown as **237.** We note that both the center frequency and the bandwidth of the HF pulses are close to equal throughout the LF focal range. In the combination of the signals in Eq. (6) the linear scattering will then be highly suppressed for the interesting imaging range where the pulses for positive and negative polarity of the LF pulse have close to the same form. Due to diffraction, it is not possible to do a complete correction on transmit only, and a transmit correction combined with a receive correction composed of frequency mixing and/or filtering generally gives the best result. In the near field of **FIG. 2d** there is a difference in the HF pulses for positive and negative polarity of the LF pulse. If the signal in the near range is important, one can for example do a limited correction on transmit combined with a depth variable frequency mixing and/or filter correction on receive so that the received pulse is independent on the transmitted LF pulse over the interesting image range.

For imaging of nonlinear scattering, it is important that the LF pressure at the HF pulse has limited variation throughout the image range. The range where the variation of the LF pressure at the HF pulse has limited variation can be extended by using multiple transmit pulses with different focal depths for different regions of the image range. For close to constant observed LF pressure with depth it can be an advantage to use an unfocused LF beam with adequately wide transmit aperture so that the actual HF image range is within the LF beam near field.

In yet another method according to the invention, pulse distortion correction for pulse compression can for a limited fast time (depth-time) interval approximately be obtained by fast time expansion (stretching) of the received HF signal over that interval, and correction for pulse expansion can for a limited fast time (depth-time) interval approximately be obtained by fast time compression of the received HF signal over the limited interval. The reason why this pulse distortion correction is approximate is that it also changes the depth-time distance between scatterers over the given interval, so that the form of the signal determined by interference between scatterers at different depths becomes incorrect. We therefore can apply the time expansion/compression correction only to a limited fast time interval. For approximate pulse distortion correction over a longer fast time interval, this longer interval can be divided into shorter fast time intervals where different fast time expansion/compression is done on each interval. The corrected signals from different intervals can then be spliced to each other for example by a fade-in/fade-out windowing technique of the signals from neighboring intervals across the interval borders. There exist in the literature several methods for fast time expansion/compression of a signal, for example reading the signal in and out of a FIFO (First In First Out) memory structure at different read in and read out rates, interpolation of the signal and selecting different samples for the corrected output signal compared to the input signal, etc. We note that by the filter pulse distortion correction in Eqs. (23,24), the interference between the scatterers is modified by the correction because we modify the scattered pulse and not the distance between the scatterers. This modifies the envelope of the distorted signal to that obtained with no pulse distortion. The frequency mixing also changes the center frequency of the received HF signal without changing the distance between the scatterers, but the signal bandwidth is unmodified so that it is not correct after the frequency mixing.

The fast time expansion/compression method is particularly interesting for low pulse distortion where it is a good approximation and a simple and fast method. For larger pulse distortions, it can be advantageous to combine the above methods of transmit pulse correction, filter correction of the received signal, frequency mixing, and fast time expansion/compression of the received signal. This specially relates to regions of very high pulse distortion, where the frequency spectrum of the distorted pulse deviates much from the frequency spectrum of the undistorted pulse, which can make the filtering correction less robust. A limited fast time expansion/compressions of the received signal and/or frequency mixing and/or approximate correction of the transmitted HF pulse can then be used to bring the frequency spectrum of the limited corrected pulse closer to the frequency spectrum of the undistorted pulse, so that the filtering correction becomes more robust.

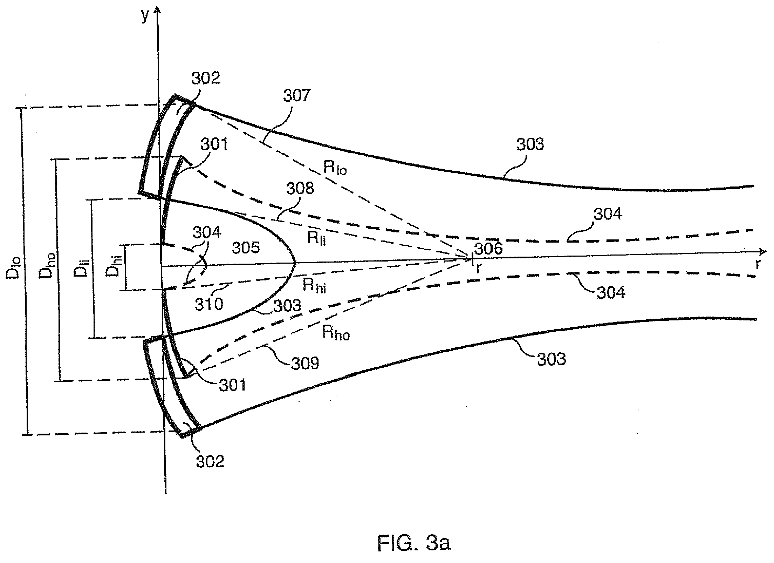

The LF wavelength is typically - 5 - 15 times longer than the HF wavelength, and to keep the LF beam adequately collimated to maintain the LF pulse pressure at deep ranges, the LF aperture is preferably larger than the outer dimension of the HF aperture. For the HF pulse one wants adequately long transmit focus, which limits the width of the HF transmit aperture. For further detailed analysis of this phase sliding it is convenient to study circular apertures where one have analytic expressions for the continuous wave fields (CW) along the beam axis. However, the basic ideas are also applicable to other shapes of the apertures, such as rectangular or elliptical shapes. We analyze the situation in **FIG. 3a** that shows a cross section of the HF (**301**) and LF (**302**) transducer arrays with indications of the boundaries of the HF beam **304** and LF beam **303**. For generality of the illustration we have chosen both an LF and HF aperture where the central part is removed, so that the inner and outer diameters of the LF aperture is $D_{li} = 2a_{li}$ and $D_{lo} = 2a_{lo}$ respectively, and the same for the HF aperture are $D_{hi} - 2a_{hi}$ and $D_{ho} = 2a_{ho}$ as shown in the Figure. The removed central part of the LF radiation aperture reduces the overlap between the LF and the HF beam in the near field, indicated as the near field region **305** where the LF field has low amplitude. The nonlinear elasticity manipulation by the LF pulse is therefore very low close to the beam axis in the near field region **305,** which reduces the near field nonlinear LF manipulation of the observed (close to the beam axis) transmitted HF pulse.

The continuous wave axial LF pressure field $P_l(r,\omega)$ with angular frequency $\omega = c_0 k$, is from a circular aperture at the point **306** (depth r) on the beam axis

$$P_l(r,\omega)=\frac{F}{r}\frac{e^{-ikR_{li}(r)}-e^{-ikR_{lo}(r)}}{F/r-1}P_{lt}(\omega) \qquad\qquad k=\omega/c_0$$

$$=i2e^{-ik(R_{lo}(r)+R_{li}(r))/2}\frac{F}{r}\frac{\sin k(R_{lo}(r)-R_{li}(r))/2}{F/r-1}P_{lt}(\omega) \tag{27}$$

where $P_{lt}$ is the LF transmit pressure on the array surface, $R_{lo}(r)$ is the distance **307** from the outer edge of the LF array to **306** on the z-axis and $R_{li}(r)$ is the distance **308** from the inner edge of the LF array to **306**. Similarly do we get the axial HF pressure field $P_h(r,\omega)$ at **306** as

$$P_h(r,\omega)=\frac{F}{r}\frac{e^{-ikR_{hi}(r)}-e^{-ikR_{ho}(r)}}{F/r-1}P_{ht}(\omega) \qquad\qquad k=\omega/c_0$$

$$=i2e^{-ik(R_{ho}(r)+R_{hi}(r))/2}\frac{F}{r}\frac{\sin k(R_{ho}(r)-R_{hi}(r))/2}{F/r-1}P_{ht}(\omega) \tag{28.}$$

where $P_{ht}$ is the HF transmit pressure on the array surface, $R_{ho}(r)$ is the distance **309** from the outer edge of the HF array to **306** on the beam axis-axis and $R_{hi}(r)$ is the distance **310** from the inner edge of the low frequency array to **306**.

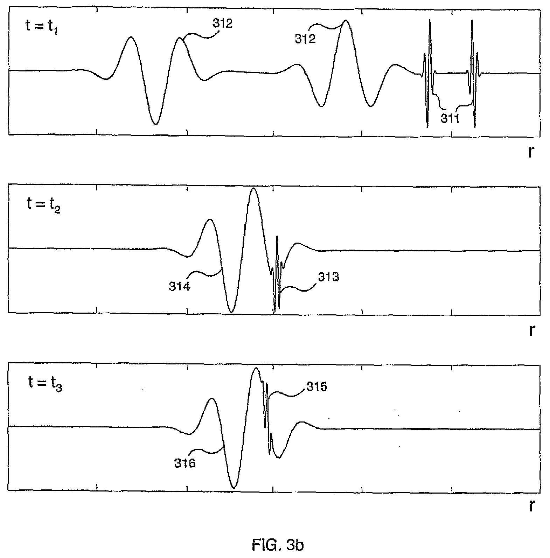

We note from the 1st lines of the expressions in Eqs.(27,28) that the pressure do in the near field break up into two pulses from the inner and outer edge of the apertures with delays $R_{li}(r)/c_0$ and $R_{lo}(r)/c_0$ for the LF pulse, and $R_{hi}(r)/c_0$ and $R_{ho}(r)/c_0$ for the HF pulse. This is illustrated in the upper panel of **FIG. 3b** where **311** shows the HF pulse and **312** shows the LF pulse at time point $t_1$ where the pulses are in the near field. The HF pulse has a center frequency of 10 MHz and the LF pulse has a center frequency of 1 MHz, both in biological tissue with assumed propagation velocity of 1540 m/sec. In the near field, absorption will reduce the pulse from the outer edge as this has the longest propagation distance to the axis. The same is found with apodization where the excitation amplitude reduces with distance from the axis.

As r increases, the delay difference between these pulses reduces, so that the two pulses start to overlap and form a single pulse as illustrated in the two lower panels of **FIG. 3b,** where **313** and **315** shows the HF pulses and **314** and **316** shows the LF pulses. In the overlap region the edge pulses interfere, both for the LF and HF waves. The interference can be both destructive, that reduces the amplitude, or constructive, that increases the amplitude in the overlap region. We hence get a pulse longer than the transmit pulses on the array surface $P_{lt}(\omega)$ and $P_{ht}(\omega)$ and with a complex internal shape that varies with the depth due to both constructive and destructive interference. In the focal zone, Taylor expansion of the second lines of Eqs.(27,28) shows that interference between the two pulses produces a pulse which approximates the time derivative ($ik = i\omega/c$) of the transmitted pressure pulses $P_{lt}(\omega)$ and $P_{ht}(\omega)$ at the transducer surface, as discussed above. The reason for the differentiation is the diffraction, which defines the focal region. As the LF wavelength is longer than the HF wavelength (typically - 5:1 - 15:1), the focal region where the differentiation of the transmitted pulse is found is typically longer for the LF than the HF pulse.

The pulse centers observe propagation lags from the transmit apertures to **306** for the LF and HF pulses as

$$\tau_l(r)=\frac{1}{2c_0}\big(R_{lo}(r)+R_{li}(r)\big) \qquad\qquad \tau_h(r)=\frac{1}{2c_0}\big(R_{ho}(r)+R_{hi}(r)\big) \tag{29}$$

where $\tau_l(r)$ is the propagation lag from the low frequency array to **306** and $\tau_h(r)$ is the propagation lag from the high frequency array to **306**. We hence see that for equal LF and HF transmit apertures with the same focus we have $R_{lo}(r)=R_{ho}(r)$ and $R_{li}(r)=R_{hi}(r)$ so that relative propagation lag between the HF and LF pulse centers becomes zero and do not vary with depth, i.e.

$$\Delta\tau(r)=\tau_l(r)-\tau_h(r)=0 \tag{30}$$

The differentiation of the transmitted LF pressure pulse $P_{lt}(\omega)$ towards the focus produces an added time advancement of the LF oscillation of $T_{LF}/4$, where $T_{LF}$ is the temporal period of the LF pulse center frequency. With absorption and/or apodization, the pressure close to the array surface is a replica of the transmitted pulse complex with a propagation delay given by the phase propagation of the LF wave front. We hence see that if we want a specific phase relationship between the LF and the HF pulse in the focal region of the LF beam, we must transmit the HF pulse a time $T_{LF}/4$ earlier in relation to the LF pulse than the relation we want in the LF focal zone, as discussed in relation to **FIG. 2a-d.** With adequately wide LF aperture, the LF pulse will in the near field be close to a replica of the LF pulse at the transmit surface, and we can design the LF aperture so that the phase relationship between the HF and LF pulses is close to the same throughout the whole LF near field region.

One can also design the LF transmit aperture so much larger than the HF transmit aperture that in the near field $\tau_h(r)$ is sufficiently less than $\tau_l(r)$ so that by adequate timing between the transmit of the HF and LF pulses, the HF pulse is spatially in front of (i.e. deeper than) the LF pulse with no overlap in the near field. An example of such a situation illustrated in **FIG. 3b.** For this location of the pulses the near field LF manipulation pressure observed by the HF pulse is efficiently zero.

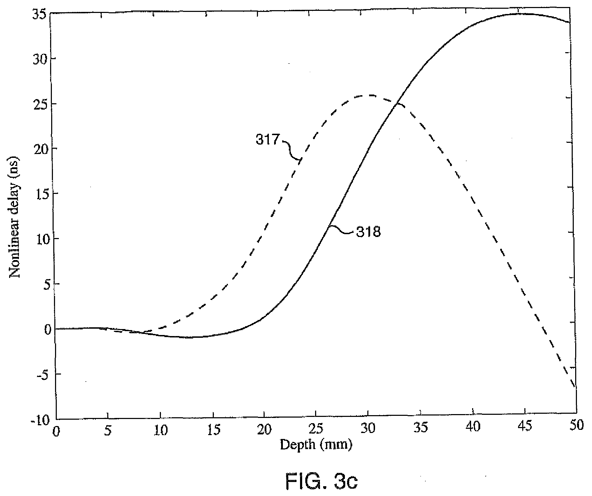

As r increases, and the time lag $\Delta\tau(r) = \tau_l(r)-\tau_h(r)$ between the pulses is reduced, the HF pulse eventually slides into the LF pulse and starts to observe a nonlinear elasticity manipulation pressure of the LF pulse. For example, at the time point $t_2 > t_1$ the pulses have reached the relative position illustrated by **313** for the HF pulse and **314** for the LF pulse. This observed LF manipulation pressure for the HF pulse, produces a nonlinear propagation delay of the HF pulse. Further propagation to a time point $t_3 > t_2$ the HF pulse **315** has undergone further sliding relative to the LF pulse **316,** where the HF pulse is now on the gradient of the LF pulse that produces a time compression of the HF pulse. An example of two developments with depth of the nonlinear propagation delays are shown in **FIG. 3c**, where **317** and **318** show the development with depth of the nonlinear propagation delay for two different transmit time lags between the HF and LF pulses. We note that the nonlinear propagation delay for **318** is close to zero up to 20 mm depth, and reaches a maximum of 17 nsec at 45 mm depth, where for 10 MHz $T_0/4 = 25$ nsec. Due to limited overlap between the HF and LF pulses, the HF pulse form distortion is also low in the near field. After subtraction of the received HF signal for positive and negative LF pulse, this curve provides a strong suppression of pulse reverberation noise where the 1st scatterer is up to 20 mm depth, with a gain of the 1st scattered signal at 45 mm depth of $2\sin(\omega_0\tau) \approx 1.75\sim4.87$ dB. As the uncorrelated electronic noise increase by 3dB in the subtraction, this gives an increase in signal to electronic noise ratio of 1.87 dB at deep ranges, with strong suppression of pulse reverberation noise relative to imaging at the fundamental frequency. Due to the steering of the near field manipulation, the suppression of the pulse reverberation noise is much stronger than for 2nd harmonic imaging, with a far-field sensitivity better than 1st harmonic imaging.

The curve **317** has a strong suppression of pulse reverberation noise with the 1st scatterer down to 10 mm, where the max gain after subtraction is found at ~ 30 mm. We hence see that by adequate selection of the HF and LF radiation surfaces and timing of the LF and HF transmit pulses, we can modify the near field range of the 1st scatterer where strong suppression of the pulse reverberation noise is found, and also the region of large gain of the 1st scatterer after the subtraction.

The sliding of the HF and LF pulses with this method do however produce pulse distortion in depth regions where the gradient of the LF pulse along the HF pulse is sufficiently large, for example as illustrated in FIG. 1b and the lower panel of **FIG. 3b.** For suppression of pulse reverberation noise, for example by subtracting the received HF signals from two pulses with opposite polarity of the LF pulse, the pulse form distortion in the image range do not produce great problems, once it is limited to deep ranges. The most important feature for the suppression of pulse reverberation noise is a large near field region with very low observed LF manipulation pressure and pressure gradient, with a rapid increase of the observed LF manipulation pressure with depth followed by a gradual attenuation of the LF manipulation pressure so that $\omega_0\tau(r)$ rises to the vicinity of $\pi/2$ and stays there, or a rapid increase in observed LF pressure gradient that produces pulse distortion. However, to suppress the linearly scattered signal to obtain the nonlinearly scattered signal, for example according to Eq. (6), the pulse distortion produces problems as discussed above. It can then become important to do pulse distortion correction according to the discussion above, for example through fast time expansion/compression of the received HF signal, or through filtering as in Eqs. (18-24), or through modification of the transmitted HF pulses to counteract the propagation pulse form distortion, or a combination of the two, as described above. These considerations give a scheme for optimizing the HF and LF radiation surfaces and their foci together with the signal processing for maximal suppression of pulse reverberation noise and enhancement of nonlinear scattering signals, depending on the application.

Hence, we can arrange the transmit time relation between the HF and LF pulses so that the HF pulse is found at a wanted phase relation to the LF pulse in the focal zone. When the HF pulse is found at pressure extremes of the LF pulse in the focal zone, the nonlinear elasticity manipulation by the LF pulse observed by the HF pulse then mainly produces a nonlinear propagation delay with limited pulse form distortion. This phase relation also maximizes the scattered signal from non-linear scatterers and resonant scatterers where the LF is adequately far from the resonance frequency. When the HF pulse is found near zero crossings of the LF pulse with large observed pressure gradient, one obtains pulse distortion with limited increase in nonlinear propagation delay. However, in the near field the HF pulse will be longer so that parts of the pulse can observe a LF pressure gradient with limited LF pressure, while other parts can observe a limited LF pressure gradient with larger observed LF pressure. This produces a complex pulse distortion of the HF pulse as deiscussed in relation to Eq.(26).

For a common focal depth F, we get the distances from outer and inner edges of the LF and HF apertures as

$$R_{go}(r) = \sqrt{r^2 + 2e_{go}(F-r)} \qquad e_{go} = F - \sqrt{F^2 - a_{go}^2} \approx \frac{a_{go}^2}{2F} \qquad g = l,h$$

$$R_{gi}(r) = \sqrt{r^2 + 2e_{gi}(F-r)} \qquad e_{gi} = F - \sqrt{F^2 - a_{gi}^2} \approx \frac{a_{gi}^2}{2F} \qquad g = l,h$$

(31)

when the last terms under the root sign are relatively small, we can approximate

$$R_{go}(r) \approx r + \frac{F-r}{2Fr} a_{go}^2 \qquad R_{gi}(r) \approx r + \frac{F-r}{2Fr} a_{gi}^2 \qquad g = l,h$$

(32)

The r variation of the propagation lag difference between the LF and HF pulses is then found by inserting Eqs. (31,32) into Eq.(30) which gives

$$\Delta\tau(r) = \frac{1}{2c_0} \frac{F-r}{2Fr} \left( a_{lo}^2 + a_{li}^2 - a_{ho}^2 - a_{hi}^2 \right)$$

(33)

Hence, by choosing

$$a_{ho}^2 + a_{hi}^2 = a_{lo}^2 + a_{li}^2$$

(34)

we obtain within the approximation zero sliding between the HF and LF pulses in the focal range of the LF pulse, even in the situation where the outer dimension of the LF transmit aperture is larger than the outer dimension of the HF aperture.

A disadvantage with the removed central part of the HF transmit aperture is that the side lobes in the HF transmit beam increase. However, these side lobes are further suppressed by a dynamically focused HF receive aperture. The approximation in Eq. (32) is best around the beam focus, and Eg. (34) do not fully remove phase sliding between the LF and HF pulses at low depths. For other than circular apertures (for example rectangular apertures) one does not have as simple formulas for the axial field as in Eq. (27,28) but the analysis above provides a guide for a selection of a HF transmit aperture with a removed center, for minimal phase sliding between the LF and the HF pulses with depth. With some two-dimensional arrays one can approximate the radiation apertures with circular apertures where Eq. (34) can be used as a guide to define radiation apertures with minimal phase sliding between the LF and HF pulses.

One can hence also design the LF and HF transmit apertures so that one gets very little sliding between the HF and LF pulses in an image range, and one can position the HF pulse at the extremes (crest or trough) of the LF pressure pulse so that one gets little pulse form distortion in this image range. Apodization of the LF drive pressure across the array is also useful for best shape of the LF pulse throughout the image range. One hence has a situation where optimal signal processing also involves the design of the LF and HF beams in detail together with pulse distortion correction, delay correction, and potential modification of the HF transmit pulse-form to counteract partially or in full the pulse form distortion that is produced by the propagation.

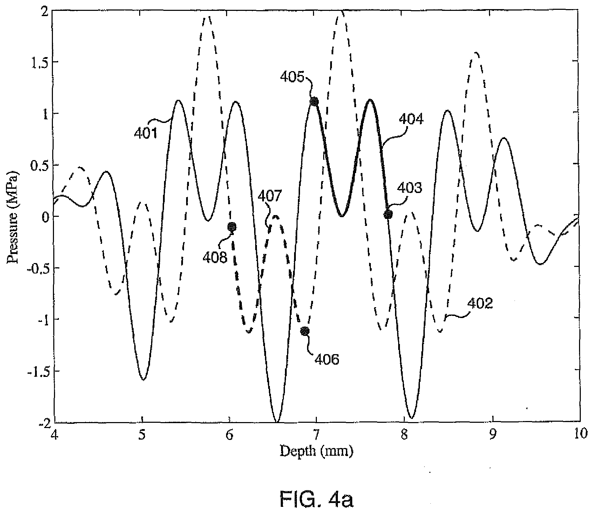

The variation in pulse distortion between positive and negative polarity of the LF pulse can according to the invention be reduced by using LF pulses with dual peaked spectra, for example by adding a 2nd harmonic band to the fundamental LF pulse band, as illustrated in **FIG. 4a**. This Figure shows a positive polarity LF pulse complex **401** and a negative polarity LF pulse complex **402** which both are obtained by adding a 2nd harmonic component to the fundamental LF band. For the positive LF pulse complex the HF pulse slides with propagation along the path **404,** for example from a near field position **403** to a far field position **405**. For the negative polarity LF pulse **402** one can place the near field position of the HF pulse so that the HF pulse slides with propagation along the path **407,** for example from a near field position **406** to a far field position **408**. The HF pulse hence slides along similar gradients of the LF pulse for both the positive and negative polarity LF pulse, and similar pulse form distortion is therefore produced for both LF polarities. Other position sliding of the HF pulse along the LF pulse that produce close to the same pulse form distortion for the positive and negative LF pulse, can be obtained with different transmit beam designs, as discussed in relation to **FIG.**

**3a.** When the pulse form distortion is the same for the positive and negative LF pulse, it is less important to correct for this distortion both to suppress multiple scattering noise, and to suppress linear scattering to provide the nonlinear scattering component, while correction for nonlinear propagation delay must still be done.

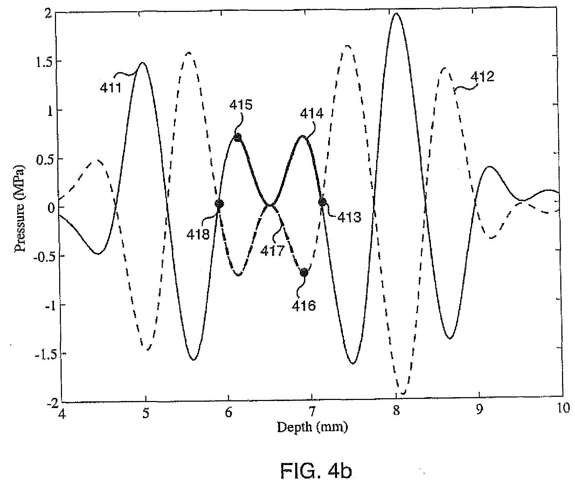

A variation of this type of LF pulse is shown in **FIG. 4b** where a frequency band around 1.5 times the center frequency of the fundamental band of the LF pulse is added. The positive polarity LF pulse is shown as **411** and the negative polarity LF pulse is shown as **412**. For the positive polarity LF pulse the HF pulse slides along the path **414,** for example from a near field position **413** until a far-field position **415**. Similarly, for the negative LF pulse the transmit timing between the HF and LF pulse is modified so that the HF pulse slides along the path **417,** for example from a near field position **416** until a far field position **418**. The HF pulse also in this case slides along similar gradients of the LF pulse for both the positive and negative polarities of the LF pulse so that similar pulse form distortion for both LF polarities is obtained similarly to the situation exemplified in **FIG. 4a.**

In the same way as the invention devices the use of LF pulses with different degree of complex shape, it is also interesting to use HF pulses of more complex shape as that shown in FIG. 1a - 1c, for example the use of longer, coded pulses, such as Barker, Golay, or chirp coded pulses, with pulse compression in the receiver to regain depth resolution, according to known methods. The longer transmitted pulses allow transmission of higher power under amplitude limitations, for example given by the MI limit, and hence improve signal to electronic noise ratio with improved penetration, without sacrificing image resolution. However, pulse form distortion will be more pronounced with the longer HF pulses as discussed in relation to Eq.(26), and should be corrected for for best possible results.

Suppression of the linear scattering to subtract nonlinear scattering is particularly useful to image micro-gas bubbles, for example in medical imaging the ultrasound contrast agent micro-bubbles as described in US Pat Appl US2005277835 and US2006052699. The micro-bubbles have a resonant scattering introduced by interaction between the co-oscillating fluid mass around the bubble (3 times the bubble volume) and the gas/shell elasticity. This resonant scattering is different from scattering from ordinary soft tissue. For an incident continuous LF wave with angular frequency $\omega_{LF}$ and low-level amplitude $P_{LF}$, the transfer function from the incident LF pressure to the radius oscillation amplitude $\delta a$ is in the linear approximation

$$\delta a = -\frac{K_0}{1-\left(\omega_{LF}/\omega_0\right)^2 + i2\zeta\left(\omega_{LF}/\omega_0\right)}P_{LF} \qquad (35)$$

where $\omega_0$ is the bubble resonance frequency for the equilibrium bubble radius $a_0$, $\zeta$ is the oscillation losses, $K_0$ is a low frequency, low amplitude radius compliance parameter. Resonance frequencies of commercial micro-bubble contrast agents are 2 - 4 MHz, but recent analysis [4] indicates that the resonance frequency may be reduced in narrow vessels below 70 $\mu$m diameter, and can in capillaries with 10 $\mu$m diameter reduce down to 25 % of the infinite fluid region resonance frequency (i.e. down to $\sim$ 0.5 - 1 MHz).

Two nonlinear effects modifies the oscillation as: 1) The low frequency compliance increases with compression where the bubble compliance is a function of the compression, f.ex. modeled as the function as $K(a_c)$, where $a_c$ is the compressed radius of the bubble so that $K(a_0) = K_0$ in Eq.(35). 2) The small amplitude resonance frequency increases with bubble compression, because the co-oscillating mass is reduced and the bubble stiffness increases. For the same physical reason (opposite effect) the small amplitude resonance frequency reduces with bubble expansion. These two effects introduce nonlinear distortions in the bubble oscillations, and for short pulses with a wide frequency spectrum, the bubble radius oscillation waveform can deviate much from the incident pressure pulse. The nonlinear compression of the bubbles therefore often deviates strongly from the 2nd order approximation of Eq.(1). The ratio of the LF to HF pulse frequencies is typically $\sim$ 1:5 - 1:15, and typical HF imaging frequencies are from 2.5 MHz and upwards. One can therefore have situations where the center frequencies of either the HF or the LF pulses are close to the bubble resonance frequency. When the low frequency is close to the resonance frequency of ultrasound contrast agent micro-bubbles, one gets a phase lag between the incident LF pressure and the radius oscillation of micro-bubble that gives an interesting effect for the nonlinear scattering.

The linear approximation of the scattered HF pulse amplitude $P_s(r)$ at distance r from the bubble, is for an incident CW HF wave with angular frequency $\omega_{HF}$ and low level amplitude $P_{HF}$

$$P_s(r) = \frac{a_c}{r}\frac{\left(\omega_{HF}/\omega_c(a_c)\right)^2}{1-\left(\omega_{HF}/\omega_c(a_c)\right)^2 + i2\zeta\left(\omega_{HF}/\omega_c(a_c)\right)}P_{HF} \qquad (36)$$

where $\omega_c(a_c)$ is the bubble resonance frequency where the bubble radius is changed to $a_c$ by the LF pulse. The nonlinear detection signal from the bubble, e.g. Eq.(6), is proportional to the difference between the scattered HF pulses for different LF pulses of the at least two pulse complexes. As for the LF bubble oscillation we get a nonlinear effect both from compression variation of the resonance frequency and from nonlinear elasticity of the bubble. However, when the HF frequency is well above the resonance, the HF oscillation amplitude is reduced and the linear approximation in Eq.(36) is good. The scattered HF signal is in the linear approximation $\sim - a_c(P_{LF})P_{HF}$, so that the nonlinear detection signal from the bubble is in this linear approximation $-\Delta a_c(P_{LF})P_{HF} = - [a_c(P_{LF}) - a_c (-P_{LF})]P_{HF}$. To get maximal detection signal the HF pulse must be close to the crest or trough of the LF bubble radius, when it hits the bubble. We also note that nonlinearity in the LF bubble oscillation will show directly in the detection signal, where the LF nonlinearity often is much stronger than the 2nd order approximation of Eq.(1).

With larger HF amplitudes the detection signal can also have a nonlinear relation to the HF pressure amplitude. When the HF frequency approaches the bubble resonance frequency, the variation of $\omega_c(a_c)$ with the LF pressure introduces a phase shift of the HF pulses from the different pulse complexes with differences in the LF pulse. This further increases the nonlinear detection signal from the bubble and makes a further deviation from the 2nd order elasticity approximation in Eq.(6). The HF detection signal hence has increased sensitivity for HF around the bubble resonance frequency.

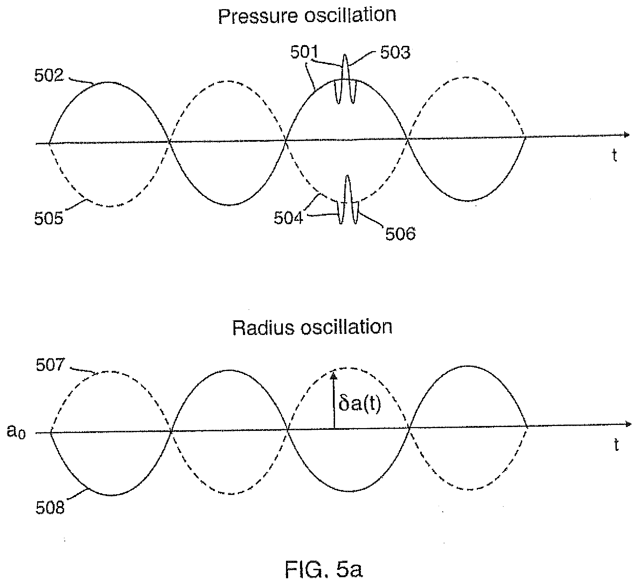

For LF well below the bubble resonance frequency, the linear approximation of Eq. (35) gives $\delta a \approx -K(P_{LF})P_{LF}$. This situation is illustrated in **FIG. 5a** which shows in the upper panel incident HF and LF pulse complexes **501,** composed of the LF pulse **502** and the HF pulse **503,** and **504** composed of the LF pulse **505** and HF pulse **506.** The lower panel shows the radius oscillation $\delta a \approx -KP_{LF}$ produced by the incident LF pulse, where the LF pressure **502** produces the solid radius oscillation curve **508,** and the LF pressure **505** produces the dashed radius oscillation curve **507.** To produce maximal HF bubble detection signal, the HF pulse must hit the LF radius oscillations at a maximum and a minimum, which is found when the HF pulse is located at the trough (**506**) and crest (**503**) of the LF pressure pulses. For LF frequencies well below the bubble resonance frequency, the transmit timing between the HF and LF pulse should therefore be so that the HF pulse is found at the crest and trough of the LF pressure pulse in the interesting image region.

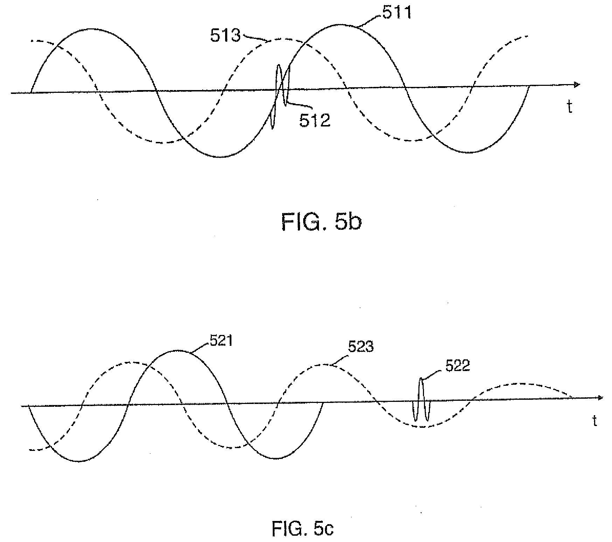

When the LF is at the bubble resonance frequency, we see from Eq. (35) that the bubble radius oscillation is 90 deg phase shifted in relation to the incident LF pulse, as illustrated in **FIG. 5b**. This Figure shows as **511** an incident LF pressure oscillation, with the resulting LF radius oscillation as **513** (dashed). For the HF pulse to hit the bubble at a maximal radius, the HF pulse must be placed at the negative to positive temporal zero crossing of the LF pulse. Switching the polarity of the incident LF pressure pulse then positions the HF pulse **512** at the positive to negative temporal zero-crossing of the LF pulse, which is then found at the trough of the LF radius oscillation. The HF pulse must then at least for the imaging distance propagate on the maximal positive or negative gradient of the LF pressure, which produces HF pulse form distortion that must be corrected for to maximally suppress the linear scattering from the tissue. As the radius oscillations have a peak at the resonance frequency one then gets the most sensitive detection signal from the bubbles with resonance frequency close to the frequency of the LF pulse. For bubbles with resonance frequency at a distance from the LF, this phase relationship between the HF and LF pulses implies that the HF pulse hits the bubble when the radius passes through the equilibrium value $a_0$ for these bubbles, and the two pulses hence have very small difference in the scattered HF signal from these bubbles with a subsequent very low detection signal.

For LF frequencies well above the bubble resonance frequency we have $\delta a \approx KP_{LF}(\omega_0/\omega_{LF})^2$, which produces low $\Delta a_c(P_{LF})$ and detection signal. To get a good detection signal from the bubble, one hence wants the LF frequency to be from slightly above the bubble resonance frequency and downwards. HF imaging frequencies are generally from ~ 2.5 MHz up to ~ 100MHz and as the LF to HF pulse frequencies have ratios ~ 1:5 - 1:100, the LF frequency can get close to the bubble resonance frequency for HF frequencies of ~ 2.5 MHz and upwards, if we include the ~ 0.5 MHz resonance frequencies of bubbles confined in capillaries. For HF imaging frequencies in the range of 10 MHz and upwards, it is possible to place the LF frequency around typical resonance frequencies of commercially available contrast agent micro bubbles, and one can then do tuned resonant detection by tuning the LF to any value within the actual resonance of the bubbles.

Selecting a phase relation between the transmitted HF and LF pulses so that the HF pulse is found at the maximal positive or negative gradient of the LF pulse in the actual image range, provides a resonant sensitive detection of the micro-bubbles, with an increased imaging sensitivity for the micro-bubbles with resonance frequency close to the LF. As the LF pulse in the LF focal range is the time derivative of the transmitted LF pulse per the discussion in relation to **FIG. 2a** and also following Eqs.(27,28), it means that we should transmit the HF pulse at the crest or trough of the LF pulse to get the phase relationship of **FIG. 5b** in the focal LF region. The detection signal from bubbles with so high or low resonance frequencies that the phase of the transfer function in Eq. (34) is either $\pi$ or 0 for the LF pulse, will then be highly suppressed. This is different from the resonant enhancement of the detection signal from the bubbles when the HF gets close to the resonance frequency (LF then well below resonance frequency), as in the former case there is a substantial detection signal also when the HF is well above the bubble resonance frequency. Selecting for example LF around - 0.5 ~ 1 MHz, which can be done with HF imaging frequencies from ~ 2.5 MHz and upwards, will provide an increased sensitivity for imaging micro-bubbles confined in capillaries where the bubble resonance is reduced to ~ 1 MHz, compared to larger vessels and cavities where the bubble resonance is the 3 - 4 MHz as in an infinite fluid. Mono-disperse micro-bubbles with a sharp resonance frequency in infinite fluid are under development. The reduction in bubble resonance introduced by the confining motion of smaller vessels can then combined with the LF resonant detection be used for selective detection of bubbles in different vessel dimensions. Tissue targeted micro-bubbles are also in development, and the resonance frequency for such bubbles also changes when the bubbles attach to tissue cells. The LF resonant detection of the micro bubbles can hence be used for enhanced detection of the bubbles that have attached to the cells.

Another method to obtain a resonance sensitive detection of the micro-bubbles is to select the frequency of the LF pulse close to the bubble resonance frequency and transmit the HF pulse with a delay after the LF pulse. When the LF is close to the bubble resonance frequency, the bubble radius oscillation will ring for an interval after the end of the incident LF pulse. The HF pulse delay is selected so that in the actual imaging range the transmitted HF pulse is sufficiently close to the tail of the transmitted LF pulse so that the HF pulse hits the resonating bubble while the radius is still ringing after the LF pulse has passed, preferably at a crest or trough of the radius oscillation, as shown in **FIG. 5c**. This Figure shows in the time domain by way of example in the actual imaging range the incident LF pressure pulse as **521** with a HF pulse **522** following at the tail of the LF pulse. The radius oscillation excited by the LF pressure is shown as **524** where it is observed that due to the resonance the radius oscillates (rings) for some time period after the end of the exciting LF pressure pulse. With the example phase arrangement between the HF and LF pulses, the HF pulse **522** hits the bubble when it has minimal radius produced by the LF pressure. The HF signal from the tissue is unaffected by the LF pulse. One then typically transmits two or more pulse complexes where the LF pulse varies for each pulse complex, typically in phase and/or amplitude and/or frequency, and combines the received HF signals from the at least two pulses to suppress the linear HF scattering from the tissue and enhance the HF nonlinear bubble scattering.

The resonance sensitive detection has interesting applications for molecular imaging with targeted micro-bubbles where micro-bubbles with different, mono disperse diameters are coated with different antibody ligands. With resonance frequency selective imaging, one can then determine which antibodies has produced attachment to specific tissues, and hence characterize potential disease in the tissue.

In the near field, the outer edge wave from the LF aperture will arrive at the beam axis with a delay compared to the LF pulse from the more central part of the LF aperture, depending on the apodization and width of the LF aperture. The outer edge wave hence extends the tail of the LF pulse in the near field, and for the HF pulse to be sufficiently close to the LF pulse to observe the bubble radius ringing in the actual imaging range, one can, depending on the arrangement of the HF and LF apertures, have an overlap between the HF and LF pulses in the near field. This overlap introduces a nonlinear propagation delay and potential pulse distortion of the HF pulse. However, for deeper ranges when the outer edge pulse from the LF aperture merges with the central LF pulse (see discussion above) the HF pulse is found behind the LF pulse without any nonlinear modification on the propagation of the HF pulse. Hence, with this method one can get considerable suppression of the linearly scattered signal from the tissue without correcting for nonlinear pulse distortion and/or nonlinear propagation delay of the HF pulse. However, when the HF pulse experiences an overlap with the LF pulse in the near field, one can obtain improved suppression of the linearly scattered signal from tissue by correcting the HF signal for nonlinear pulse distortion and/or propagation delay from the overlap region, or modify the transmitted HF pulses so that combined with the near field pulse distortion one gets the same form of the HF pulses in the imaging region for different variations of the LF pulse.

Multiple scattering of the HF pulse produces acoustic noise, which reduces image quality and produces a problem for the suppression of the linear scattering. Current ultrasound image reconstruction techniques take as an assumption that the ultrasound pulse is scattered only once from each scatterer within the beam (1st order scattering). In reality will the 1st order scattered pulse be rescattered by a 2nd scatterer producing a 2nd order scattered wave that rescattered by a 3rd scatterer (3rd order scattered wave) etc. The 1st scatterer will always be inside the transmit beam. Forward scattering will follow the incident wave and will not produce disturbing noise, but when the subsequent scattering (2nd scatterer) is at an angle from the forwards direction (most often back scattering) the multiply scattered waves where the last scatterer is inside the receive beam will produce signals with an added delay which appears as acoustic noise. To observe the last scatterer within the receive beam, one will only observe odd order scattering, which we generally refer to as pulse reverberation noise. Since the pulse amplitude drops in each scattering, it is mainly the 3rd order scattering that is seen as the multiple scattering or pulse reverberation noise.

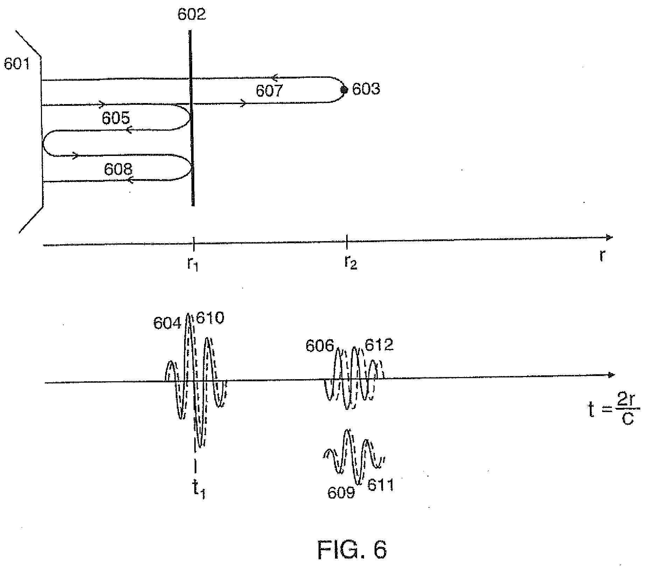

To suppress pulse reverberation noise, one can for example according to US Pat Appl US2005277835 and US2006052699 transmit two pulses with different phase and/or amplitude and/or frequency of the LF pulses, and subtract the received HF signal from the two pulses. An example of a situation that generates strong pulse reverberation noise of this type is shown in **FIG. 6a** which shows a transducer array **601** and a strong reflecting layer **602** at depth $r_1$ in the object, for example a fat layer, and an ordinary scatterer **603** at $r_2$, about twice the depth of **602** for the example. A transmitted ultrasound pulse observes a 1st reflection at **602** to give the received HF pulse **604** following the indicated path **605**. The pulse is further partly transmitted at **602** and observes a 1st order scattering from **603** to give the received HF pulse **606** along the 1st order scattering path indicated as **607.** The 1st order scatering from **602** observes a 2nd

reflection from the transducer with a 3rd scattering from **602** along the path indicated as **608** to give the received HF pulse **609** at about the same time lag as **606**, the 1st order scattering from **603**, and appears hence as noise in the image.

At the 1st reflection from the layer **602** the amplitude of the LF pulse (and also the HF pulse) drops so much that the nonlinear manipulation of the propagation velocity for the HF pulse by the LF pulse can be neglected after the 1st reflection. The nonlinear propagation lag and also pulse form distortion for the 3rd order scattered pulse **609** is therefore about the same as for the 1st order scattered pulse **604** from the layer **602**. Changing the polarity of the LF pulse will then produce pulses **610** and **611** from the 1st and 3rd reflection from the layer **602** with a limited nonlinear propagation delay and pulse form distortion relative to the pulses **604** and **609**. With a change in the polarity of the LF pulse, the 1st reflection from **603** will then produce a pulse **612** with comparatively larger nonlinear propagation delay and potential pulse form distortion to the pulse **606**, because the HF pulse follows the high amplitude LF pulse for a longer distance to the scatterer **603**. Subtracting the received signals from the two transmitted pulses will then suppress the multiple reflection noise (pulse reverberation noise) **609/611** relative to the 1st order reflections **606/612** from **603**.

In the given example there is already obtained some nonlinear propagation delay and potentially also pulse form distortion between the multiply reflected pulses **609/611**, which limits the suppression of the multiple scattering noise. The subtraction of the two signals with a delay $2\tau(r)$ from each other produces for band-limited signals a suppression factor $\approx 2\sin(\omega_0\tau(r))$ where $\omega_0$ is the HF pulse center frequency and $\tau(r)$ is the nonlinear propagation delay for the positive LF pulse with a nonlinear propagation delay of $-\tau(r)$ for the negative LF pulse. The approximation in the gain factor is because we have neglected any pulse form distortion and we are representing the gain for all frequencies to $\omega_0$ (narrow band approximation).

To maximize the signal after the subtraction of the pulses **612** and **606** from depth $r_2$, we want $\omega_0\tau(r_2) = \pi/2$, which corresponds to $\tau(r_2)=T_0/4$ where $T_0$ is the period of the HF center frequency.

For strong suppression of the 3rd order scattered pulses **609** and **611** we want $\omega_0\tau(r_1)$ to be small.

When this is not achieved, strong reflectors **602** in the near field can then produce disturbing multiple scattering noise. One method to reduce the nonlinear propagation delay and pulse form distortion at the 1st scatterer **602** in the near field, is to remove part of the LF radiation aperture around the center of the HF aperture, for example as illustrated in **FIG. 3a**. The removed part of the LF radiation aperture reduces the overlap between the LF and the HF beams in the near field indicated as the near field region **305** where the LF field has low amplitude. The nonlinear elasticity manipulation by the LF pulse is therefore very low in the near field region **305**.

The difference between the pulses **609** and **611** in nonlinear propagation lag and pulse form distortion, can also be reduced through delay correction and correction filtering as above. For better understanding of these phenomena we refer to the following formula for the 3rd order scattered HF signal

$$dY_{r3}(\omega;\underline{r}_1,\underline{r}_3) = k^4 U_r\left(\omega;\underline{r}_1\right)ikH_r(\underline{r}_3;\omega;\underline{r}_r)v(\underline{r}_3)d^3r_3 H_{rev}(\underline{r}_3,\underline{r}_1;\omega)H_t(\underline{r}_1;\omega;\underline{r}_t)v(\underline{r}_1)d^3r_1$$

(3?

$$H_{rev}(\underline{r}_3,\underline{r}_1;\omega) = \int_{V_2} d^3r_2 G(\underline{r}_3,\underline{r}_2;\omega)\sigma(\underline{r}_2;\omega)G(\underline{r}_2,\underline{r}_1;\omega)$$

where $\omega$ is the angular frequency and $k = \omega/c$ is the wave number with c as the propagation velocity. $\underline{r}_1$ is the location of the 1st volume scatterer $v(\underline{r}_1)d^3r_1$ ($d^3r_1$ is the volume element at $\underline{r}_1$) that is hit by the transmit beam profile focused at $\underline{r}_t$ with spatial frequency response $H_t(\underline{r}_1;\omega;\underline{r}_t)$. The 1st order scattered wave is further scattered at 2nd scatterers in $V_2$ and the 2nd scattered wave propagates to the location of the 3rd volume scatterer $v(\underline{r}_3)d^3r_3$ at $\underline{r}_3$ ($d^3r_3$ the volume element at $r_3$) by the reverberation function $H_{rev}(\underline{r}_3,r_1;\omega)$. The variable $\sigma(\underline{r}_2;\omega)$ in $H_{rev}(\underline{r}_3,\underline{r}_1;\omega)$ represents the 2nd volume scatterer at $r_2$ and $G(\underline{r}_i,r_j;\omega)$ is the Green's function for propagation in the linear medium from location $\underline{r}_j$ to $\underline{r}_i$. The nonlinear effect of the LF pulse is negligible after the 1st reflection, so that HF pulse propagation from $\underline{r}_1$ via $\underline{r}_2$ to $\underline{r}_3$ is linear and can be described by the Green's function. The 3rd order scattered wave is then picked up by the receive transducer array with a spatial sensitivity given by the receive beam $H_r(\underline{r}_3;\omega;\underline{r}_r)$ focused at $\underline{r}_r$, and $U_r(\omega;\underline{r}_1)$ is the Fourier transform of the received pulse when the 1st scatterer is at $\underline{r}_1$. The total signal is found by summing over all the 1st and 3rd scatterers, i.e. integration over $d^3r_1$ and $d^3r_3$.

The transducer array itself is in medicine often the strongest 2nd scatterer, and can be modeled as

$$\sigma(\underline{r}_2;\omega) = ik2R(\underline{r}_2;\omega)\delta\left(S_R(\underline{r}_2)\right)$$

(3£

where $R(\underline{r}_2;\omega)$ is the reflection coefficient of the reflecting transducer surface $S_R$ defined by $S_R(\underline{r}_2) = 0$, and $\delta(\ )$ is the delta function. Body wall fat layers can also have so strong reflection that 3rd order scattering becomes visible. $\sigma(\underline{r}_2;\omega)$

can then be modeled by a volume scattering distribution as

$$\sigma(\underline{r}_2;\omega) = -k^2 v(\underline{r}_2) \qquad (39$$

In technical applications one often have strongly reflecting layers at a distance from the transducer, for example a metal layer, which can be modeled as Eq.(38) where $S_R(\underline{r}_2) = 0$ now defines the layer surface. Strong volume scatterers are then modeled as in Eq.(39).

The LF pulse changes the propagation velocity of the transmitted HF pulse up to $\underline{r}_1$, given by the average LF pressure along the HF pulse, and changes the frequency content of the HF pulse due to the pulse distortion produced by gradients of the LF pressure along the HF pulse. The effect of the LF pulse will vary somewhat across the HF transmit beam, but as the receive beam is narrow, the effect can be included in received pulse Fourier transform $U_r(\omega;\underline{r}_1)$, both as a change in the linear phase produced by the nonlinear propagation delay (propagation velocity) and a change of the frequency content produced by the pulse distortion. By this we can leave the spatial frequency response of the HF transmit beam $H_t(\underline{r}_1;\omega;r_t)$ unchanged by the co-propagating LF pulse. After the 1st scttering at $\underline{r}_1$ the LF pulse amplitude drops so much that one can neglect nonlinear changes of the HF pulse after this point, and $U_r(\omega;\underline{r}_1)$ therefore only depends on the location $\underline{r}_1$ of the 1st scatterer. The Green's function is reciprocal, i.e. $G(\underline{r}_i,\underline{r}_j;\omega)=G(\underline{r}_j,\underline{r}_i;\omega)$, which implies that $H_{rev}(\underline{r}_3,\underline{r}_1;\omega)=H_{rev}(\underline{r}_1,\underline{r}_3;\omega)$. When the transmit and the receive beams are the same, i.e. $H_r(r_3;\omega;\underline{r}_r)=H_t(\underline{r}_1;\omega;\underline{r}_t)$, the 3rd order scattered signal will be the same when the 1st and 3rd scatterer changes place for $U_r(\omega;\underline{r}_1)=U_r(\omega;\underline{r}_3)$. This is found for zero LF pulse where $U_r(\omega;\underline{r}_1)=U_r(\omega)=U_r(\omega;\underline{r}_3)$, while this equality do not hold for $r_3 \neq r_1$ when the LF pulse produces nonlinear propagation delay and/or pulse distortion.

Higher order pulse reverberation noise can be described by adding further orders of scattering, where odd orders of scattering will contribute to the pulse reverberation noise and will have the same basic properties as described for the 3rd order scattering. Integration over the volume of scatterers and inverse Fourier transform then give us the following model for the received HF signal with a linearly and nonlinearly scattered component plus pulse reverberation noise as

$$s_k(t) = \int dt_0 v_{k1}(t-t_0;t_0)x_1(t_0) + \int dt_0 v_{k2}(t-t_0;t_0)x_2(t_0) + \int_0^t dt_1 \int dt_0 \ v_k^r(t-t_0;t_1)x_r(t_0;t_1)$$

$$k = 1, \mathrm{K}, K \qquad (40$$

where k denotes the pulse number coordinate with variations in the transmitted HF/LF pulse complexes, the 1st and 2nd terms represents the linear and nonlinear 1st order scattering, where $v_{k1}(t-t_0;t_0)$ represents both the nonlinear propagation delay and pulse form distortion for the linearly scattered signal $x_1(t) = x_1(t)$.

Similarly $v_{k2}(t-t_0;t_0)$ represents also both the nonlinear propagation delay and pulse form distortion for the nonlinearly scattered signal, but can in addition represent nonlinear frequency changes in the nonlinearly scattered signal from f.ex. micro-bubbles with different LF and HF pressures so that the nonlinear scattering can be represented by a single signal $x_2(t)$. The last term represents the pulse reverberation noise at fast time t. $x_r(t;t_1)dt_1$ represents the pulse reverberation noise at t for zero LF pulse where the 1st scatterers are in the interval $(t_1, t_1+dt_1)$, and $v_k^r(t-t_0;t_1)$ represents the nonlinear propagation delay and pulse form distortion of the received pulse for non-zero LF pulse where the 1st scatterer is at fast time location $t_1$. For simplicity of notation we have let the filter impulse responses represent both the nonlinear propagation delay and the pulse form distortion, where in practical corrections one would separate the correction for the nonlinear propagation delay and pulse form distortions to reduce the required length of the impulse responses for efficient calculation as discussed in relation to Eq. (24). The filter responses also include variations in the received HF signals from variations in the transmitted HF pulses.

For zero transmitted LF pulse for the first measurement signal we have

$$v_{11}(t-t_0;t_0)=v_1^r(t-t_0;t_1)=\delta(t-t_0) \qquad v_{12}(t-t_0;t_0)=0$$

$$(41)$$

$$s_1(t)=x_1(t)+\int_0^t dt_1 x_r(t;t_1)$$

The last term hence represents the pulse reverberation noise at fast time t when there is no transmitted LF pulse. Generally $v_k^r(t-t_0;t_1)$ has a slow variation with $t_1$, and we can divide the fast time interval into a set of sub-intervals $T_1$ and approximate the integral over $t_1$ with a sum as

$$s_k(t)=\int dt_0 v_{k1}(t-t_0;t_0)x_1(t_0)+\int dt_0 v_{k2}(t-t_0;t_0)x_2(t_0)$$
$$+\sum_{l=3}^{L}\int dt_0\ v_k^r(t-t_0;t_l)\int_{T_l} dt_1 x_r(t_0;t_1)$$

$$(42)$$

which gives the following set of linear operator equations with a finite set of unknowns

$$s_k(t)=V_{k1}\{x_1(t)\}+V_{k2}\{x_2(t)\}+\sum_{l=3}^{L}V_{kl}\{x_l(t)\}$$

$$(43)$$

$$x_l(t)=\int_{T_l} dt_1 x_r(t;t_1) \qquad V_{kl}\{x_l(t)\}=\int dt_1\ v_k^r(t-t_0;t_l)x_l(t_0) \qquad l=3,\mathrm{K},L$$

The operators $V_{kl}$ are linear operators and Eq.(43) can then be viewed as a set of algebraic linear operator equations, which can be solved by similar methods as for a set of ordinary linear algebraic equations. We introduce the vector and operator matrix notations

$$\underline{s}=V\{\underline{x}\}$$

where

$$\underline{s}^T(t)=\{s_1(t),s_2(t),...,s_K(t)\} \qquad \underline{x}^T(t)=\{x_1(t),x_2(t),...,x_L(t)\}$$

$$(44)$$

$$V\{\ \}=\begin{pmatrix} V_{11}\{\ \} & V_{12}\{\ \} & \mathrm{L} & V_{1L}\{\ \} \\ V_{21}\{\ \} & V_{22}\{\ \} & \mathrm{L} & V_{2L}\{\ \} \\ \mathrm{M} & & \mathrm{O} & \mathrm{M} \\ V_{K1}\{\ \} & V_{K2}\{\ \} & \mathrm{L} & V_{KL}\{\ \} \end{pmatrix}$$

where we have introduced arbitrary dimension K, L of the equation set. When K = L, the set of equations can for example be solved with the determinant method for matrix inversion

$$\underline{x}=V^{-1}\{\underline{s}\}$$

where $V^{-1}$ can be calculated as

$$V^{-1} = \left[DetV\right]^{-1}\left[K_{kl}\right]^T = \left[DetV\right]^{-1}\left[K_{lk}\right]$$

$$(45)$$

$$K_{kl} = (-1)^{k+l} M_{kl}$$

$$M_{kl}\{\ \} = Det\begin{pmatrix} V_{11}\{\ \} & L & \text{M} & L & V_{1L}\{\ \} \\ M & & \text{M} & & M \\ L\,L & L\,L & V_{kl}\{\ \} & L\,L & L\,L \\ M & & \text{M} & & M \\ V_{K1}\{\ \} & L & \text{M} & L & V_{KL}\{\ \} \end{pmatrix}$$

$K_{kl}$ is the cofactor matrix where for calculation of $M_{kl}$ the k-th row and 1-th column is removed from the operator matrix $V$. $DetV$ is the operator determinant of the operator matrix $V$ that is calculated in the same way as for a linear algebraic matrix. In these calculations the multiplication of the elements means successive application of the element operators. $DetV$ is hence an operator where $[DetV]^{-1}$ means the inverse of this operator, for example calculated through the Wiener type formula of the Fourier transform as in Eqs. (21-26).

We want to eliminate the noise terms (l = 3, ..., L) to obtain the linearly and nonlinearly scattered signals, $x_1(t)$ and $x_2(t)$. This is obtained through the following estimates

$$x_l(t) = x_1(t) = \left[DetV\right]^{-1}\left[K_{k1}\left\{s_k(t)\right\}\right]$$

$$(46)$$

$$x_n(t) = x_2(t) = \left[DetV\right]^{-1}\left[K_{k2}\left\{s_k(t)\right\}\right]$$

where summation is done over the equal indexes $k$. Again we note that the algebraic multiplication of the operators in the equations above means successive application of the operators to the signals. We note that if we remove $[DetV]^{-1}$ we will get a filtered version of $x_1(t)$ and $x_2(t)$.

It is also interesting to have more measurements than unknowns, i.e. K > L, where one can find the set of unknowns $x_1(t)$ so that the model $\underline{s} = V\{\underline{x}\}$ gives the best approximation to the measured signals $\underline{s}(t)$ for example in the least square sense. The solution to this problem is given by the pseudo inverse of V

$$\underline{x}(t) = \left(V^T V\right)^{-1} V^T\{\underline{s}\}$$

$$(47)$$

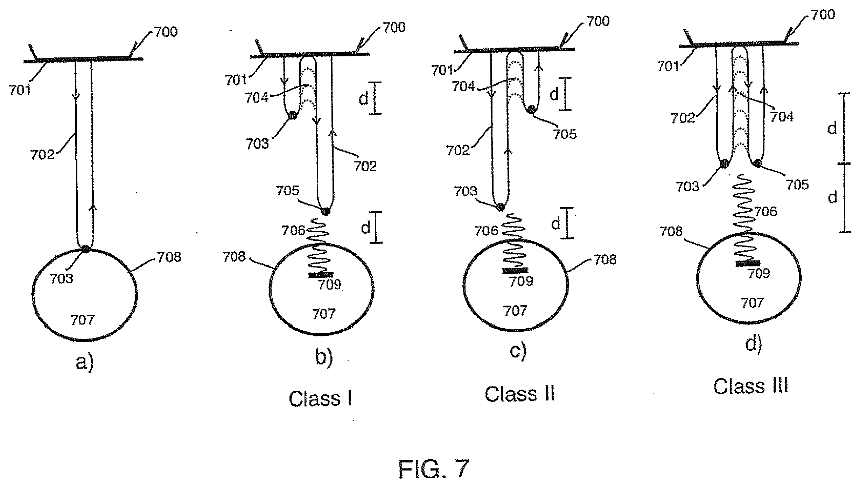

For more detailed analysis of the reverberation noise we refer to **FIG. 7** which schematically illustrates 3 different classes of pulse reverberation noise, Class I - III, b) -d), together with the propagation situation for 1st order scattering in a). The Figures all show an ultrasound transducer array **700** with a front transmit/receive and reflecting surface **701**. The pulse propagation path and direction is indicated with the lines and arrows **702**, where the 1st scatterer is indicated by the dot **703**, and the 3rd scatterer is indicated by the dot **705**. Positions of multiple 2nd scatterers are indicated as **704**. Due to the multiple positions of the 2nd scatterers the two 1st and 3rd scatterers **703** and **705** then generates a tail of pulse reverberation noise **706** following the deepest of the 1st and 3rd scatterers. **707** shows for illustrative example a low echogenic region, for example in medicine the cross section of a vessel or a cyst, with a scattering surface **708**, that we want to image with high suppression of pulse reverberation noise. The object has so low internal scattering that the pulse reverberation noise **706** generated in the different classes produces disturbances in the definition of the scattering surface **708**.

To obtain visible, disturbing reverberation noise in the image, the 1st - 3rd scatterers in Eq.(37) must be of a certain strength. In medical applications this is often found by fat layers in the body wall, while in technical applications one can encounter many different structures, depending on the application. In medical applications, the strongest 2nd scatterer is often the ultrasound transducer array surface itself, as this can have a reflection coefficient that is ~ 10dB or more higher than the reflection coefficient from other soft tissue structures. The pulse reverberation noise that involves the transducer surface as the 2nd scatterer, is therefore particularly strong indicated by **709**.

Further description of the Classes is:

**Class I** (b) is the situation where the 1st scatterer (**703**) is closest to the transducer. The distance between the 3rd scatterer and the noise point is the same as the distance between the 1st and the 2nd scatterer. With the 2nd scatterer is a distance d behind the 1st scatterer, the noise point is found a distance d from the 3rd scatterer **705** which in the Figure coincides with the object surface **706**. With varying positions of the 2nd scatterer **704,** we get a tail **706** of pulse reverberation noise. When the transducer surface **701** is the strongest 2nd scatterer, we get particularly strong noise at **709**. As the 1st scatterer is closest to the transducer for Class I, the nonlinear propagation lag and pulse distortion at the 1st scatterer is here low, and especially with a LF aperture that lacks the central part as described in relation to **FIG. 3a** the nonlinear propagation lag and pulse distortion can often be neglected at the 1st scatterer. Subtraction of the received signals from two pulse complexes with opposite polarity of the LF pulse will then produce a strong suppression of the pulse reverberation noise of this Class. When the central part of the LF transmit aperture is not missing, the suppression can however improve by a correction for pulse form distortion lag and/or the nonlinear propagation at the 1st scatterer, as discussed below.

**Class II** (c) The 1st scatterer **703** has moved so deep that the 3rd (**705**) scatterer is closer to the transducer than the 1st scatterer. When strong scatterers are found at **703** and **705** for Class I, we also have Class II noise with 1st and 3rd scatterers interchanged. Class II hence shows the inverse propagation **702** of Class I, and the two classes of noise therefore always coexist. The reasoning for the tail of pulse reverberation noise **706** is the same as that for Class I/II, where a strongly reflecting transducer surface gives the noise **709**. With the same transmit and receive beams (focus and aperture), Eq.(37) shows that the pulse reverberation noise with zero LF pulse is the same for Class I and Class II. However, with nonzero LF pulse, the received HF signals from these two classes are different, because one gets higher nonlinear propagation lag and pulse distortion for Class II than for Class I because the 1st scatterer is deeper for Class II. However, the two noise classes can be summed to one class which reduces the number of unknowns in the equation as in Eqs.(69-73).

**class III** (d) This Class is found as a merger of Class I and Class II where the 1st scatterer **(703)** has moved so deep that the 1st and 3rd **(705)** scatterers have similar depths or are the same, so that the nonlinear propagation lag and pulse distortions are approximately the same when the 1st and 3rd scatterers are interchanged. This noise hence includes a factor 2 in the amplitude, as a sum of the 1st and 3rd scatterer being **703** and **705** and the reverse path where the 1st and 3rd scatterers are **705** and **703**. This situation is often found with strongly scattering layers, as a metal layer in industrial applications and a fat layer or bone structure in medical applications. The reasoning for the tail of pulse reverberation noise **706** is the same as that for Class I/II, where a strongly reflecting transducer surface gives the noise **709**.

With defined regions of the 1st and 3rd scatterers for Class I - II noise, Eq. (43) can be approximated to

$$
\begin{aligned}
s_1(t) &= V_{11}\{x_1(t)\} + V_{12}\{x_2(t)\} + V_{13}\{x_3(t)\} + V_{14}\{x_4(t)\} + V_{15}\{x_5(t)\} \\
s_2(t) &= V_{21}\{x_1(t)\} + V_{22}\{x_2(t)\} + V_{23}\{x_3(t)\} + V_{24}\{x_4(t)\} + V_{25}\{x_5(t)\} \\
s_3(t) &= V_{31}\{x_1(t)\} + V_{32}\{x_2(t)\} + V_{33}\{x_3(t)\} + V_{34}\{x_4(t)\} + V_{35}\{x_5(t)\} \\
s_4(t) &= V_{41}\{x_1(t)\} + V_{42}\{x_2(t)\} + V_{43}\{x_3(t)\} + V_{44}\{x_4(t)\} + V_{45}\{x_5(t)\} \\
s_5(t) &= V_{51}\{x_1(t)\} + V_{52}\{x_2(t)\} + V_{53}\{x_3(t)\} + V_{54}\{x_4(t)\} + V_{55}\{x_5(t)\}
\end{aligned} \tag{48}
$$

where $\underline{x}^T(t) = \{x_1(t), x_2(t), x_3(t), x_4(t), x_5(t)\}$ is the initial signals where $x_1(t)$ is the 1st order linearly scattered signal for zero LF pulse, $x_2(t)$ is the 1st order nonlinearly scattered signal for a normalized HF and LF pulse and $x_3(t), x_4(t), x_5(t)$ are the pulse reverberation noise of Class I, III, and II for zero LF pulse. One example is a fixed HF pulse with a relative variation of the $L_F$ transmit amplitudes between the 5 pulse complexes relative to the normalized LF pulse by $p_1$ - $p_5$. Typically we can use $p_1 = 0$, $p_5 = -p_2$, and $p_4 = -p_3 = -p_2/2$. For the 2nd order elasticity where the approximation of the nonlinear scattering of Eq.(5) is adequate we can set $V_{k2} = p_k V_{k1}$, as the operators represent the nonlinear propagation delay and pulse distortion of the forward propagating pulse. However, with heavily resonant HF scattering as from micro gas-bubbles, the LF pressure can also affect the scattered waveform so that $V_{k2} \neq p_k V_{k1}$. However, making the assumption of $V_{k2} = p_k V_{k1}$ can help us to solve the above equations as the nonlinearly scattered signal is generally much weaker than the linearly scattered signal. In the same way as in Eq. (40-44), one can let $Vk_1$ include variations in the transmitted HF pulse, both in amplitude, polarity, frequency, and form (for example variations of a coded pulse).

For the practical calculation we can also solve the set of equations in Eqs. (43-48) through a successive elimination similar to the Gauss elimination for linear algebraic equations. We can typically start by eliminating the noise signals in a sequence, for example starting with elimination of Class II noise, $x_5(t)$, as

$$
\begin{aligned}
z_1(t) &= V_{15}^{-1}\{s_1(t)\} - V_{25}^{-1}\{s_2(t)\} \\
&= V_{15}^{-1}V_{11}\{x_1(t)\} - V_{25}^{-1}V_{21}\{x_1(t)\} + V_{15}^{-1}V_{12}\{x_2(t)\} - V_{25}^{-1}V_{22}\{x_2(t)\} \\
&\quad + V_{15}^{-1}V_{13}\{x_3(t)\} - V_{25}^{-1}V_{23}\{x_3(t)\} + V_{15}^{-1}V_{14}\{x_4(t)\} - V_{15}^{-1}V_{24}\{x_4(t)\} \\
&= A_{11}\{x_1(t)\} + A_{12}\{x_2(t)\} + A_{13}\{x_3(t)\} + A_{14}\{x_4(t)\} \\
&\quad a)
\end{aligned}
$$

$$
\begin{aligned}
z_2(t) &= V_{15}^{-1}\{s_1(t)\} - V_{35}^{-1}\{s_3(t)\} \\
&= V_{15}^{-1}V_{11}\{x_1(t)\} - V_{35}^{-1}V_{31}\{x_1(t)\} + V_{15}^{-1}V_{12}\{x_2(t)\} - V_{35}^{-1}V_{32}\{x_2(t)\} \\
&\quad + V_{15}^{-1}V_{13}\{x_3(t)\} - V_{35}^{-1}V_{33}\{x_3(t)\} + V_{15}^{-1}V_{14}\{x_4(t)\} - V_{35}^{-1}V_{34}\{x_4(t)\} \\
&= A_{21}\{x_1(t)\} + A_{22}\{x_2(t)\} + A_{23}\{x_3(t)\} + A_{24}\{x_4(t)\} \\
&\quad b)
\end{aligned}
$$

$$
\begin{aligned}
z_3(t) &= V_{13}^{-1}\{s_1(t)\} - V_{45}^{-1}\{s_4(t)\} \\
&= V_{13}^{-1}V_{11}\{x_1(t)\} - V_{45}^{-1}V_{41}\{x_1(t)\} + V_{13}^{-1}V_{12}\{x_2(t)\} - V_{45}^{-1}V_{42}\{x_2(t)\} \\
&\quad + V_{13}^{-1}V_{13}\{x_3(t)\} - V_{45}^{-1}V_{43}\{x_3(t)\} + V_{14}^{-1}V_{14}\{x_4(t)\} - V_{45}^{-1}V_{44}\{x_4(t)\} \\
&= A_{31}\{x_1(t)\} + A_{32}\{x_2(t)\} + A_{33}\{x_3(t)\} + A_{34}\{x_4(t)\} \\
&\quad c)
\end{aligned}
$$

$$
\begin{aligned}
z_4(t) &= V_{13}^{-1}\{s_1(t)\} - V_{55}^{-1}\{s_5(t)\} \\
&= V_{13}^{-1}V_{11}\{x_1(t)\} - V_{55}^{-1}V_{51}\{x_1(t)\} + V_{13}^{-1}V_{12}\{x_2(t)\} - V_{55}^{-1}V_{52}\{x_2(t)\} \\
&\quad + V_{13}^{-1}V_{13}\{x_3(t)\} - V_{55}^{-1}V_{53}\{x_3(t)\} + V_{13}^{-1}V_{14}\{x_4(t)\} - V_{55}^{-1}V_{54}\{x_4(t)\} \\
&= A_{41}\{x_1(t)\} + A_{42}\{x_2(t)\} + A_{43}\{x_3(t)\} + A_{44}\{x_4(t)\} \\
&\quad d)
\end{aligned}
$$

$$(49)$$

where $V_{kl}^{-1}$ is the inverse operator for $V_{k1}$, for example obtained along the lines of the Wiener type filter in Eqs. (21 - 26). In the above notation both the nonlinear propagation delay and the pulse form distortion is included in the filter operators. However, for practical calculations of the inverse operators, one would do a direct delay correction for the nonlinear propagation delay, with a filter for the pulse form correction. This separation reduces the required length of the filter impulse responses.

Note that if the 1st measured signal ($s_1$) is obtained with zero LF pulse, we have $V_{1l} = V_{1l}^{-1} = I$ , i.e. the identity operator. Applying $V_{k5}^{-1}$ on $s_k(t)$, k = 2, ..., 5 and subtracting provides heavy suppression of $x_5(t)$. We can then proceed in the same way to suppress $x_4(t)$ from the above expressions by forming the signals

$$u_1(t) = A_{14}^{-1}\{z_1(t)\} - A_{24}^{-1}\{z_2(t)\}$$
$$= A_{14}^{-1}A_{11}\{x_1(t)\} - A_{24}^{-1}A_{21}\{x_1(t)\} + A_{14}^{-1}A_{12}\{x_2(t)\} - A_{24}^{-1}A_{22}\{x_2(t)\}$$
$$+ A_{14}^{-1}A_{13}\{x_3(t)\} - A_{24}^{-1}A_{23}\{x_3(t)\}$$
$$= B_{11}\{x_1(t)\} + B_{12}\{x_2(t)\} + B_{13}\{x_3(t)\}$$
$$a)$$

$$u_2(t) = A_{14}^{-1}\{z_1(t)\} - A_{34}^{-1}\{z_3(t)\}$$
$$= A_{14}^{-1}A_{11}\{x_1(t)\} - A_{34}^{-1}A_{31}\{x_1(t)\} + A_{14}^{-1}A_{12}\{x_2(t)\} - A_{34}^{-1}A_{32}\{x_2(t)\}$$
$$+ A_{14}^{-1}A_{13}\{x_3(t)\} - A_{34}^{-1}A_{33}\{x_3(t)\}$$
$$= B_{21}\{x_1(t)\} + B_{22}\{x_2(t)\} + B_{23}\{x_3(t)\}$$
$$b) \qquad\qquad (50)$$

$$u_3(t) = A_{14}^{-1}\{z_1(t)\} - A_{44}^{-1}\{z_4(t)\}$$
$$= A_{14}^{-1}A_{11}\{x_1(t)\} - A_{44}^{-1}A_{41}\{x_1(t)\} + A_{14}^{-1}A_{12}\{x_2(t)\} - A_{44}^{-1}A_{42}\{x_2(t)\}$$
$$+ A_{14}^{-1}A_{13}\{x_3(t)\} - A_{44}^{-1}A_{43}\{x_3(t)\}$$
$$= B_{31}\{x_1(t)\} + B_{32}\{x_2(t)\} + B_{33}\{x_3(t)\}$$
$$c)$$

and proceeding in the same manner to suppress $x_3(t)$, we obtain signals where Class I-III multiple scattering noise is heavily suppressed

$$q_1(t) = B_{13}^{-1}\{u_1(t)\} - B_{23}^{-1}\{u_2(t)\}$$
$$= B_{13}^{-1}B_{11}\{x_1(t)\} - B_{23}^{-1}B_{21}\{x_1(t)\} + B_{13}^{-1}B_{12}\{x_2(t)\} - B_{23}^{-1}B_{22}\{x_2(t)\}$$
$$= C_{11}\{x_1(t)\} + C_{12}\{x_2(t)\}$$
$$a) \qquad\qquad (51)$$

$$q_2(t) = B_{13}^{-1}\{u_1(t)\} - B_{33}^{-1}\{u_3(t)\}$$
$$= B_{13}^{-1}B_{11}\{x_1(t)\} - B_{33}^{-1}B_{31}\{x_1(t)\} + B_{13}^{-1}B_{12}\{x_2(t)\} - B_{33}^{-1}B_{32}\{x_2(t)\}$$
$$= C_{21}\{x_1(t)\} + C_{22}\{x_2(t)\}$$
$$c)$$

We can then proceed in the same manner and eliminate $x_2(t)$, or we can solve Eq. (51) with the algebraic formulas for two linear equations with two unknowns as

$$x_l(t) = x_1(t) = \left[C_{11}C_{22} - C_{12}C_{21}\right]^{-1}\{C_{22}\{q_1(t)\} - C_{12}\{q_2(t)\}\} \qquad a)$$
$$(52)$$
$$x_n(t) = x_2(t) = \left[C_{11}C_{22} - C_{12}C_{21}\right]^{-1}\{C_{11}\{q_2(t)\} - C_{21}\{q_1(t)\}\} \qquad b)$$

The linearly scattered HF signal $x_1(t)$ is often much stronger than the nonlinear signal $x_2(t)$, and one can then approximate $x_1(t) = x_1(t) = C_{11}^{-1}\{q_1(t)\}$. We note as for Eq. (45) that if we remove the inverse determinant we get filtered versions of the linearly and nonlinearly scattered signals, $x_1(t)$ and $x_2(t)$.

The operators in Eqs. (43,44,48) are generally filters with impulse/frequency responses that can be approximated to stationary within intervals $T(t_1)$ as in Eq. (20). The inverse filters of Eqs.(45-52) can be solved via a Fourier transform as in Eqs. (20 - 26). One can also do a Fourier transform of Eqs. (43,44,48) for each interval, which gives a set of ordinary algebraic equations for each frequency of the Fourier transforms $X_1(\omega)$ of $x_1(t)$. These equations are then solved by well known analytic or numerical methods for such equations, and the time functions for $x_1(t)$ and $x_2(t)$ are then obtained by Fourier inversion.

For estimation of the actual delay and pulse distortion corrections, the $V_{k1}$ operators in Eqs. (43,44,48) can be estimated based on the following considerations: The LF pulse has so low frequency compared to the image depth (typical image depth $\sim 30\lambda_{LF}$) that one can often neglect individual variations of absorption and wave front aberration for the LF beam. The nonlinear propagation delays and pulse distortion can therefore often be obtained with useful accuracy from simulations of the composite LF/HF elastic waves with known, assumed, adjusted, or estimated local propagation parameters. The propagation parameters are composed of the local mass density and linear and nonlinear elasticity parameters of the material. For adequately adjusted local nonlinear elasticity parameters as a function of propagation path of the HF pulse, one can use the formula in Eq.(3) to obtain an estimate of the nonlinear propagation delay $\tau(t)$.

The correction delays can for example therefore be obtained from

1. simulated nonlinear propagation delays in simulations of the composite LF/HF elastic waves with one of

- assumed local propagation parameters for the materials, or
- manual adjustment of local propagation material parameters to one or both of

    i) minimize the pulse reverberation noise in the observed image, and
    ii) minimize the linear scattering component in the image, or

2. manual adjustment of the local correction delays to one or both of

    i) minimize the pulse reverberation noise in the observed image, and
    ii) minimize the linear scattering component in the image, or

3. as estimates of the nonlinear propagation delays for example as described in US Pat Appl US2005277835 and US2006052699 , or

4. a combination of the above.

The pulse distortion correction can similarly be obtained from

**1.** simulated nonlinear pulse distortions in simulations of the composite LF/HF elastic waves with one of

- assumed local propagation parameters for the materials, or
- manual adjustment of local material propagation parameters to one or both of

    i) minimize the pulse reverberation noise in the observed image, and
    ii) minimize the linear scattering component in the image, or

- elastic parameters obtained from the fast time gradient of estimated correction delays, for example according to Eq. (7). Said estimated correction delays are for example obtained from manual local adjustment of the nonlinear propagation delays or estimations from received signals as described above, or

2. from direct estimation of the pulse distortion from the frequency spectrum of the received HF signals from a region of scatterers, and this spectral estimate is used to estimate fast time expansion/compression or $v_k(t,t_0)$ in Eqs. (18 - 20), or

3. a combination of point 1 and 2.

The manual adjustment of local propagation (elasticity) parameters or nonlinear correction delays can for example be done through multiple user controls in a set of selected depth intervals, for maximal suppression of the linearly scattered signal, or for maximal suppression of pulse reverberation noise as discussed. For feedback one can for example use the image signal as displayed on the screen. One can also conveniently combine the methods, for example that the simulation estimates with assumed local elasticity parameters are applied first with further automatic estimation or manual adjustments to one or both of i) minimize the pulse reverberation noise in the observed image, and ii) minimize the linear scattering component in the image.

When the object scatterers move between pulse reflections, the $V_{k1}\{\}$ operators also contain a Doppler delay in addition to the nonlinear propagation delay. With a relative variation in pulse amplitude of $p_k$ between the LF pulses, the nonlinear propagation delay can be approximated as $p_k\tau(t)$, and estimating the total propagation delay *as* $\tau_{Tk}(t)=kT_d(t)+p_k\tau(t)$, the sum of the Doppler and nonlinear propagation delay for several received signals, will then allow estimation of the Doppler and nonlinear propagation delays separately, for example as described in US Pat Appl US2005277835 and US2006052699 .

To further elaborate on methods of suppression of pulse reverberation noise and linear scattering, we first give an example according to the invention using a sequence of 3 transmitted pulse complexes, where the transmitted LF pulse amplitudes of the successive pulse complexes are $P_0$, 0, -$P_0$. A strong scatterer close to fast time $t_1$ produces pulse reverberation noise, and according to Eqs.(43,48) we can express the received HF signals from the 3 pulse complexes as

$$s_+(t) = V_t^+\{x_+(t+\tau(t))\} + V_{t_1}^+\{w(t+\tau(t_1))\} \quad x_+(t) = x_l(t) + x_{n+}(t)$$

$$s_0(t) = x_l(t) + w(t)$$

$$s_-(t) = V_t^-\{x_-(t-\tau(t))\} + V_{t_1}^-\{w(t-\tau(t_1))\} \quad x_-(t) = x_l(t) - x_{n-}(t)$$

$$(53)$$

$$V_t^{\pm}\{x_{\pm}(t)\} = \int dt_0 v_{\pm}(t-t_0,t_0)x_{\pm}(t_0)$$

$$V_{t_1}^{\pm}\{w(t)\} = \int dt_0 v_{\pm}(t-t_0,t_1)w(t_0)$$

where now $V_t^{\pm}$ represents only the pulse form distortion while the nonlinear propagation delay is explicitely included in the time argument of the functions as in Eqs. (19-26) (See comments after Eqs. (24,40)). The pulse reverberation noise is $w(t)$ with the 1st and 3rd scatterer 602 at the fast time (depth-time) location around $t_1 = 2r_1/c$. We then do a correction for the + and - signals for the pulse form distortion and nonlinear propagation delay at the 1st strong scatterer 602 located at fast time $t_1$. With the Wiener type inverse filters as given in Eq.(21) we get

$$y_+(t) = H_{t_1}^+\{s_+(t-\hat{\tau}(t_1))\} = H_{t_1}^+V_t^+\{x_+(t+\tau(t)-\hat{\tau}(t_1))\} + w(t)$$

$$y_-(t) = H_{t_1}^-\{s_-(t+\hat{\tau}(t_1))\} = H_{t_1}^-V_t^-\{x_-(t-\tau(t)+\hat{\tau}(t_1))\} + w(t)$$

$$H_{t_1}^+\{s_+(t)\} = \int dt_0 h_+(t-t_0,t_1)s_+(t_0)$$

$$H_{t_1}^-\{s_-(t)\} = \int dt_0 h_-(t-t_0,t_1)s_-(t_0)$$

$$(54)$$

where $\hat{\tau}(t_1)$ is the estimated nonlinear propagation delay at fast time $t_1$, and $H_{t_1}^+\{\ \}$ represents the pulse distortion correction for the positive LF pulse with the distortion at $t_1$, and $H_{t_1}^-\{\ \}$ represents the pulse distortion correction for the negative LF pulse with the distortion at $t_1$. The $H_t^{\pm}$ represents a general pulse distortion correction that for example can be done through fast time expansion/compression of the received HF signal as discussed above, or through filtering as indicated in Eq. (54), or through a modification of the transmitted pulses so that the pulse reverberation noise for 1st scatterers at depth time $t_1$ is independent on the LF pulse per the discussion in relation to **FIG 2d,** or a combination of these. One can hence obtain a HF signal with improved suppression of the pulse reverberation noise **(609/611)** by the combination

$$y_+(t) - y_-(t) = H_{t_1}^+V_t^+\{x_+(t+\tau(t)-\hat{\tau}(t_1))\} - H_{t_1}^-V_t^-\{x_-(t-\tau(t)+\hat{\tau}(t_1))\}$$

$$(55)$$

For noise suppression with stationary objects, one might hence transmit only two pulse complexes with amplitudes $P_0$ and $-P_0$ of the LF pulse, or potentially with zero LF pulse in one of the pulse complexes.

For additional suppression of the HF linear scattering components to provide a nonlinear HF signal representing nonlinear scattering from the object, we can form the following combinations of the 3-pulse sequence for suppression of the pulse reverberation noise

$$z_1(t) = y_+(t) - s_0(t) = H_{t_1}^+ V_t^+ \left\{ x_+ \left( t + \tau(t) - \hat{\tau}(t_1) \right) \right\} - x_l(t)$$

(56)

$$z_2(t) = s_0(t) - y_-(t) = x_l(t) - H_{t_1}^- V_t^- \left\{ x_- \left( t - \tau(t) + \hat{\tau}(t_1) \right) \right\}$$

The signal component from the scatterer 603 obtains further pulse form distortion and nonlinear propagation delay due to the further propagation along the transmit LF pulse from **602** to **603**. The HF pulses for positive and negative LF transmit pulses then obtains opposite pulse form distortion, i.e. a HF pulse length compression for the positive LF pulse gives a similar HF pulse length expansion for the negative LF pulse (and vice versa), and the nonlinear propagation delays have opposite signs. With zero transmitted LF pulse there is no HF pulse form distortion or nonlinear propagation delay. We then delay $z_1$ with $(\hat{\tau}(t)-\hat{\tau}(t_1))/2$ and advance $z_2$ with $(\tau(t)-\tau(t_1))/2$, where $\tau(t)$ is the estimated nonlinear propagation delay of the signals at fast time t, and pulse distortion correct $z_1$ and $z_2$ for half the pulse length compression/expansion from **602** to **603.**

We note from Eq. (53) that the nonlinear component of the scattered signal is zero for zero LF pressure. With pulse distortion correction for half the LF pressure, we note that for the linear components of HF scattered signal we have

$$H_{t;t_1}^{+1/2} H_{t_1}^+ V_t^+ \left\{ x_l \left( t + \left( \tau(t) - \hat{\tau}(t_1) \right) / 2 \right) \right\} = H_{t;t_1}^{-1/2} \left\{ x_l \left( t + \left( \tau(t) - \hat{\tau}(t_1) \right) / 2 \right) \right\}$$

(57)

$$H_{t;t_1}^{-1/2} H_{t_1}^- V_t^- \left\{ x_l \left( t - \left( \tau(t) - \hat{\tau}(t_1) \right) / 2 \right) \right\} = H_{t;t_1}^{+1/2} \left\{ x_l \left( t - \left( \tau(t) - \hat{\tau}(t_1) \right) / 2 \right) \right\}$$

where the $H_{t;t_1}^{+1/2}$ implies correcting the signals for half the pulse distortion produced by the positive LF pulse from $t_1$ to t, and the $H_{t;t_1}^{-1/2}$ implies correcting for half the pulse distortion of the negative LF pulse from $t_1$ to t. These corrections can for the 2$^{nd}$ order approximation of the nonlinear elasticity as in Eq. (1) be estimated from the distortion from $t_1$ to t for a signal with half the transmitted LF amplitude.

Hence does the following combination produce improved suppression of the linear scattering components to give a signal that represents the nonlinear HF scattering with highly suppressed reverberation noise

$$\hat{x}_n(t) = H_{t;t_1}^{+1/2} \left\{ z_1 \left( t - \left( \hat{\tau}(t) - \hat{\tau}(t_1) \right) / 2 \right) \right\} - H_{t;t_1}^{-1/2} \left\{ z_2 \left( t + \left( \hat{\tau}(t) - \hat{\tau}(t_1) \right) / 2 \right) \right\}$$

$$\approx H_{t;t_1}^{+1/2} H_{t_1}^+ V_t^+ \left\{ x_+ \left( t + \left( \tau(t) - \hat{\tau}(t_1) \right) / 2 \right) \right\} - H_{t;t_1}^{+1/2} \left\{ x_l \left( t - \left( \tau(t) - \hat{\tau}(t_1) \right) / 2 \right) \right\}$$
$$- H_{t;t_1}^{-1/2} \left\{ x_l \left( t + \left( \tau(t) - \hat{\tau}(t_1) \right) / 2 \right) \right\} + H_{t;t_1}^{-1/2} H_{t_1}^- V_t^- \left\{ x_- \left( t - \left( \tau(t) - \hat{\tau}(t_1) \right) / 2 \right) \right\}$$

(58)

$$\approx H_{t;t_1}^{+1/2} H_{t_1}^+ V_t^+ \left\{ x_{n+} \left( t + \left( \tau(t) - \hat{\tau}(t_1) \right) / 2 \right) \right\} - H_{t;t_1}^{-1/2} H_{t_1}^- V_t^- \left\{ x_{n-} \left( t - \left( \tau(t) - \hat{\tau}(t_1) \right) / 2 \right) \right\}$$

An estimate of the linear scattering can be obtained as

$$\hat{x}_l(t) = H_{t;t_1}^{+}\left\{z_1\left(t-\left(\hat{\tau}(t)-\hat{\tau}(t_1)\right)\right)\right\} - H_{t;t_1}^{-}\left\{z_2\left(t+\left(\hat{\tau}(t)-\hat{\tau}(t_1)\right)\right)\right\}$$

$$= H_t^{+}V_t^{+}\left\{x_l(t)\right\} + H_t^{+}V_t^{+}\left\{x_{n+}(t)\right\} - H_{t;t_1}^{+}\left\{x_l\left(t-\left(\hat{\tau}(t)-\hat{\tau}(t_1)\right)\right)\right\}$$
$$- H_{t;t_1}^{-}\left\{x_l\left(t+\left(\hat{\tau}(t)-\hat{\tau}(t_1)\right)\right)\right\} + H_t^{-}V_t^{-}\left\{x_l(t)\right\} - H_t^{-}V_t^{-}\left\{x_{n-}(t)\right\} \qquad (59)$$

$$\approx 2x_l(t) - H_{t;t_1}^{+}\left\{x_l\left(t-\left(\hat{\tau}(t)-\hat{\tau}(t_1)\right)\right)\right\} - H_{t;t_1}^{-}\left\{x_l\left(t+\left(\hat{\tau}(t)-\hat{\tau}(t_1)\right)\right)\right\}$$

where $H_{t;t_1}^{\pm}$ represents the correction for the pulse form distortion from $t_1$ to $t$, so that $H_{t;t_1}^{\pm}H_{t_1}^{\pm} = H_t^{\pm}$ which is the correction for the pulse form distortion from 0 to t. We hence have $H_{t;t_1}^{\pm}H_{t_1}^{\pm}V_t^{\pm} = H_t^{\pm}V_t^{\pm} = I$, i.e. the identity operator.

We have assumed that $x_{n-}(t) \approx x_{n+}(t)$ which is exact for 2nd order elasticity. However, the nonlinear scattering is much weaker than the linear scattering which makes the approximations in Eq. (59) adequate also for other nonlinear scattering.

The combination of signals with opposite polarity of the LF pulse as in Eqs. (55,58,59), reduces required accuracy in the estimated delay and pulse distortion corrections, as seen from the following analysis.. This opens for an approximate but robust method to suppress both the pulse reverberation noise and the linear scattering to obtain estimates of the nonlinear scattering from the received HF signals in Eq. (53). We model the pulse form distortion as a pure time compression/expansion as

$$V_t^{\pm}\left\{x_h\left(t\pm\tau(t)\right)\right\} = \int dt_0 u\left[\left(1\pm a(t)\right)\left(t-t_0\right),t_0\right]\sigma_h\left(t_0\pm\tau(t)\right) \qquad (60)$$

where $u(t,t_0)$ is the received pulse from a point scatterer at $t_0$ with zero LF pulse, $\sigma_h(t_0)$ is the scatterer density for the linear scattering ($h = 1$), the nonlinear scattering with positive LF pulse ($h = n+$), the nonlinear scattering for negative LF pulse ($h = n-$), and $\sigma_w(t_0)$ is an equivalent scatterer density for multiple scattering noise where the 1st scatterer is at $t_1$. $\tau(t)$ is the nonlinear propagation delay down to fast time t and the factor $(1\pm a(t))$ represents pulse time compression/expansion produced by gradients of the LF pulses along the HF pulses, where the sign of $\pm a(t)$ is given by the sign of the LF pulses. $\tau_1$ and $a_1$ are the nonlinear propagation delay and pulse time compression factor where the 1st scatterer is found at $t_1$, which is the situation for the pulse reverberation noise. We note that for materials that can be approximated with 2nd order nonlinear elasticity as in Eqs. (1,5) we have $\sigma_{n-}(t_0) = \sigma_{n+}(t_0)$.

When the HF pulse is long compared to the LF pulse period, the approximation in Eq.(60) of pure time compression/expansion of the pulse becomes less accurate, and one can improve the approximation by introducing higher order terms $(1\pm a_1(t))(t-t_0)ma_2(t)(t-t_0)^2\pm\dots$ in the pulse argument in Eq. (60), as discussed in relation to Eq. (26). However the analysis of the corrections will be similar, and for simplified notation we therefore use only the linear compression in Eq. (60), where the expansion to higher order terms are obvious to anyone skilled in the art. We correct the signals in Eq. (60) for pulse compression/expansion for example with a filter in analogy with Eqs.(19-26), through modifications of the transmitted HF pulses as described in relation to **FIG**. **2d,** through time expansion/compression of the received signal as desribed above, or a combination of the above. Combined correction for nonlinear propagation delay and pulse form distortion of the signals in Eq.(60) then gives

$$H_{\pm a_c} V_t^{\pm} \left\{ x_h \left( t \pm (\tau(t) - \tau_c) \right) \right\}$$

$$= \int dt_0 u \left[ (1 \pm (a(t) - a_c))(t - t_0), t_0 \right] \sigma_h \left( t_0 \pm (\tau(t) - \tau_c) \right)$$

$$\approx x_h(t) \pm (a(t) - a_c) \int dt_0 (t - t_0) u \mathcal{S}(t - t_0, t_0) \sigma_h(t_0)$$

$$\pm (\tau(t) - \tau_c) \int dt_0 u(t - t_0, t_0) \mathcal{S}_h(t_0)$$

$$\tag{61}$$

$$H_{\pm a_c} V_{t_1}^{\pm} \left\{ w \left( t \pm (\tau_1 - \tau_c) \right) \right\}$$

$$= \int dt_0 u \left[ (1 \pm (a_1 - a_c))(t - t_0), t_1 \right] \sigma_w \left( t_0 \pm (\tau_1 - \tau_c) \right)$$

$$\approx w(t) \pm (a_1 - a_c) \int dt_0 (t - t_0) u \mathcal{S}(t - t_0, t_1) \sigma_w(t_0)$$

$$\pm (\tau_1 - \tau_c) \int dt_0 u(t - t_0, t_1) \mathcal{S}_w(t_0)$$

where

$$x_h(t) = \int dt_0 u(t - t_0, t_0) \sigma_h(t_0)$$

$$w(t) = \int dt_0 u(t - t_0, t_1) \sigma_w(t_0)$$

and $H_{\pm a_c}$ is the correction for the pulse time compression/expansion with the factor $1 \pm a_c$, and the approximate expressions are obtained with Taylor series expansion of the functions to the 1st order. The accuracy of the expansion then depends on the magnitude of $a - a_c$ and $\tau - \tau_c$, and can be adjusted by adjusting $a_c$ and $\tau_c$. The corrected received signals then take the form

$$y_\pm(t) = H_{\pm a_c} \left\{ s_\pm \left( t \mp \tau_c \right) \right\}$$

$$= H_{\pm a_c} V_t^{\pm} \left\{ x_l \left( t \pm (\tau(t) - \tau_c) \right) \right\} \pm H_{\pm a_c} V_t^{\pm} \left\{ x_{n\pm} \left( t \pm (\tau(t) - \tau_c) \right) \right\} \tag{62}$$

$$+ H_{\pm a_c} V_{1t}^{\pm} \left\{ w \left( t \pm (\tau(t) - \tau_c) \right) \right\}$$

Choosing $a_c \approx a_1$ and $\tau_c \approx \tau_1$ we can in analogy with Eq. (55) form a noise suppressed estimate of the linear scattering as

$$y_+(t) - y_-(t) = H_{a_1} \left\{ s_+ \left( t - \tau_1 \right) \right\} - H_{-a_1} \left\{ s_- \left( t + \tau_1 \right) \right\}$$

$$= x_l(t) + (a(t) - a_1) \int dt_0 (t - t_0) u \mathcal{S}(t - t_0, t_0) \sigma_l(t_0)$$

$$+ (\tau(t) - \tau_1) \int dt_0 u(t - t_0, t_0) \mathcal{S}_l(t_0) + w(t) \tag{63}$$

$$- x_l(t) + (a(t) - a_1) \int dt_0 (t - t_0) u \mathcal{S}(t - t_0, t_0) \sigma_l(t_0)$$

$$+ (\tau(t) - \tau_1) \int dt_0 u(t - t_0, t_0) \mathcal{S}_l(t_0) - w(t)$$

which gives

$$y_+(t) - y_-(t) \approx 2(a(t) - a_1) \int dt_0 (t - t_0) u \mathcal{S}(t - t_0, t_0) \sigma_l(t_0)$$

$$+ 2(\tau(t) - \tau_1) \int dt_0 u(t - t_0, t_0) \mathcal{S}_l(t_0) \tag{64}$$

where we have neglected the nonlinear scattering compared to the linear scattering.

A noise suppressed estimate of the nonlinearly scattered signal can be found through an approximation of Eqs. (56,58) as

$$\hat{x}_n(t) = y_+(t) - s_0(t) - \left(s_0(t) - y_-(t)\right)$$
$$= y_+(t) + y_-(t) - 2s_0(t) \tag{65}$$

Inserting the approximated expressions for the corrected signals from Eqs. (61,62) gives

$$\hat{x}_n(t) \approx x_l(t) + \left(a(t) - a_c\right)\int dt_0 \left(t - t_0\right)u\&(t - t_0, t_0)\sigma_l(t_0)$$
$$+ \left(\tau(t) - \tau_c\right)\int dt_0 u\left(t - t_0, t_0\right)\&_l(t_0) + H_{a_c}V_t^+ \left\{x_{n+}\left(t + \left(\tau(t) - \tau_c\right)\right)\right\}$$
$$+ w(t) + \left(a_1 - a_c\right)\int dt_0 \left(t - t_0\right)u\&(t - t_0, t_1)\sigma_w(t_0) + \left(\tau_1 - \tau_c\right)\int dt_0 u\left(t - t_0, t_1\right)\&_w(t_0)$$

$$+ x_l(t) - \left(a(t) - a_c\right)\int dt_0 \left(t - t_0\right)u\&(t - t_0, t_0)\sigma_l(t_0) \tag{66}$$
$$- \left(\tau(t) - \tau_c\right)\int dt_0 u\left(t - t_0, t_0\right)\&_l(t_0) - H_{-a_c}V_t^- \left\{x_{n-}\left(t - \left(\tau(t) - \tau_c\right)\right)\right\}$$
$$+ w(t) - \left(a_1 - a_c\right)\int dt_0 \left(t - t_0\right)u\&(t - t_0, t_1)\sigma_w(t_0) - \left(\tau_1 - \tau_c\right)\int dt_0 u\left(t - t_0, t_1\right)\&_w(t_0)$$

$$-2x_l(t) - 2w(t)$$

which reduces to

$$\hat{x}_n(t) \approx H_{a_c}V_t^+ \left\{x_{n+}\left(t + \left(\tau(t) - \tau_c\right)\right)\right\} - H_{-a_c}V_t^- \left\{x_{n-}\left(t - \left(\tau(t) - \tau_c\right)\right)\right\} \tag{67}$$

Using the 1st order Taylor expansion in a(t) in Eq.(61) we can approximate Eq.(67) to

$$\hat{x}_n(t) \approx x_{n+}\left(t + \left(\tau(t) - \tau_c\right)\right) - x_{n-}\left(t - \left(\tau(t) - \tau_c\right)\right)$$
$$+ \left(a(t) - a_c\right)\int dt_0 \left(t - t_0\right)u\&(t - t_0, t_0)\left\{\sigma_{n+}\left(t_0 + \left(\tau(t) - \tau_c\right)\right) + \sigma_{n-}\left(t_0 - \left(\tau(t) - \tau_c\right)\right)\right\} \tag{68}$$

For materials where the 2nd order approximation of the nonlinear elasticity in Eq.(1) is adequate, we have $x_{n-}(t) = x_{n+}(t)$ and $\sigma_{n-}(t) = \sigma_{n+}(t)$. However, when $\tau_c \neq \tau(t)$ and/or $a(t) \neq a_c$, the detection signal in Eq. (68) is nonzero also in this case. With scattering from micro gas bubbles we can have $x_{n-}(t) \neq x_{n+}(t)$ and $\sigma_{n-}(t) \neq \sigma_{n+}(t)$ which increases the detection signal. This is specially found for HF close to the bubble resonance frequency where a change in the polarity of the LF pulse changes the phase of the nonlinear scattering signal, or with so high LF pulse amplitude that the LF bubble compression shows strong deviation from the 2nd order nonlinearity.

We hence see that the sum of $y_+$ and $y_-$ in Eq.(65,66) gives robust and strong canceling of the pulse form distortion and the nonlinear propagation delays. Accurate assessment of $a_c$ and $\tau_c$ is hence not so critical which makes the procedures very robust. When $a(t) - a_c$ and $\tau(t) - \tau_c$ become so large that 2nd and higher order even terms in the Taylor series of $u[(1 \pm (a-a_c))(t-t_0), t_0]\sigma_h(t_0 \pm (\tau - \tau_c))$ become important, the suppression of the linear scattering and the pulse reverberation noise is reduced. However, by choosing $\tau_c$ close to $\tau_1$ and $a_c$ close to $a_1$, Eq.(63,66) gives good approximations for the pulse reverberation noise, and choosing $\tau_c$ close to $\tau(t)$ and $a_c$ close to $a(t)$ Eq.(66) gives good suppression for the linear scattering at fast time t. In many situations suppression of pulse reverberation noise is most important up to medium depths, while suppression of linear scattering is the most important for larger depths. One can then use a delay and compression correction $\tau_c \approx \tau_1$ and $a_c \approx a_1$ for the lower depths and a delay and compression

correction delay $\tau_c \approx \tau(t)$ and $a_c \approx a(t)$ for the larger depths.

We have in Eqs. (53-68) analyzed the situation of a single 1st scatterer, but as discussed in relation to Eq.(37), Class I and Class II noise always coexists, and are equal for zero LF pulse when the transmit and received beams are equal. Suppose we transmit two pulse complexes with opposite polarity of the LF pulse, and correct and subtract the received HF signals for the nonlinear propagation lags and possible pulse form distortions at the location of the 1st scatterer of Class I noise. This will produce a strong suppression of the Class I noise, but a limited suppression of the Class II noise. For the Class III noise the depths of the 1st and 3rd scatterers are so close that adequate suppression of this noise is found for both directions of the propagation path **702**.

To derive a method of suppression of all classes of pulse reverberation noise according to the invention, we note that when the transmit and receive beams are close to the same and the major 2nd scatterer is the transducer array itself, we can approximate $x_r(t;t_1)=x_r(t;t-t_1)$ in Eq.(40). This allows us to modify Eq.(40) to

$$s_k(t) = \int dt_0 v_{k1}\left(t-t_0;t_0\right)x_1(t_0) + \int dt_0 v_{k2}\left(t-t_0;t_0\right)x_2(t_0)$$
$$+ \int_0^{t/2} dt_1 \int dt_0 \left\{v_k^r\left(t-t_0;t_1\right)+v_k^r\left(t-t_0;t-t_1\right)\right\}x_r(t_0;t_1) \qquad (69)$$

As the operators are linear, we can merge the sum in the last integral to one operator, which gives

$$s_k(t) = \int dt_0 v_{k1}\left(t-t_0;t_0\right)x_1(t_0) + \int dt_0 v_{k2}\left(t-t_0;t_0\right)x_2(t_0)$$
$$+ \int_0^{t/2} dt_1 \int dt_0 v_k^s\left(t-t_0;t_1\right)x_r(t_0;t_1) \qquad (70)$$

$$v_k^s\left(t-t_0;t_1\right) = v_k^r\left(t-t_0;t_1\right)+v_k^r\left(t-t_0;t_0-t_1\right)$$

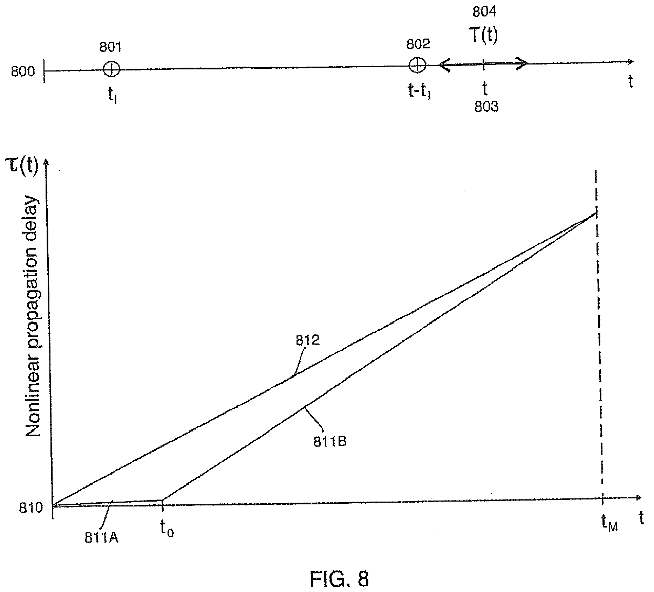

The Class III pulse reverberation noise is here included for $t_1 = t/2$. In **FIG. 8**, **800** shows a fast time scale with a strong 1st scatterer at at $t_1 = t_1$ (**801**). To have substantial Class I pulse reverberation noise at fast time t (**803**) with the 1st strong scatterer at $t_1 = t_1$, we must also have a 3rd strong scatterer at $t - t_1$, illustrated as **802** on the time scale **800**. We then have a similar Class II pulse reverberation noise where the 1st scatterer is at **802** ($t - t_1$), and the 3rd scatterer is at **801** ($t_1$), all where the 2nd scatterer is the transducer array. The sum of the Class I and Class II pulse reverberation noise is then from Eq.(70)

$$\Delta t_l \int dt_0 v_k^s\left(t-t_0;t_l\right)x_r(t_0;t_l) = \Delta t_l \int dt_0 \left(v_k^r\left(t-t_0;t_l\right)+v_k^r\left(t-t_0;t-t_l\right)\right)x_r(t_0;t_l)$$

Fourier transform of this expression gives

$$\Delta t_l V_k^s\left(\omega;t_l\right)x_r(\omega;t_l) = \Delta t_l \left\{V_k^r\left(\omega;t_l\right)+V_k^r\left(\omega;t-t_{1l}\right)\right\}X_r(\omega;t_l)$$
$$\qquad (71)$$
$$= \Delta t_l \left\{e^{-i\omega\tau_k(t_l)}\overset{o}{V}_k\left(\omega;t_l\right)+e^{-i\omega\tau_k(t-t_l)}\overset{o}{V}_k\left(\omega;t-t_l\right)\right\}X_r(\omega;t_l)$$

where we in the last line have separated out the nonlinear propagation time lag. The expression can be further modified as

$$= e^{-i\omega(\tau_k(t_l)+\tau_k(t-t_l))/2}\Delta t_l \left\{e^{-i\omega(\tau_k(t_l)-\tau_k(t-t_l))/2}\overset{o}{V}_k\left(\omega;t_l\right)+e^{i\omega(\tau_k(t_l)-\tau_k(t-t_l))/2}\overset{o}{V}_k\left(\omega;t-t_l\right)\right\}X_r(\omega;t_l)$$

$$= e^{-i\omega(\tau_k(t_l)+\tau_k(t-t_l))/2}\Delta t_1\left\{\left[\tilde{V}_k^{96}(\omega;t_l)+\tilde{V}_k^{96}(\omega;t-t_l)\right]\cos\left[\omega\left(\tau_k(t_l)-\tau_k(t-t_l)\right)/2\right]\right.$$
$$\left.-i\left[\tilde{V}_k^{96}(\omega;t_l)-\tilde{V}_k^{96}(\omega;t-t_l)\right]\sin\left[\omega\left(\tau_k(t_l)-\tau_k(t-t_l)\right)/2\right]\right\}X_r(\omega;t_l) \quad (72)$$

If the pulseform distortion at $t_1$ and t - $t_1$ is close to the same, which in practice means that the pulse form distortion is negligible, the last term vanishes, and we get

$$\approx e^{-i\omega(\tau_k(t_l)+\tau_k(t-t_l))/2}\Delta t_1\left[\tilde{V}_k^{96}(\omega;t_l)+\tilde{V}_k^{96}(\omega;t-t_l)\right]\cos\left[\omega\left(\tau_k(t_l)-\tau_k(t-t_l)\right)/2\right]X_r(\omega;t_l) \quad (73)$$

We can hence suppress the combined Class I and Class II pulse reverberation noise in an interval T(t) **(804)** around t, produced by strong scatterers **801/802** by correcting for the average nonlinear propagation delay $[\tau_k(t_l)+\tau_k(t-t_l)]/2$ and pulse form distortion

$$\left[\tilde{V}_k^r(\omega;t_l)+\tilde{V}_k^{96}(\omega;t-t_l)\right]\cos\left[\omega\left(\tau_k(t_l)-\tau_k(t-t_l)\right)/2\right].$$

For $\omega[\tau_k(t_l)-\tau_k(t-t_l)]/2$ sufficiently small, we can for narrowband signals approximate $\cos\omega[\tau_k(t_l)-\tau_k(t-t_l)]/2 \approx const\leq 1$. This procedure is specially interesting with combinations of the received HF signal from opposite polarity LF pulses similar to what is done in Eqs.(60-68) above, where sensitivity to the accuracy of the corrections are greatly reduced.

The variation of $\tau(t)$ with the fast time depends on the details of the LF beam and local position of the HF pulse relative to the LF pulse. When the LF transmit aperture has a large inactive region around the center, one can get a variation of $\tau(t)$ indicated as **811a/B** in the diagram **810** of **FIG. 8.** In this case one gets a near field region with very low LF pressure, which gives close to zero nonlinear propagation delay (and pulse form distortion) shown as **811A** in the Figure, followed by a close to linear increase **811B** of the nonlinear propagation delay. An advantage with this LF field is that $s_+(t) - s_-(t)$ or $s_+(t) - s_0(t)$ provides strong suppression of pulse reverberation noise where both the 1st and 3rd scatterers are in the near field region **(811A),** i.e. Class I, II, and III noise. The design of the LF radiation aperture is then conveniently done so that the part **811A** of $\tau(t)$ covers the body wall where one typically has very strong scatterers/reflectors. Suppression of pulse reverberation noise where both scatterers are in the body wall can then be strongly suppressed without corrections for nonlinear propagation delay and pulse form distortion. However, when one of the scatterers move into the region of manipulating LF pulse **(811B)** one should do a correction as described after Eq.(73), and we shall develop some details on this situation in relation to Eq.(74) below, but first we shall analyze the simpler situation of a close to linear variation of $\tau(t)$, shown as **812** in the Figure

With a large, full LF aperture so that the HF imaging region is in the near field, it is possible to obtain a close to linear increase of the nonlinear propagation delay, shown as **812.** In this situation we note that $[\tau_k(tl)+\tau_k(t-t_l)]/2\approx\tau_k(t/2)$

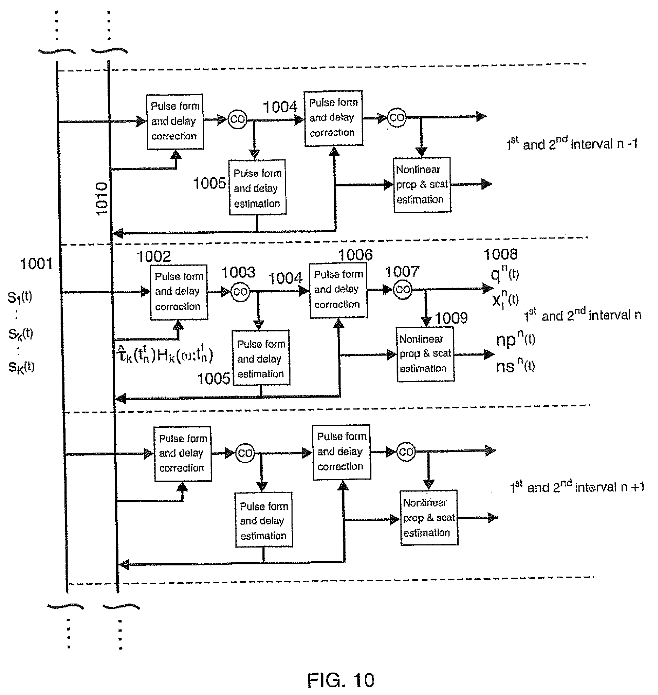

and $\tilde{V}_k^r(\omega;t_l)+\tilde{V}_k^{96}(\omega;t-t_l)\approx 2\tilde{V}_k^{96}(\omega;t/2)$, one hence corrects for combined Class I and II plus Class III pulse reverberation noise in an interval around t by correcting the received HF signals for the nonlinear propagation delay and pulse form distortion at t/2, before combination of the corrected HF signals. This stimulates the introduction of a multi-step suppression of multiple scattering noise as illustrated in the processing block diagram of **FIG. 10.** In this **Figure 1001** indicates the received HF signals $s_1(t)... s_k(t)...s_K(t)$ from K transmitted pulse complexes with variations in the LF pulse, and with k as the pulse number coordinate (slow time coordinate). The variations in the LF pulse can be in the amplitude and/or phase and/or frequency of the transmitted LF pulse, and one can also have variations in the LF transmit aperture and focus. The whole image depth is divided into a set of image intervals, referred to as 2nd intervals, where noise suppression and potential estimation of nonlinearly scattered signals, and nonlinear propagation and scattering parameters are done for each 2nd interval separately according to the block diagram of **FIG. 10** which we describe in more detail below. The 2nd intervals can optionally be placed at a region of special interest of the image depth.

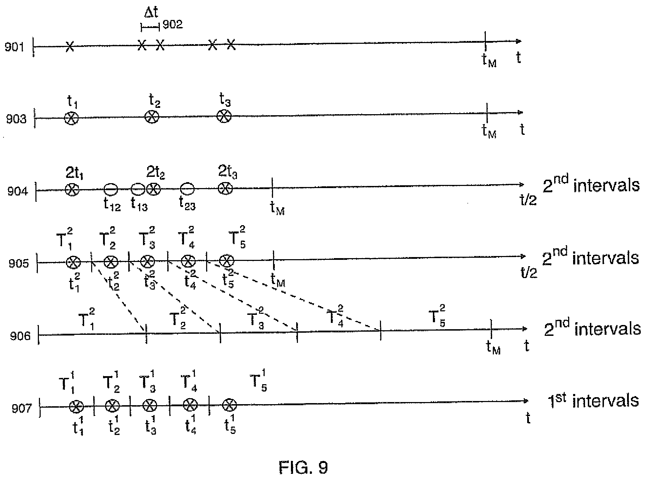

We first describe with reference to **FIG. 9** an example according to the invention on how said 2nd intervals can be selected.

**1. We** first select locations in the fast time of strong scatterers that produces visible pulse reverberation noise. These are by example indicated by the $\times$ on the fast time line **901** in the Figure. These scatterers can be selected manually, for example through direct pointing on the display screen of a 2D image, a M-mode or an A-mode display, etc., or through automatic selection of the strongest back scatter amplitudes compensated for attenuation in the object, or a combination of these. The manual pointing can be done with a cursor technique or touch screen technique according to known methods. The attenuation compensation can for example be done with the depth gain controls found in ultrasound imaging instruments, or based on assumed or estimated attenuation coefficients. The scatterers can be selected for each beam direction, or they can be the same for a group of beam directions or the whole image.

2. The rate of change of the nonlinear propagation lag and the pulse form distortion with fast time defines a resolution requirement $\Delta t$ along the fast time, indicated as **902** in the Figure. The nonlinear propagation delay and pulse form distortion changes accumulatively, Eq.(3), and hence vary slowly with depth. $\Delta t$ is therefore selected so that one has limited variation of the nonlinear propagation delay and pulse form distortion within $\Delta t$, where the tolerable variation depends on the required suppression of the pulse reverberation noise. Strong scatterers that are closer to each other than $\Delta t$ are then grouped together and merged and represented by an equivalent scatterer of the group, for example at the point or gravity, indicated by $\otimes$ on the fast time line **903** in the Figure. These equivalent scatterers have the fast time positions $t_i$, $i = 1, ..., I$, where the example in **903** of **FIG. 9** shows 3 equivalent scatterers with positions $t_1$, $t_2$, $t_3$ on the fast time line **903**.

3. The equivalent scatterers give rise to Class III pulse reverberation noise at the fast time noise points $2ti$ shown as $\otimes$ on the 2nd intervals fast time scale **904**, shown compressed versus $t/2$. The equivalent scatterers also generate Class I and II type pulse reverberation noise at the Class I/II time points $t_{ij} = t_i + tj$, $i,j = 1, 2, ..., I$. The locations of these Class I/II time points are in our example shown as $\square$ on the time line **904**.

4. Noise points of Class I/II and Class III noise on the line **904** that are closer than $2\Delta t$, are merged to groups and replaced by center noise point of the 2nd intervals, $t^2_n$, $n = 1, ..., N$, e.g. the point of gravity of the groups. This gives in our example the center noise points, $t^2_1$, $t^2_2$, $t^2_3$, $t^2_4$, $t^2_5$, indicated by $\otimes$ on the compressed fast time line **905**. The end points $(t^2_{sn}, t^2_{en})$ of the 2nd intervals $T^2_n$ can then for example be defined by the midpoints between center noise points that gives the 2nd intervals $T^2_1$, $T^2_2$, $T^2_3$, $T^2_4$, $T^2_5$ on the compressed fast time line **905** and the uncompressed fast time line **906**. The right endpoint of the last interval $(T^2_5)$ can typically be set to the maximal image depth $t_M$.

5. For suppression of pulse reverberation noise in the 2nd interval $T^2_n$, the received signals are corrected with the nonlinear propagation delay and pulse form distortion at the 1st interval correction time points $t^1_n = t^2_n/2$, and combined in the pulse number coordinate, for example as in Eqs.(56). The fast time line **907** shows as $\otimes$ the correction time points $t^1_1$, $t^1_2$, $t^1_3$, $t^1_4$, $t^1_5$ for the five 2nd intervals in the example. The midpoints between the correction points can be used to define the 1st set of intervals $T^1_n = (t^1_{sn}, t^1_{en})$ defined for the continuous distribution of noise generating scatterers described below.

The processing block diagram in **FIG. 10** is divided into a set of parallel processing structures for each 2nd interval $T^2_n = (t^2_{sn}, t^2_{en})$. The parallel structure is shown for overview, and in a practical processor, the processing can be done in time series, starting with the 1st set of paired intervals. To obtain the noise suppressed signal for the n-th 2nd interval, the received HF signals are in unit **1002** delay corrected with estimates of the nonlinear propagation delay, and potentially also pulse distortion, at a characteristic depth $t^1_n$ in the pair wise matched 1st interval $T^1_n = (t^1_{sn}, t^1_{en})$ to form corrected HF signals for the nth 2nd interval. Said corrected HF signals are then combined in unit **1003** in the pulse number coordinate, for example through a high pass filter along the pulse number coordinate (slow time), to form a set of noise suppressed HF signals **1004**, $z^n_1(t), ..., z^n_m(t), ..., z^n_M(t)$, for the pair wise matched 2nd interval $T^2_n$ where the pulse reverberation noise (multiple scattering noise) is highly suppressed, similar to Eq. (56).

The noise suppressed signals for each 2nd intervals $T^2_n = (t^2_{sn}, t^2_{en})$ are then in unit **1005** used for estimation of corrections for the nonlinear delays $\hat{\tau}(t)$ and pulse distortion correction in said each 2nd intervals. One of the received HF signals, and the noise suppressed signals, are then used as intermediate HF signals, and said intermediate HF signals are corrected for nonlinear propagation delay and potentially also pulse form distortion in said each 2nd intervals in unit **1006** to form corrected intermediate HF signals that are combined in unit **1007** for strong suppression of the linear scattering in the HF signals to form estimates **1008** of nonlinear scattering HF signals that represent nonlinear scattering from the object in $T^2_n = (t^2_{sn}, t^2_{en})$, $\hat{x}_n(t)$, as exemplified in Eq.(58). Similarly can one estimate the linear scattering HF signal for the 2nd interval $T^2_n$, $\hat{x}_l(t)$, as exemplified in Eq.(59).

From the estimated nonlinear propagation delay one estimates the nonlinear propagation parameter, Eqs.(9-11), and from the envelopes of the nonlinear and linear scattering HF signals one estimates the nonlinear scattering parameter, Eqs.(15-17), in unit **1009**. Signal based estimation of the nonlinear propagation delay $\hat{\tau}(t_1)$ and pulse distortion correction for a particular 1<sup>st</sup> interval can typically be estimated from the noise suppressed signals **1004** in a unit **1005.** The estimates from this unit is used directly for correction for nonlinear propagation delay and pulse form distortion for suppression of linear scattering HF signal components in the 2<sup>nd</sup> intervals in unit **1006**. The estimates are also communicated on the bus **1010** so that in the correction unit **1002** the corrections for the nonlinear propagation delays and pulse form distortions at the time points $t^1_n$ for the 1<sup>st</sup> intervals can be taken from the estimates of a previous block.

In some situations one has a dense distribution of strong scatterers that produces pulse reverberation noise, where it is difficult to select an adequate discrete set of strong scatterers as marked by the $\times$ on the fast timeline **901** in **FIG**. **9.** We can then by example divide the region of strong scatterers into a set of 1<sup>st</sup> intervals $T_{1n}$ with endpoints $(t^1_{sn}, t^1_{en})$, of maximal length $\Delta t$, and represent the scatterers in each interval by an equivalent scatterer at $t^1_n$, for example as the $\otimes$ shown on the timeline **907** in **FIG**. **9**. The intervals and equivalent scatterers can be selected for each beam direction, or they can be the same for a group or beam directions or the whole image. We then define the set of 2<sup>nd</sup> intervals, $T^2_n = (t^2_{sn}, t^2_{en})$ where each 2<sup>nd</sup> interval $T^2_n$ corresponds to the 1<sup>st</sup> interval $T^l_n = (t^1_{sn}, t^1_{en})$, pair wise. The nonlinear propagation delays and pulse form distortions at the equivalent scatterers in the 1<sup>st</sup> intervals are then used to define corrections for the nonlinear propagation delay and pulse form distortion in the corresponding 2<sup>nd</sup> intervals before the signals are combined to produce noise suppressed HF signals **904**, $z^n_1(t), ..., z^n_m(t), ..., z^n_M(t)$, for the 2<sup>nd</sup> interval $T^2_n = (t^2_{sn}, t^2_{en})$.

One can also work the opposite way, where one starts by selecting the fast time $t^2_n$ as the center of a 2<sup>nd</sup> interval where one wants to correct for pulse reverberation noise, and then apply the corrections for the nonlinear propagation delays and pulse form distortion at $t/2$ before the signals are combined to produce noise suppressed HF signals **904**, $z^n_1(t), ..., z^n_m(t), ..., z^n_M(t)$, for the 2<sup>nd</sup> interval $T^2_n = (t^2_{sn}, t^2_{en})$. This procedure will automatically select the intervals. The advantage with the 1<sup>st</sup> procedure where one starts by selecting the strong scatterers that produces pulse reverberation noise, is that we directly address the regions where the pulse reverberation noise is strong. There will always be some errors in the procedure, particularly in the estimation of the corrections for the nonlinear propagation delay and pulse form distortion, and these errors might introduce visible noise where the pulse reverberation noise is low.

The nonlinear propagation delay and pulse form distortion changes accumulatively, Eq.(3), and hence vary slowly with depth. The length of the 1<sup>st</sup> intervals $T^1_n$ is therefore often selected so that one have limited variation of the HF nonlinear propagation delay and HF pulse distortion within each interval (f.ex. given by $\Delta t$ in **902**), where the limit is given by the required suppression of the pulse reverberation noise. The tolerable variation of the nonlinear propagation delay and pulse form distortion also depends on how many strong scatterers/reflectors are found within said 1<sup>st</sup> interval. One can similarly set the boundaries between said 1<sup>st</sup> intervals fixed, or fixedly related to the selected measurement or image depth, with a possibility to adjust the boundaries from the instrument control or automatically by a signal processor, for improved noise suppression.

When the nonlinear delay and pulse distortion has a zero interval in the near-field, as shown by **811A** in **FIG. 8,** we get a more complex relationship between 2<sup>nd</sup> intervals and the time point that defines the corrections for the nonlinear propagation delay and pulse form distortion for the pulse reverberation noise. We first note that suppression of pulse reverberation noise where both scatterers are within **811A** can be done without corrections for nonlinear propagation delay and pulse form distortion. When one of the scatterers move into the region of manipulating LF pulse (**811B**) $(t_1 > t_0)$ we note that the average propagation delay, as described in Eq.(73), takes the form

$$\frac{1}{2}\left\{\tau_k(t_l) + \tau_k(t - t_l)\right\} = \begin{cases} \dfrac{\tau_k(t - t_1)}{2} = \dfrac{\tau_k(t)}{2}\dfrac{t - t_l - t_0}{t - t_0} & for \qquad t_l < t_0 \\[4mm] \dfrac{\tau_k(t - t_0)}{2} = \dfrac{\tau_k(t)}{2}\dfrac{t - 2t_0}{t - t_0} & for \; t > 2t_0 \; and \; t_0 < t_l < t/2 \end{cases} \qquad (74)$$

To suppress Class I and II pulse reverberation noise combined at a fast time t, we can for example select a set of strong scatterers in the near field, for example as on line **901** discussed under point 1 in relation to **FIG. 9** above. The delay correction for Class I and II noise for each of the strong scatterers is then given by Eq.(74), and we merge the scatterers together with accuracy $\Delta t$ similar to point 2 in the description for **FIG. 9.** We then discretize Eq.(70) around the merged strong scatterers (on line **903**) and establish a set of measurement equations similar to Eqs.(43), where $V_{kl}\{x_l(t)\}$, $1 = 3, ..., L$, represents the combined Class I - III pulse reverberation noise for L-2 1<sup>st</sup>/3<sup>rd</sup> scatterers. We note that the nonlinear propagation delay and pulse form distortion for pulse reverberation noise where both scatterers are in the region of manipulating LF pulse, i.e. $t_1 > t_0$, will for all Class I/II/III be equal to the value at t/2, which for the nonlinear propagation delay is $\tau_k(t/2) = \tau_k(t-t_0)/2$. Class I/II/III pulse reverberation noise can for these scatterers be grouped into one term. Eq.(70) can then be approximated by a set of linear operator equations similar to Eqs.(43,48) which can be solved according to similar methods.

When **811A** covers the body wall (i.e. the body wall is inside $t_0$) and the image point t < $2t_0$, the pulse reverberation noise is often dominated by noise where both the 1st and 3rd satterer is inside **811A.** We then get good suppression of pulse reverberation noise without correction for nonlinear propagation delay and pulse form distortion. Another situation for approximation is when to is adequately short in relation to the image depth. We can then often for deeper image ranges approximate the nonlinear propagation delay and pulse form distortion for pulse reverberation noise of Class I-III to originate at a fixed point $t_c(t) \approx t/2 + offset,$ and the methods described in relation to the fully linear delay curve **812** can then be applied with the corrections for the nonlinear pulse delay and pulse form correction at $t_c(t)$ to suppress the Class I-III pulse reverberation noise.

Said nonlinear and linear scattering HF signals are then used for further processing to form final measurement or image signals, for example according to known methods such as envelope detected signals, Doppler signals, fast time spectral content signals, etc.

The noise suppressed HF signals and the nonlinear scattering signals are useful both for backscatter imaging and for computer tomographic (CT) image reconstructions from forward transmitted and angularly scattered signals. The nonlinear propagation lag, Eq.(3), and the integrated pulse distortion is observable directly for the transmitted wave through the object, and are also candidates for CT image reconstruction of local object elasticity parameters.

The suppression of pulse reverberation noise in the signals greatly improves the estimation of corrections for wave front aberrations in the element signals from 1.75D and 2D arrays, for example according to the methods described in US Pat 6,485,423, US Pat 6,905,465, US Pat 7,273,455, and US Pat Appl US2005277835 and US2006052699 . When estimation of the corrections for the wave front aberrations are based on signal correlations with the summed beam-former output signal with highly suppressed reverberation noise, the reverberation noise in the element signals is uncorrelated to the beam-former output signal. When slow updates of the aberration correction estimates are acceptable, one can use so long correlation time that the effect of the reverberation noise in the element signals on the correction estimates can be negligible. However, when the correlation time is low, it is preferable to also suppress the reverberation noise in the element signals before the estimation of the aberration corrections, to reduce errors in the aberration correction estimates produced by the reverberation noise.

Similar expressions as discussed for the bulk pressure waves above, can be developed for shear waves in solids, and while we do many of the derivations with reference to bulk pressure waves, it is clear to anyone skilled in the art that the invention is also applicable to shear waves.

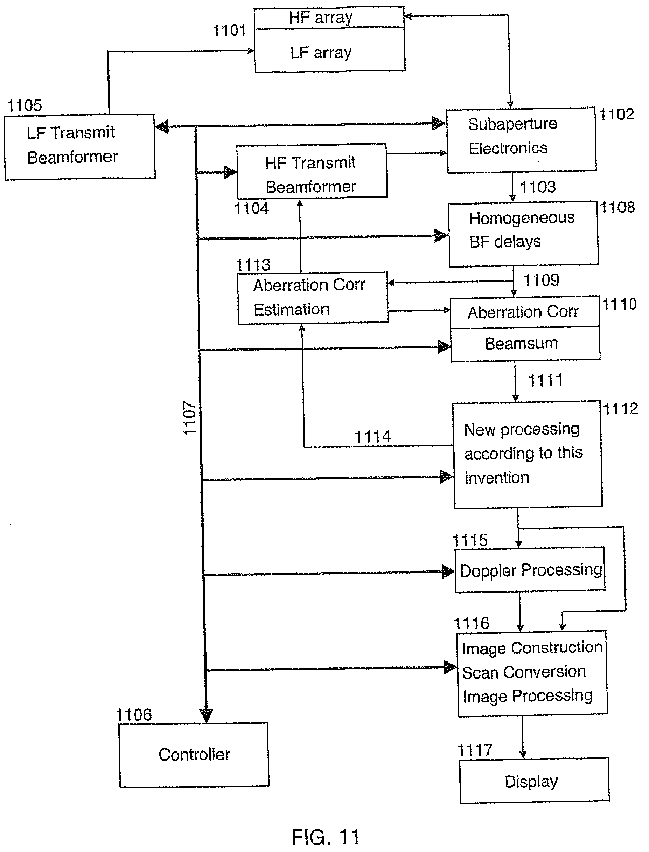

A block diagram of an imaging instrument that uses the described methods for back scatter imaging in its broadest sense according to the invention, is shown in **FIG. 11,** where **1101** shows the acoustic transducer array that has a high frequency (HF) and low frequency (LF) section. In this broadest implementation of the methods, the array has a two dimensional distribution of elements, which allows full electronic 3D steering of the high and the low frequency beams, referred to as 2D array, and the instrument is also capable of both estimating and correcting for wave front aberrations. It is clear however that the methods can be used with less complex arrays, as discussed bellow.

The high frequency part of the array can in full 3D imaging applications have a large number of elements, for example 3000 - 10,000, and the number of receive and transmit channels are then typically reduced in a sub-aperture unit **1102,** where in receive mode the signals from several neighboring array elements are delayed and summed to sub-aperture signals **1103** for further processing. For aberration corrections, the widths on the array surface of the sub-aperture groups are less than the correlation length of the wave front aberrations, where a typical number of sub-aperture groups and signals could be 100 - 1000.

For transmission of the pulse complexes, the HF transmit beam former **1104** feeds pulses to the sub-aperture unit **1102,** that delays and distributes the signals to all or sub-groups of HF-array elements, while the LF transmit beam former **1105** simultaneously feeds pulses to the LF array elements. The pulse complex transmission is triggered by the instrument controller **1106,** which communicates with the sub-units over the instrument bus **1107.**

The receive sub-aperture signals **1103** are fed to the unit **1108,** where the sub-aperture signals are delayed for steering of receive beam direction and focusing under the assumption of a homogeneous medium with the constant, average propagation velocity, referred to as homogeneous delays. 3D beam steering and focusing can also be done with sparse arrays, where the sub-aperture unit **1102** could typically be missing. With 1.75 D arrays, the number of HF array elements can also be reduced so much that the sub-aperture units could be left out. In the following we therefore use element and sub-aperture signals synonymously.

The element signals that are corrected with the homogenous delays, **1109,** are fed to a unit **1110** where corrections for the wave front aberrations are applied, for example estimated according to the methods described in US Pat 6,485,423, 6,905,465, 7,273,455, and US Pat Appl US2005277835 and US2006052699 , before the element signals are summed to the final receive beam signal. For 3D imaging one would use multiple receive beams with small angular offsets that covers a wide transmit beam in parallel (simultaneously). The aberration corrections for the angularly offset beams could be a side shifted version of the corrections for the central beam, that are added together with the homogeneous delays for the angular offset in the unit **1110**. The output **1111** of the unit **1110** is hence one or more RF-signals for one or more receive beam directions in parallel, that is fed to the processing unit **1112** according to this invention, that performs one or more of the operations according to the methods described above and Eqs.(21-26, 43-74, 6-17).

The aberration corrections are estimated in the unit **1113,** for example according to the methods described in relation to the cited patents and patent applications. The unit **1113** takes as its input the homogeneously delay corrected signals **1109** and possibly also final beam signals **1114** with suppression of the pulse reverberation noise according to this invention. The delay corrected element signals **1109** are typically first processed with methods according to this invention, typically the method described in relation to **FIGs. 6-10** and Eqs. **(40-73)** to suppress the pulse reverberation noise before estimation of the delay corrections. One should note that use of signal from moving scatterers as for example found with blood or myocardium and as prescribed in US Pat 6,485,423, would improve the function of methods of suppression of pulse reverberation noise. The estimates based on the nonlinear propagation delays for the individual element/sub-aperture signals as described in and US Pat Appl US2005277835 and US2006052699 also represent interesting estimates themselves, and also as a starting point to for further estimations according to the cited patents, both to focus the 1st transmit beam and as starting points of an iteration scheme.

When estimation of the corrections for the wave front aberrations are based on signal correlations with the beam-former output signal **1114** with highly suppressed reverberation noise, the reverberation noise in the element signals is uncorrelated to the beam-former output signal. When slow updates of the aberration correction estimates are acceptable, one can use so long correlation time that the effect of the reverberation noise in the element signals on the correction estimates can be negligible. However, when the correlation time is low, it is preferable to also suppress the reverberation noise in the element signals before the estimation of the aberration corrections, to reduce errors in the aberration correction estimates produced by the reverberation noise.

The outputs of the unit **1112** are the linearly and nonlinearly scattered signals with suppression of pulse reverberation noise, the two quantitative nonlinear parameters, and Doppler phase and frequency data. These data can be fed directly to the image construction and scan converter unit **1116** that presents images of compressed and colorized versions of the amplitudes of the linearly and nonlinearly scattered signals, the quantitative nonlinear parameters/signals, and object radial displacements, velocities, displacement strains and strain rates. However, to measure the radial velocities of blood or gas bubbles or other objects or fluids, one must further process the linearly or nonlinearly scattered signals in the slow time domain to suppress clutter echo from the object to retrieve the fluid signals for Doppler processing according to known methods, which is done in unit **1115**. The outputs of this unit are fed to the image construction unit **1116** to be selected and overlaid the images of the other information. The unite **1116** feeds its output to a display **1117**.

Many of the units of the instrument can be implemented as software programs in a general programmable computer, particularly the units **1110, 1112, 1113, 1115, 1116,** and **1117,** where with added support processors, like a graphics processor, unit **1108** can also be implemented as software in the same computer.

It should be clear to any-one skilled in the art, that many simplifications of the instrument as presented in **FIG**. **11** can be done while still utilizing essential aspects of the invention in the instrument. For example one can have a coarse division of elements in the elevation direction, which would limit electronic direction steering of the beam in the elevation direction, while one still can obtain corrections for the wave front aberrations and dynamic focusing with depth in the elevation direction. This is often referred to as 1.75D arrays and has much less total number of array elements than 2D arrays for full 3D steering of the beam, whereby the sub-aperture unit could be removed. Sparse arrays are another way to remove the number of elements so that it becomes practical to remove the sub-aperture unit **1102**. However, the gain in using the sub-aperture unit is found as long as the dimension of the sub-aperture group along the array surface is less than the correlation length of the wave front aberrations.

One could also remove the estimations and the corrections for the wave front aberrations, i.e. units **1110** and **1113,** and still be able to do the processing in unit **1112** to produce both linearly and nonlinearly scattered signals etc. as described above. The array could then be further simplified where elements symmetrically around the beam scan axis (the azimuth axis) are galvanically connected to further reduce the number of independent channels, often referred to as 1.5D arrays. One could similarly use one dimensional (ID) arrays and also annular arrays with mechanical scanning of the beam direction, where the main modification to the block diagram in **FIG**. **11** is that the sub-aperture unit **1102,** the aberration correction unit **1110** and aberration correction estimation unit **1113** are removed. Hence, the invention defines instruments with different complexity, or selectable complexity, and also instruments that can select between the different methods of processing described above, for best performance according to the measurement situation. The complexity of operation can be selected automatically by the instrument controller according to the application selected, or manually by the instrument operator to optimize image quality.

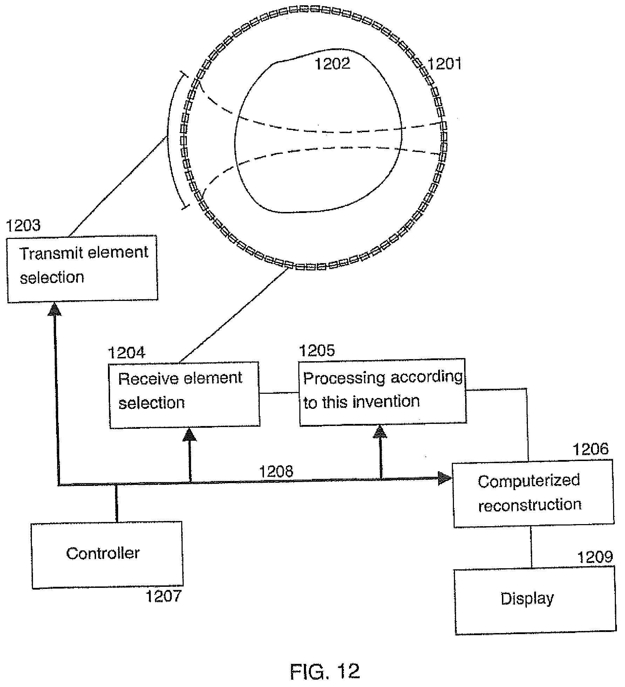

For tomographic image reconstruction, the processing according to this invention would typically be done on the individual receive element signals, before the signals are processed according to the reconstruction algorithms of various kinds found in prior art. A block schematic of a typical instrument for tomographic image reconstruction according to the invention is shown in **FIG. 12.** The Figure shows measurements with a ring array **1201,** where it is clear for anyone skilled in the art that other array configurations, also transducer arrays that would wholly or partly use mechanical scanning to collect the data, could be used without departing from the invention. The array surrounds the object **1202.** A unit **1203** selects a group of transmit elements, freely out of all the elements, and generates a transmit pulse complex composed of a low and a high frequency pulse overlapping in time and for example as visualized in **FIG. 1.** Transmissions of the pulse complexes are triggered by the controller unit **1207** via the controller bus **1208.** The unit **1204** selects receive elements, sequentially or in parallel or a combination of parallel-sequential manner, from the whole group of elements, and amplifies and digitizes the element signals for further processing according to the invention in the unit **1205.** This unit operates according to the principles according to the invention. The processing in unit **1205** provides one or more of the linearly scattered and transmitted signals with substantial suppression of the pulse reverberation noise (multiple scattering), nonlinearly scattered signals, and quantitative nonlinear propagation and scattering parameters that are forwarded to the unit **1206** that provides computerized tomographic images of 2D slices of the object. By mechanically moving the array relative to the object in the direction normal to the Figure, one obtains a 3D reconstructed image of the object.

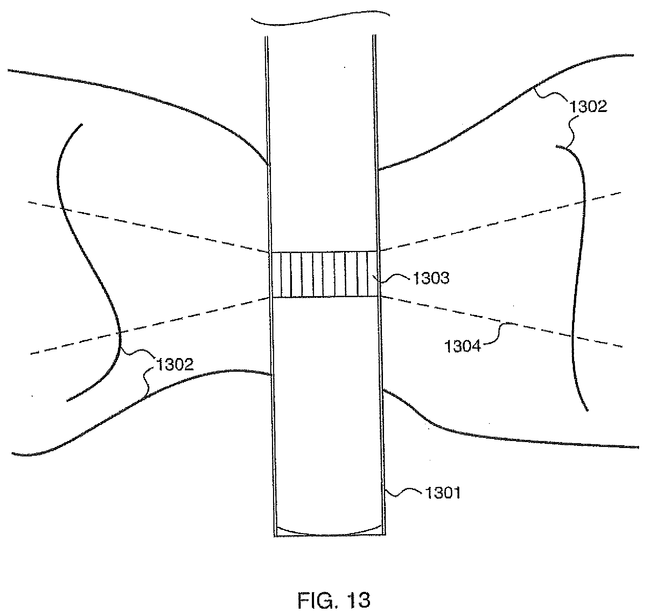

An example instrumentation for use of the methods for acoustic imaging of geologic structures around an oil well, is shown in **FIG. 13. 1301** indicates the perforated oil-well production liner with surrounding geologic structures **1302** that typically is composed of porous rock filled with oil, gas, water or mixtures of these, where also solid rock regions can be found. **1303** illustrates an acoustic array for transmission and reception of acoustic pulse complexes according to the invention in selectable sector beams **1304** around the production liner. The received acoustic signals are processed according to the methods described above with the exemplified instrumentation shown in **FIG. 11.** During drilling, one can also use a single element transduce, or annular array transducer, or a linear array transducer mounted vertically to provide a vertical 2D image of the rock around the well, that is mounted to the rotating drill string above the drill bit, for continuous 360 deg acoustic observation of the rock around the drill with the methods of back scatter imaging according to the invention. With the vertical linear array (switched or phase array) one obtains a 3D mapping of the rock properties around the well. Similar 3D mapping is obtained with single element or annular array transducers as the drill moves through the rock. With oil wells sufficiently close to each other one can also use transmission measurements between oil wells and reconstruct images from transmission and angular measurements as described in relation to **FIG. 12.**

**Claims**

**1.** A method for measurement or imaging of nonlinear scattering of elastic waves from a region of an object, where

   a) at least two elastic wave pulse complexes (101, 104; 501, 504) are transmitted towards said region where said pulse complexes are composed of a pulse (103, 106, 107, 109, 111-113; 311, 313, 315; 503, 506, 512, 522) in a high frequency, hereinafter referred to as HF, band and a pulse (102, 105, 108, 110; 312, 314, 316; 401, 402, 411, 412; 502, 505; 511, 521) in a low frequency, hereinafter referred to as LF, band with the same or overlapping beam directions and where the HF pulse is spatially so close to the LF pulse that the HF pulse observes the nonlinear modification of the object by the LF pulse at least for a part of the image depth, and where at least the transmitted LF pulse varies for each transmitted pulse complex in order to nonlinearly manipulate the scatterer elasticity observed by the HF pulse along at least for a part of the image depth, where the LF pulse of a pulse complex can be zero and the LF pulse for at least one pulse complex is nonzero, and where
   b) received HF signals (604, 606, 609, 610-612; 706) are picked up by transducers (208; 301; 601; 700; 1101; 1201; 1303) from one or both of scattered and transmitted HF components from at least two transmitted pulse complexes, and

   processing said received HF signals to form measurement or image signals for display,
   where said processing is **characterized in that**:

   c) in the process of forming said measurement or image signals, intermediate HF signals are formed from said received HF signals as said received HF signals, and where said intermediate HF signals are

      - delay corrected in the fast time, i.e. depth-time, and
      - pulse distortion corrected in the fast time, so as to form corrected intermediate HF signals from at least two transmitted pulse complexes with differences in the LF pulse, said corrected intermediate HF signals from different pulse complexes are combined to form nonlinear measurement HF signals that represent the object local nonlinear elastic properties, and where said nonlinear measurement HF signals are used for further processing to form measurement or image signals.

2. A method according to claim 1, where the pulse distortion correction is done as at least one of

- fast time expansion/compression in intervals of the signal, and
- frequency shifting through frequency mixing, and
- filtering in the fast time in a pulse distortion correction filter, and
- pre-distortion of the transmitted HF pulses, and
- amplitude correction.

3. A method according to claim 1, wherein said correction delays are obtained from one of

i) simulations of composite LF and HF elastic wave fields using local wave propagation parameters that have one of assumed values based on material knowledge prior to the measurements, and values obtained by manual adjustment for minimization of at least one of the pulse reverberation noise and the linear scattering signal in an image display, and
ii) manual adjustment of said correction delays for maximal suppression of one or both of

iia) multiple scattering noise in the an image as shown on a display screen, and
iib) linearly scattered signal in the image as shown on a display screen, and

iii) are estimated through signal processing techniques on one of said received HF signals and said noise suppressed HF signals.

4. A method according to claim 1, where said pulse distortion corrections are obtained from one of

i) simulations of the composite LF and HF elastic wave fields using local wave propagation parameters that have one of

ia) assumed values based on material knowledge prior to the measurements, and
ib) values obtained by manual adjustment for minimization of at least one of the pulse reverberation noise and the linear scattering signal as seen on an image display, and
ic) values obtained from the fast time gradient of obtained correction delays, and

ii) direct estimation of the pulse distortion from the frequency spectrum of one of the received HF signals and noise suppressed HF signals.

5. A method according to claim 1, where the time relation between the transmitted HF and LF pulses is selected so that for the actual imaging range the HF pressure pulse is found at zero-crossings of a pressure of the LF pulse to enhance in said nonlinear scattered HF signals the signal scattered from resonant scatterers with resonance frequency close to the center frequency of the LF pulse.

6. A method according to claim 1, wherein said at least two intermediate HF signals from at least two transmitted HF pulses also are combined to provide linear measurement HF signals that represent linear scattering from the object with a same attenuation as said nonlinear measurement HF signals from the object, and a local nonlinear scattering parameter is formed as the ratio of the local envelopes of said nonlinear measurement HF signals and said linear measurement HF signals.

7. A method according to claim 6, where a quantitative local scattering parameter is obtained by normalizing said local nonlinear scattering parameter with an estimate of the local LF pressure amplitude at the location of the propagating HF pulse.

8. A method according to claim 1, where a local propagation parameter of the object is obtained from the fast time gradient of said correction delays.

9. A method according to claim 8, wherein a local quantitative propagation parameter is obtained by normalizing the local propagation parameter with an estimate of a local LF pressure amplitude at a location of the propagating HF pulse.

10. A method according to claim 7 or 9, where one or both of said quantitative local scattering parameter and said quantitative local propagation parameter is used to estimate a local temperature of the object.

11. A method according to claim 1, where said nonlinear measurement HF signal are used for computer tomographic image reconstructions.

12. A method according to claim 1, where at least one of

- the polarity of the transmitted HF pulse, and
- the phase of the transmitted HF pulse, and
- the amplitude of the transmitted HF pulse is varied for each transmitted pulse complex, and this variation is compensated for in the processing of the received HF signal.

13. An instrument for measurement or imaging with elastic waves in a region of an object, comprising

a) transmit means for transmitting at least two elastic wave pulse complexes (101, 104; 501, 504), towards said region each of the pulse complexes composed of a pulse (103, 106, 107, 109, 111-113; 311, 313, 315; 503, 506, 512,522) in a high frequency, hereinafter referred to as HF, band and a pulse (102, 105, 108, 110; 312, 314, 316; 401, 402, 411, 412; 502, 505; 511, 521) in a low frequency, hereinafter referred to as LF, band with the same or overlapping beam directions and where the HF pulse is spatially so close to the LF pulse that said HF pulse observes the nonlinear modification of the object by the LF pulse at least for a part of the image depth, and at least the transmitted LF pulse varies for each transmitted pulse complex in order to nonlinearly manipulate the scatterer elasticity observed by the HF pulse along at least for a part of the image depth, where the LF pulse of a pulse complex can be zero, and the LF pulse for at least one pulse complex is nonzero, and
b) transducer (208; 301; 601; 700; 1101; 1201; 1303) and receiver means for picking up received HF signals (604, 606, 609, 610-612; 706) from one or both of scattered and transmitted HF components from at least two transmitted pulse complexes, and
c) a processor configured for processing the received HF signals to form said measurement or image signals;

**characterized in that** said processor includes means:

to form intermediate HF signals from said received HF signals as said received HF signals,
to delay correct said intermediate HF signals in the fast time, and
to pulse distortion correct said intermediate HF signals in the fast time, so as to form corrected intermediate HF signals from at least two transmitted pulse complexes with differences in the LF pulse, said corrected intermediate HF signals from different pulse complexes are combined to form nonlinear measurement HF signals that represent the object local nonlinear elastic properties, and where said nonlinear measurement HF signals are used for further processing to form measurement or image signals.

14. An instrument according to claim 13, wherein said processor includes means for one of calculation and estimation of at least said correction delays, and said pulse distortion corrections.

15. An instrument according to claim 13, where said processor includes means to estimate one or more of measurement or image signals that represent,

- linear scattering from the object, and
- nonlinear scattering from the object, and
- a nonlinear propagation parameter of the object, and
- a quantitative nonlinear propagation parameter of the object, and
- a local nonlinear scattering parameter of the object, and
- a quantitative, local nonlinear scattering parameter of the object, and
- at least one of object radial displacement, and
- at least one of displacement strain and strain rate, and
- local variations in object temperature.

16. An instrument according to claim 13, where the LF transmit aperture is a large, unfocused aperture so that the HF imaging range is within the near-field of the LF aperture.

17. An instrument according to claim 13, that includes means for estimation of wave front aberration corrections and means for correcting for wave front aberrations in the beamforming. 4694608-1-TPROCK

**Patentansprüche**

1. Verfahren zur Messung oder Abbildung nichtlinearer Streuung elastischer Wellen aus einem Bereich eines Objekts, worin

a) mindestens zwei Impulskomplexe elastischer Wellen (101, 104; 501, 504) zu dem Bereich übertragen werden, worin die Impulskomplexe aus einem Impuls (103, 106, 107, 109, 111-113; 311, 313, 315; 503, 506, 512, 522) in einem Hochfrequenzband, nachfolgend als HF bezeichnet, und einem Impuls (102, 105, 108, 110; 312, 314, 316; 401, 402, 411, 412; 502, 505; 511, 521) in einem Niederfrequenzband, nachfolgend als NF bezeichnet, mit den gleichen oder überschneidenden Strahlrichtungen zusammengesetzt sind und worin der HF-Impuls räumlich so dicht am NF-Impuls ist, dass der HF-Impuls zumindest für einen Teil der Bildtiefe die nichtlineare Modifikation des Objekts durch den NF-Impuls beobachtet, und worin mindestens der übertragene NF-Impuls für jeden übertragenen Impulskomplex variiert, um die durch den HF-Impuls beobachtete Elastizität des Streuobjekts entlang mindestens eines Teils der Bildtiefe nichtlinear zu manipulieren, worin der NF-Impuls eines Impulskomplexes null sein kann und der NF-Impuls für mindestens einen Impulskomplex von null verschieden ist, und worin
b) empfangene HF-Signale (604, 606, 609, 610-612; 706) durch Messwandler (208; 301; 601; 700; 1101; 1201; 1303) aus einer oder beiden von gestreuten und durchgelassenen HF-Komponenten aus mindestens zwei übertragenen Impulskomplexen aufgenommen werden, und

Verarbeiten der empfangenen HF-Signale, um Messungs- oder Bildsignale zur Anzeige zu bilden, wobei das Verarbeiten **dadurch gekennzeichnet ist, dass**:

c) beim Prozess des Bildens der Messungs- oder Bildsignale aus den empfangenen HF-Signalen HF-Zwischensignale als die empfangenen HF-Signale gebildet werden, und worin die HF-Zwischensignale

- in der Schnellzeit, d.h. Tiefenzeit, verzögerungskorrigiert werden, und
- in der Schnellzeit impulsverzerrungskorrigiert werden,

um dadurch korrigierte HF-Zwischensignale aus mindestens zwei übertragenen Impulskomplexen mit Differenzen im NF-Impuls zu bilden, wobei die korrigierten HF-Zwischensignale aus unterschiedlichen Impulskomplexen kombiniert werden, um nichtlineare HF-Messungssignale zu bilden, welche die nichtlinearen elastischen Objekteigenschaften darstellen, und worin die nichtlinearen HF-Messungssignale zur weiteren Verarbeitung verwendet werden, um Messungs- oder Bildsignale zu bilden.

2. Verfahren nach Anspruch 1, worin die Impulsverzerrungskorrektur als mindestens eines von Folgendem durchgeführt wird:

- Schnellzeit-Expansion/Kompression in Intervallen des Signals, und
- Frequenzverschiebung durch Frequenzmischung, und
- Schnellzeitfiltern in einem Impulsverzerrungskorrekturfilter, und
- Vorverzerrung der übertragenen HF-Impulse, und
- Amplitudenkorrektur.

3. Verfahren nach Anspruch 1, worin die Korrekturverzögerungen aus einem von Folgendem erlangt werden:

i) Simulationen von zusammengesetzten elastischen NF- und HF-Wellenfeldern unter Verwendung lokaler Wellenausbreitungsparameter, die eines von Folgendem haben:

angenommene Werte, die auf Materialkenntnis vor den Messungen beruhen, und
Werte, die durch manuelle Anpassung zur Minimierung von mindestens einem von Folgendem erlangt werden: Impulsechorauschen und das lineare Streusignal in einer Bildanzeige, und

ii) manuelle Anpassung der Korrekturverzögerungen zur maximalen Unterdrückung eines oder beider von Folgendem:

iia) Mehrfachstreuungsrauschen im Bild, wie es auf einem Anzeigeschirm gezeigt wird, und
iib) linear gestreutes Signal im Bild, wie es auf einem Anzeigeschirm gezeigt wird, und

iii) durch Signalverarbeitungsmethoden an einem der empfangenen HF-Signale und der entrauschten HF-Signale geschätzt werden.

4. Verfahren nach Anspruch 1, worin die Korrekturverzögerungen aus einem von Folgendem erlangt werden:

i) Simulationen der zusammengesetzten elastischen NF- und HF-Wellenfelder unter Verwendung lokaler Wellenausbreitungsparameter, die eines von Folgendem haben:

ia) angenommene Werte, die auf Materialkenntnis vor den Messungen beruhen, und
ib) Werte, die durch manuelle Anpassung zur Minimierung von mindestens einem von Folgendem erlangt werden: Impulsechorauschen und das lineare Streusignal, wie es auf einer Bildanzeige gesehen wird, und
ic) Werte, die aus dem Schnellzeitgradienten erlangter Korrekturverzögerungen erlangt werden, und

ii) direkte Schätzung der Impulsverzerrung aus dem Frequenzspektrum eines der empfangenen HF-Signale und entrauschten HF-Signale.

5. Verfahren nach Anspruch 1, worin die zeitliche Beziehung zwischen den übertragenen HF- und NF-Impulsen so ausgewählt wird, dass für die tatsächliche Abbildungsreichweite der HF-Druckimpuls bei Nulldurchgängen eines Drucks des NF-Impulses gefunden wird, um in den nichtlinearen gestreuten HF-Signalen das Signal zu verbessern, das von resonanten Streuobjekten gestreut wird, deren Resonanzfrequenz nahe der Mittenfrequenz des NF-Impulses liegt.

6. Verfahren nach Anspruch 1, worin die mindestens zwei HF-Zwischensignale aus mindestens zwei HF-Impulsen außerdem kombiniert werden, um lineare HF-Messungssignale bereitzustellen, welche die lineare Streuung von dem Objekt mit einer gleichen Dämpfung wie die nichtlinearen HF-Messungssignale von dem Objekt repräsentieren, und ein lokaler nichtlinearer Streuparameter als das Verhältnis der lokalen Hüllkurven der nichtlinearen HF-Messungssignale und der linearen HF-Messungssignale gebildet wird.

7. Verfahren nach Anspruch 6, worin ein quantitativer lokaler Streuparameter durch Normieren des lokalen nichtlinearen Streuparameters mit einem Schätzwert der lokalen NF-Druckamplitude am Ort des sich ausbreitenden HF-Impulses erlangt wird.

8. Verfahren nach Anspruch 1, worin ein lokaler Ausbreitungsparameter des Objekts aus dem Schnellzeitgradienten der Korrekturverzögerungen erlangt wird.

9. Verfahren nach Anspruch 8, worin ein lokaler quantitativer Ausbreitungsparameter durch Normieren des lokalen Ausbreitungsparameters mit einem Schätzwert der lokalen NF-Druckamplitude an einem Ort des sich ausbreitenden HF-Impulses erlangt wird.

10. Verfahren nach Anspruch 7 oder 9, worin einer oder beide des quantitativen lokalen Streuparameters und des quantitativen lokalen Ausbreitungsparameters verwendet werden, um eine lokale Temperatur des Objekts zu schätzen.

11. Verfahren nach Anspruch 1, worin die nichtlinearen HF-Messungssignale für computertomografische Bildrekonstruktionen verwendet werden.

12. Verfahren nach Anspruch 1, worin mindestens eines von Folgendem:

- die Polarität des übertragenen HF-Impulses, und
- die Phase des übertragenen HF-Impulses, und
- die Amplitude des übertragenen HF-Impulses,

für jeden übertragenen Impulskomplexe variiert wird und diese Variation bei der Verarbeitung des empfangenen HF-Signals kompensiert wird.

13. Instrument zur Messung oder Abbildung mit elastischen Wellen in einem Bereich eines Objekts, umfassend:

a) Übertragungsmittel zum Übertragen von mindestens zwei Impulskomplexen elastischer Wellen (101, 104; 501, 504) zu dem Bereich, worin jeder der Impulskomplexe aus einem Impuls (103, 106, 107, 109, 111-113; 311, 313, 315; 503, 506, 512, 522) in einem Hochfrequenzband, nachfolgend als HF bezeichnet, und einem Impuls (102, 105, 108, 110; 312, 314, 316; 401, 402, 411, 412; 502, 505; 511, 521) in einem Niederfrequenzband, nachfolgend als NF bezeichnet, mit den gleichen oder überschneidenden Strahlrichtungen zusammengesetzt ist und worin der HF-Impuls räumlich so dicht am NF-Impuls ist, dass der HF-Impuls zumindest für einen Teil der Bildtiefe die nichtlineare Modifikation des Objekts durch den NF-Impuls beobachtet, und worin mindestens der übertragene NF-Impuls für jeden übertragenen Impulskomplex variiert, um die durch den HF-Impuls beobachtete Elastizität des Streuobjekts entlang zumindest für einen Teil der Bildtiefe nichtlinear zu manipulieren, worin der NF-Impuls eines Impulskomplexes null sein kann und der NF-Impuls für mindestens einen Impulskomplex von null verschieden ist, und

b) Messwandler- (208; 301; 601; 700; 1101; 1201; 1303) und Empfängermittel zum Aufnehmen empfangener HF-Signale (604, 606, 609, 610-612; 706) aus einer oder beiden der gestreuten und durchgelassenen HF-Komponenten aus mindestens zwei übertragenen Impulskomplexen, und

c) einen Prozessor, der zum Verarbeiten der empfangenen HF-Signale, um die Messungs- oder Bildsignale zu bilden, konfiguriert ist;

**dadurch gekennzeichnet, dass** der Prozessor Mittel aufweist:

aus den empfangenen HF-Signalen HF-Zwischensignale als die empfangenen HF-Signale zu bilden, die HF-Zwischensignale in der Schnellzeit zu verzögerungskorrigieren, und die HF-Zwischensignale in der Schnellzeit zu impulsverzerrungskorrigieren, um dadurch korrigierte HF-Zwischensignale aus mindestens zwei übertragenen Impulskomplexen mit Differenzen im NF-Impuls zu bilden, wobei die korrigierten HF-Zwischensignale aus unterschiedlichen Impulskomplexen kombiniert werden, um nichtlineare HF-Messungssignale zu bilden, welche die nichtlinearen elastischen Objekteigenschaften darstellen, und worin die nichtlinearen HF-Messungssignale zur weiteren Verarbeitung verwendet werden, um Messungs- oder Bildsignale zu bilden.

14. Instrument nach Anspruch 13, worin der Prozessor Mittel für eines von Folgendem einschließt: Berechnung und Schätzung mindestens der Korrekturverzögerungen und der Impulsverzerrungskorrekturen.

15. Instrument nach Anspruch 13, worin der Prozessor Mittel einschließt, um eines oder mehrere von Messungs- oder Bildsignalen zu schätzen, die Folgendes repräsentieren:

- lineare Streuung vom Objekt, und
- nichtlineare Streuung vom Objekt, und
- einen nichtlinearen Ausbreitungsparameter des Objekts, und
- einen quantitativen nichtlinearen Ausbreitungsparameter des Objekts, und
- einen lokalen nichtlinearen Streuparameter des Objekts, und
- einen quantitativen lokalen nichtlinearen Streuparameter des Objekts, und
- mindestens eine der radialen Verschiebung des Objekts,
- mindestens eines von Verschiebungsformänderung und Formänderungsgeschwindigkeit des Objekts
- lokale Abweichungen in der Objekttemperatur.

16. Instrument nach Anspruch 13, worin die LF-Blende eine große, nicht fokussierte Blende ist, sodass die HF-Abbildungstiefe innerhalb des Nahfelds der LF-Blende liegt.

17. Instrument nach Anspruch 13, das Mittel zur Schätzung von Wellenfrontabbildungsfehlerkorrekturen und Mittel zum Korrigieren von Wellenfrontabbildungsfehlern bei der Strahlformung einschließt.

**Revendications**

1. Procédé de mesure ou d'imagerie de dispersion non linéaire d'ondes élastiques à partir d'une zone d'un objet, où

a) au moins deux complexes d'impulsions d'ondes élastiques (101, 104 ; 501, 504) sont transmis vers ladite zone où lesdits complexes d'impulsions sont constitués d'une impulsion (103, 106, 107, 109, 111-113 ; 311, 313, 315 ; 503, 506, 512, 522) dans une bande haute fréquence, appelée ci-après « bande HF », et d'une impulsion (102, 105, 108, 110 ; 312, 314, 316 ; 401, 402, 411, 412 ; 502, 505 ; 511, 521) dans une bande basse fréquence, appelée ci-après « bande LF », avec les mêmes directions de faisceau ou des directions de faisceau en chevauchement, et où l'impulsion HF est spatialement si proche de l'impulsion LF que l'impulsion HF observe la modification non linéaire de l'objet par l'impulsion LF au moins pour une partie de la profondeur d'image, et où au moins l'impulsion LF transmise varie pour chaque complexe d'impulsions transmis en vue de manipuler de manière non linéaire l'élasticité de disperseurs observée par l'impulsion HF le long d'au moins une partie de la profondeur d'image, où l'impulsion LF d'un complexe d'impulsions peut être nulle et l'impulsion LF pour au moins un complexe d'impulsions est non nulle, et où
b) les signaux HF reçus (604, 606, 609, 610-612 ; 706) sont capturés par des transducteurs (208 ; 301 ; 601 ; 700 ; 1101 ; 1201 ; 1303) à partir d'une ou des deux composantes parmi des composantes HF transmises et des composantes HF dispersées à partir d'au moins deux complexes d'impulsions transmis ; et

comportant l'étape consistant à traiter lesdits signaux HF reçus en vue de former des signaux d'image ou de mesure à des fins d'affichage,
où ledit traitement est **caractérisé en ce que** :

c) au cours du processus de formation desdits signaux d'image ou de mesure, des signaux HF intermédiaires sont formés à partir desdits signaux HF reçus en tant que lesdits signaux HF reçus, et où lesdits signaux HF intermédiaires sont :

- corrigés en termes de retard dans le temps réduit, à savoir « profondeur-temps » ; et
- corrigés en termes de déformation d'impulsion dans le temps réduit,

de manière à former des signaux HF intermédiaires corrigés à partir d'au moins deux complexes d'impulsions transmis avec des différences dans l'impulsion LF, lesdits signaux HF intermédiaires corrigés à partir de complexes d'impulsions différents sont combinés en vue de former des signaux HF de mesure non linéaire qui représentent les propriétés élastiques non linéaires locales d'objet, et où lesdits signaux HF de mesure non linéaire sont utilisés dans le cadre d'un traitement ultérieur en vue de former des signaux d'image ou de mesure.

2. Procédé selon la revendication 1, où la correction de déformation d'impulsion est mise en oeuvre en tant qu'au moins l'un(e) parmi :

- une expansion/compression de temps réduit dans des intervalles du signal ; et
- un déphasage à travers un mélange de fréquences ; et
- un filtrage dans le temps réduit dans un filtre de correction de déformation d'impulsion ; et
- une pré-déformation des impulsions HF transmises ; et
- une correction d'amplitude.

3. Procédé selon la revendication 1, où lesdits retards de correction sont obtenus à partir de l'un(e) parmi :

i) des simulations de champs d'ondes élastiques LF et HF composites utilisant des paramètres de propagation d'ondes locales présentant l'une parmi :

des valeurs supposées sur la base d'une connaissance matérielle avant les mesures ; et
des valeurs obtenues par ajustement manuel pour la minimisation d'au moins l'un parmi le bruit de réverbération d'impulsion et le signal de dispersion linéaire dans un affichage d'image ; et

ii) un ajustement manuel desdits retards de correction pour la suppression maximale d'un ou des deux parmi :

iia) un bruit à dispersion multiple dans l'image tel que montré sur un écran d'affichage ; et
iib) un signal dispersé linéairement dans l'image tel que montré sur un écran d'affichage ; et

iii) sont estimés par le biais de techniques de traitement de signal sur l'un desdits signaux HF reçus et desdits signaux HF à bruit supprimé.

4. Procédé selon la revendication 1, où lesdites corrections de déformation d'impulsions sont obtenues à partir d'une parmi :

i) des simulations des champs d'ondes élastiques LF et HF composites utilisant des paramètres de propagation d'ondes locales qui présentent l'une parmi :

ia) des valeurs supposées sur la base d'une connaissance matérielle avant les mesures ; et
ib) des valeurs obtenues par ajustement manuel pour la minimisation d'au moins l'un parmi le bruit de réverbération d'impulsion et le signal de dispersion linéaire, tels que vus sur un affichage d'image ; et
ic) des valeurs obtenues à partir du gradient de temps réduit de retards de correction obtenus ; et

ii) une estimation directe de la déformation d'impulsion à partir du spectre de fréquences de l'un parmi les signaux HF reçus et les signaux HF à bruit supprimé.

5. Procédé selon la revendication 1, où la relation temporelle entre les impulsions HF transmises et les impulsions LF transmises est sélectionnée de sorte que, pour la plage d'imagerie en cours, l'impulsion de pression HF est située à des passages à zéro d'une pression du signal LF en vue d'optimiser, dans lesdits signaux HF dispersés non linéaires, le signal dispersé à partir de disperseurs résonnants avec une fréquence de résonnance proche de la fréquence centrale de l'impulsion LF.

6. Procédé selon la revendication 1, où lesdits au moins deux signaux HF intermédiaires à partir d'au moins deux impulsions HF transmises sont également combinés en vue de fournir des signaux HF de mesure linéaire qui représentent une dispersion linéaire à partir de l'objet avec la même atténuation que lesdits signaux HF de mesure non linéaire à partir de l'objet, et un paramètre de dispersion non linéaire locale est formé en tant que le rapport des enveloppes locales desdits signaux HF de mesure non linéaire et desdits signaux HF de mesure linéaire.

7. Procédé selon la revendication 6, où un paramètre de dispersion locale quantitative est obtenu en normalisant ledit paramètre de dispersion non linéaire locale avec une estimation de l'amplitude de pression LF locale à l'emplacement de l'impulsion HF de propagation.

8. Procédé selon la revendication 1, où un paramètre de propagation locale de l'objet est obtenu à partir du gradient de temps réduit desdits retards de correction.

9. Procédé selon la revendication 8, où un paramètre de propagation quantitative locale est obtenu en normalisant ledit paramètre de propagation locale avec une estimation d'une amplitude de pression LF locale à l'emplacement de l'impulsion HF de propagation.

10. Procédé selon la revendication 7 ou 9, où l'un ou les deux parmi ledit paramètre de dispersion locale quantitative et ledit paramètre de propagation locale quantitative est/sont utilisé(s) en vue d'estimer une température locale de l'objet.

11. Procédé selon la revendication 1, où lesdits signaux HF de mesure non linéaire sont utilisés à des fins de reconstructions d'images tomographiques informatisées.

12. Procédé selon la revendication 1, où au moins l'une parmi

- la polarité de l'impulsion HF transmise ;
- la phase de l'impulsion HF transmise ; et
- l'amplitude de l'impulsion HF transmise

est variée pour chaque complexe d'impulsions transmis, et où cette variation est compensée dans le cadre du traitement du signal HF reçu.

13. Instrument de mesure ou d'imagerie avec des ondes élastiques dans une zone d'un objet, comprenant :

a) un moyen de transmission pour transmettre au moins deux complexes d'impulsions d'ondes élastiques (101, 104 ; 501, 504), vers ladite zone où chacun des complexes d'impulsions est constitué d'une impulsion (103, 106, 107, 109, 111-113 ; 311, 313, 315 ; 503, 506, 512, 522) dans une bande haute fréquence, appelée ci-après « bande HF », et d'une impulsion (102, 105, 108, 110 ; 312, 314, 316 ; 401, 402, 411, 412 ; 502, 505 ; 511, 521) dans une bande basse fréquence, appelée ci-après « bande LF », avec les mêmes directions de faisceau ou des directions de faisceau en chevauchement, et où l'impulsion HF est spatialement si proche de l'impulsion LF que ladite impulsion HF observe la modification non linéaire de l'objet par l'impulsion LF au moins pour une partie de la profondeur d'image, et où au moins l'impulsion LF transmise varie pour chaque complexe d'impulsions transmis en vue de manipuler de manière non linéaire l'élasticité de disperseurs observée par l'impulsion HF le long d'au moins une partie de la profondeur d'image, où l'impulsion LF d'un complexe d'impulsions peut être nulle et l'impulsion LF pour au moins un complexe d'impulsions est non nulle, et

b) un moyen de transducteur (208 ; 301 ; 601 ; 700 ; 1101 ; 1201 ; 1303) et un moyen de récepteur pour capturer des signaux HF reçus (604, 606, 609, 610-612 ; 706) à partir d'une ou des deux composantes parmi des composantes HF transmises et des composantes HF dispersées à partir d'au moins deux complexes d'impulsions transmis ; et

c) un processeur configuré de manière à traiter lesdits signaux HF reçus en vue de former des signaux d'image ou de mesure ;

**caractérisé en ce que** ledit processeur inclut des moyens :

pour former des signaux HF intermédiaires à partir desdits signaux HF reçus en tant que lesdits signaux HF reçus ;

pour corriger en termes de retard lesdits signaux HF intermédiaires dans le temps réduit ; et

pour corriger en termes de déformation d'impulsion lesdits signaux HF intermédiaires dans le temps réduit, de manière à former des signaux HF intermédiaires corrigés à partir d'au moins deux complexes d'impulsions transmis avec des différences dans l'impulsion LF, lesdits signaux HF intermédiaires corrigés à partir de complexes d'impulsions différents sont combinés en vue de former des signaux HF de mesure non linéaire qui représentent les propriétés élastiques non linéaires locales d'objet, et où lesdits signaux HF de mesure non linéaire sont utilisés dans le cadre d'un traitement ultérieur en vue de former des signaux d'image ou de mesure.

14. Instrument selon la revendication 13, dans lequel ledit processeur inclut un moyen pour l'un parmi un calcul et une estimation d'au moins lesdits retards de correction et lesdites corrections de déformation d'impulsion.

15. Instrument selon la revendication 13, dans lequel ledit processeur inclut un moyen pour estimer un ou plusieurs signaux parmi des signaux d'image ou de mesure qui représentent :

- une dispersion linéaire à partir de l'objet ; et
- une dispersion non linéaire à partir de l'objet ; et
- un paramètre de propagation non linéaire de l'objet ; et
- un paramètre de propagation non linéaire quantitative de l'objet ; et
- un paramètre de dispersion non linéaire locale de l'objet ; et
- un paramètre de dispersion non linéaire locale et quantitative de l'objet ; et

au moins l'un d'un déplacement radial d'objet ; et

au moins l'un(e) parmi une contrainte de déplacement et un taux de contrainte ; et des variations locales en termes de température d'objet.

16. Instrument selon la revendication 13, où l'ouverture de transmission LF est une ouverture large non focalisée, de sorte que la plage d'imagerie HF se situe dans le champ proche de l'ouverture LF.

17. Instrument selon la revendication 13, lequel inclut un moyen pour estimer des corrections d'aberrations de fronts d'ondes et un moyen pour corriger des aberrations de fronts d'ondes dans le cadre de la conformation de faisceau.

a)

b)

c)

FIG. 1

FIG. 2a

FIG. 2b

FIG. 2c

FIG. 2d

FIG. 3a

FIG. 3b

FIG. 3c

FIG. 4a

FIG. 4b

Pressure oscillation

Radius oscillation

FIG. 5a

FIG. 5b

FIG. 5c

FIG. 6

FIG. 7

FIG. 8

FIG. 9

FIG. 10

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005277835 A
- US 2006052699 A
- US 6485423 B
- US 6905465 B
- US 7273455 B